(19) Europäisches Patentamt
European Patent Office
Office européen des brevets

(11) EP 2 322 604 A1

(12) # EUROPEAN PATENT APPLICATION
published in accordance with Art. 153(4) EPC

(43) Date of publication:
**18.05.2011 Bulletin 2011/20**

(21) Application number: **09804952.1**

(22) Date of filing: **03.08.2009**

(51) Int Cl.:
***C12N 5/10** (2006.01)*
***A61L 27/00** (2006.01)*
***C12N 1/04** (2006.01)*
***C12N 5/07** (2010.01)*
***C12N 15/09** (2006.01)*

(86) International application number:
**PCT/JP2009/063768**

(87) International publication number:
**WO 2010/016469 (11.02.2010 Gazette 2010/06)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **04.08.2008 JP 2008201346**

(71) Applicant: **Showa University**
**Tokyo, 1428555 (JP)**

(72) Inventors:
- **NAKAMURA Masanori**
  **Tokyo 142-8555 (JP)**
- **KURODA Shunichi**
  **Suita-shi**
  **Osaka 565-0871 (JP)**
- **ODA Mitsuo**
  **Shinagawa-ku,Tokyo 142-8555 (JP)**
- **MAYAHARA Mitsuori**
  **Shinagawa-ku,Tokyo 142-8555 (JP)**
- **KENMOTSU Sachiyo**
  **Shinagawa-ku,Tokyo 142-8555 (JP)**
- **IGARASHI Koichi**
  **Mino-shi**
  **Osaka 562-0015 (JP)**
- **OIE Kazunori**
  **Mino-shi**
  **Osaka 562-0015 (JP)**

(74) Representative: **Høiberg A/S**
**St. Kongensgade 59 A**
**1264 Copenhagen K (DK)**

(54) **TECHNIQUE FOR REGULATING REGENERATION OF TISSUE OR FAULTY OR ABNORMAL PART IN ORGAN USING NELL-1**

(57) The object aims to form and support a cell, a tissue or an organ induced by differentiation. Disclosed is a composition for inducing the differentiation of a cell capable of being differentiated in a given direction to thereby produce a cell, a tissue or an organ through the further induction of the differentiation in the given direction. The composition comprises NELL-1 or a substance which can be altered so as to act as NELL-1 upon the differentiation. Also disclosed is a composition for supporting a cell, a tissue or an organ produced by the induction of the differentiation.

EP 2 322 604 A1

## Description

TECHNICAL FIELD

The present invention relates to a technique for heterotropic regeneration and control of a cell, tissue and organ. More specifically, the present invention relates to a technique for heterotropic regeneration and control of a cell, tissue and organ by using NELL-1.

BACKGROUND ART

There are currently numerous patients who need transplantation of organs such as liver, pancreas, thyroid gland and kidney. For example, "Abstract of the Survey on National Health and Nutrition, 2006" from the Ministry of Labour, Health and Welfare describes that the total number of diabetic patients and possible patients thereof is about 18, 700, 000, increased by 2, 500, 000 (15.4%) from that in 2002. In particular, it is difficult for the patients with endogenous insulin deficiency to live without control of blood glucose by self-injection of insulin every day, and there are many patients in need of transplantation of pancreas (pancreatic β cells) or a technique alternative thereto.

Furthermore, excessive secretion of thyroid hormones leads to abnormal activation of metabolism, which in turn leads to Basedow's disease, or the like. On the other hand, insufficient secretion of thyroid hormones causes hypothyroidism. For recovery of the thyroid function, organ transplantation or a technique alternative thereto is required not only for patients with lowered secretion but also for patients with extremely lowered metabolism, which is a complication of thyroid gland ablation operations conducted for patents with excessive secretion of thyroid hormones.

However, for actual transplantation of an organ, the organ to be transplanted needs to be obtained, but it can currently be obtained only by benevolent donation from a third person. There are many techniques of forced induction into a particular differentiation direction of an undifferentiated cell, and the techniques are still under intensive studies. Various research and developments are under progress in the field of regenerative medicine for clinical application of such techniques. However, success so far has been limited to induced differentiation of grafts such as cornea, skin/ mucous membranes, bones, and cartilages, and it is still not successful to provide an organ that can function normally in the body.

NELL-1 is reported to be involved in induction of osteogenic cells and induction of chondrogenic cells (Patent Documents 1 to 4 and Non-patent Document 1). The bone repair with NELL-1 occurs *in situ* where it should occur inherently. However, it has been completely unknown or even unpredicted that NELL-1 has an action to form and/or support various organs heterotopically.

Patent Document 5 describes formation of various tissues by pretreatment of an ectoderm sample from amphibia in the presence of activin and subsequent transplantation of the sample in embryo. The method is targeted to amphibia, which have a much higher self repair capability than mammals, but the survival rate of the amphibia after transplantation is only 30% (70% dead). It is thus impossible to use the method described in Patent Document 5 in mammals. There is no report of a cultured mammal tissue containing added TGF β or activin surviving after transplantation.

When the method described in Patent Document 5 is applied to humans, neofetus should be separated from placenta and treated in expectation of its possible future disease, and thus, such a method is impossible both physically and ethically. Because the transplanted cells are introduced into an embryo in this method, there is no way to help the transplanted individual, even if the cells proliferate abnormally or if the method cause disadvantages in terms of activity. Thus, the method described in Patent Document 5 is not suited for mammals.

Non-patent Documents 2 and 3 describe heterotopical formation of bones in the living body by using BMP that is known as an osteogenic factor. However, the organs reported to be formed with BMP were only bones, and there is no report of other cells, tissues or organs formed.

Non-patent Document 4 describes that a human ear cartilage has been formed by decomposing, *in vitro,* a cartilaginous tissue resected from human, adding a potent chondrogenic protein thereto, inoculating the mixture on an auricle-shaped collagen sponge, and transplanting it into a nude mouse. However, the organ reported to be formed by the chondrogenic protein is only ear cartilage, and there is no report of other cells, tissues and organs formed.

Patent Document 1: International Publication WO 2006/089023 pamphlet
Patent Document 2: International Publication WO 2004/072100 pamphlet
Patent Document 3: International Publication WO 2004/024893 pamphlet
Patent Document 4: International Publication WO 01/024821 pamphlet
Patent Document 5: Japanese Laid-Open Publication 2000-217571
Non-patent Document 1: J. Bone Miner Res. 2007 Jun; 22 (6) 918-30
Non-patent Document 2: J. Dent. Res. 82(8): 581-584, 2003
Non-patent Document 3: Plast. Reconstr. Surg. 108: 952, 2001

Non-patent Document 4: Isogai N, Comparison of different chondrocytes for use in tissue engineering of cartilage model structures. Tissue Eng. 2006 Apr; 12(4): 691-703

SUMMARY OF THE INVENTION

PROBLEMS TO BE SOLVED BY THE INVENTION

It is an object of the present invention to provide a technique for forming and/or supporting a differentiated cell, tissue or organ that is applicable to regenerative medicine.
It is another object of the present invention to provide a method of protecting a differentiated cell, tissue or organ transplanted in the living body from immunological rejection reaction and supporting it in the living body.

MEANS FOR SOLVING THE PROBLEMS

As a result of intensive studies to achieve the objects above, the present inventors have discovered that NELL-1 has a potency to induce further differentiation of cells already in the differentiation phase and thus completed the present invention.
The present inventors have also discovered that NELL-1 has an action to inhibit *in vivo* immunological rejection and thus completed the present invention.
Such actions of NELL-1 have not been known and are provided for the first time by the present invention.
In order to achieve the above objects, the present invention provides, for example, the means below.

(Item 1)

A composition for inducing differentiation of a cell having been directed to a given differentiation, to a further differentiated cell, tissue or organ directed to the given differentiation, wherein the composition comprises NELL-1 or a substance which is altered to function as NELL-1 at the time of said inducing differentiation.

(Item 2)

The composition according to the above item, wherein the composition is for heterotopically forming said further differentiated cell, tissue or organ directed to the given differentiation in the presence of fat tissue and blood vessels wherein the blood vessels support the fat tissue.

(Item 3)

The composition according to the above items, wherein said cell having been directed to a given differentiation is a somatic stem cell.

(Item 4)

The composition according to the above items, wherein said somatic stem cell is a somatic stem cell present in a tissue selected from the group consisting of hematopoietic tissue, epithelial tissue, connective tissue, muscle tissue and nerve tissue.

(Item 5)

The composition according to the above items, wherein said somatic stem cell is a stem cell having been directed to differentiation of hematopoietic tissue, epithelial tissue or connective tissue.

(Item 6)

The composition according to the above items, wherein the somatic stem cell present in the hematopoietic tissue is a hematopoietic stem cell, which is a mother cell of a blood cell having self repair capability and multipotency.

(Item 7)

The composition according to the above items, wherein the somatic stem cell present in the epithelial tissue

is a stem cell having been directed to differentiation of epithelial tissue, which is a mother cell of epithelial system cell having self repair capability and multipotency.

(Item 8)

The composition according to the above items, wherein the somatic stem cell present in the epithelial cell is adenoblast or a cell included in a hair root.

(Item 9)

The composition according to the above items, wherein the differentiated cell, tissue or organ is differentiated hematopoietically or epithelially.

(Item 10)

The composition according to the above items, wherein the hematopoietically differentiated cell, tissue or organ is a blood cell differentiated from hematopoietic stem cell, selected from the group consisting of a leukocyte selected from the group consisting of neutrophil, eosinophil, basophil and lymphocyte; erythrocyte; platelet; macrophage; and a combination thereof.

(Item 11)

The composition according to the above items, wherein the epithelially differentiated cell, tissue or organ is exocrine gland and a duct thereof.

(Item 12)

The composition according to the above items, wherein the exocrine gland is selected from the group consisting of perspiratory gland, sebaceous gland, intestinal gland, gastric gland and salivary gland.

(Item 13)

The composition according to the above items, wherein the epithelially differentiated cell, tissue or organ is a hair, a hair bulb, a hair root or a gland tissue associated with a hair root.

(Item 14)

The composition according to the above items, wherein said cell having been directed to a given differentiation is an adenoblast, and said differentiated cell, tissue or organ is an exocrine gland and a duct thereof.

(Item 15)

The composition according to the above items, wherein said cell having been directed to a given differentiation is a cell included in a hair root, and said differentiated cell, tissue or organ is a hair, a hair bulb, a hair root or a gland tissue associated with a hair root.

(Item 16)

The composition according to the above items, which comprises said NELL-1 at about 0.01 μg/ml or more.

(Item 17)

The composition according to item 16, which comprises said NELL-1 from about 5 μg/ml to about 50 μg/ml.

(Item 18)

The composition according to the above items, wherein said differentiated cell, tissue or organ has a function corresponding to that present in nature.

(Item 19)

A material for inducing differentiation of a cell having been directed to a given differentiation, to a further differentiated cell, tissue or organ directed to the given differentiation, said material comprising:

A) a sustained-releasing scaffold; and
B) NELL-1 or a substance which is altered to function as NELL-1 at the time of said inducing differentiation.

(Item 20)

The material according to the above items, wherein the material is for heterotopically forming a further differentiated cell, tissue or organ directed to the given differentiation in the presence of fat tissue and blood vessels wherein the blood vessels support the fat tissue.

(Item 21)

The material according to the above items, wherein said sustained-releasing scaffold is an extracellular matrix.

(Item 22)

The material according to the above items, wherein said sustained-releasing scaffold is selected from the group consisting of collagen and atelocollagen.

(Item 23)

The material according to the above items, wherein said cell having been directed to a given differentiation is a somatic stem cell.

(Item 24)

The material according to the above items, wherein said somatic stem cell is a somatic stem cell present in a tissue selected from the group consisting of hematopoietic tissue, epithelial tissue, connective tissue, muscle tissue and nerve tissue.

(Item 25)

The material according to the above items, wherein said somatic stem cell is a somatic stem cell present in hematopoietic tissue, epithelial tissue or connective tissue.

(Item 26)

The material according to the above items, wherein the somatic stem cell present in the hematopoietic tissue is a hematopoietic stem cell, which is a mother cell of a blood cell having self repair capability and multipotency.

(Item 27)

The material according to the above items, wherein the somatic stem cell present in the epithelial tissue is a stem cell having been directed to differentiation of epithelial tissue, which is a mother cell of epithelial system cell having self repair capability and multipotency.

(Item 28)

The material according to the above items, wherein the somatic stem cell present in the epithelial cell is an adenoblast or a cell included in a hair root.

(Item 29)

The material according to the above items, wherein the differentiated cell, tissue or organ is differentiated

hematopoietically or epithelially.

(Item 30)

The material according to the above items, wherein the hematopoietically differentiated cell, tissue or organ is a blood cell differentiated from a hematopoietic stem cell, selected from the group consisting of a leukocyte selected from the group consisting of a neutrophil, an eosinophil, a basophil and a lymphocyte; an erythrocyte; a platelet; a macrophage; and a combination thereof.

(Item 31)

The material according to the above items, wherein the epithelially differentiated cell, tissue or organ is an exocrine gland and a duct thereof.

(Item 32)

The material according to the above items, wherein the exocrine gland is selected from the group consisting of a perspiratory gland, a sebaceous gland, an intestinal gland, a gastric gland and a salivary gland.

(Item 33)

The material according to the above items, wherein the epithelially differentiated cell, tissue or organ is a hair, a hair bulb, a hair root or a gland tissue associated with a hair root.

(Item 34)

The material according to the above items, wherein said cell having been directed to a given differentiation is an adenoblast, and said differentiated cell, tissue or organ is exocrine gland and a duct thereof.

(Item 35)

The material according to the above items, wherein said cell having been directed to a given differentiation is a cell included in a hair root, and said differentiated cell, tissue or organ is a hair, a hair bulb, a hair root or a gland tissue associated with a hair root.

(Item 36)

The material according to the above items, wherein said NELL-1 is comprised at about 0.01 μg/ml or more.

(Item 37)

The material according to the above items, wherein said NELL-1 is comprised from about 5 μg/ml to about 50 μg/ml.

(Item 38)

The material according to item 19, wherein said differentiated cell, tissue or organ has a function corresponding to that present in nature.

(Item 39)

A kit for inducing differentiation of a cell having been directed to a given differentiation, to a further differentiated cell, tissue or organ directed to the given differentiation, wherein said kit comprises NELL-1 or a substance which is altered to function as NELL-1 at the time of said inducing differentiation.

(Item 40)

The kit according to the above items, wherein the kit is for heterotopically forming a further differentiated cell,

tissue or organ directed to the given differentiation in the presence of fat tissue and blood vessels wherein the blood vessels support the fat tissue.

(Item 41)

The kit according to the above items, wherein the differentiated cell, tissue or organ is differentiated hematopoietically or epithelially.

(Item 42)

The kit according to the above items, wherein the hematopoietically differentiated cell, tissue or organ is a blood cell differentiated from a hematopoietic stem cell, selected from the group consisting of a leukocyte selected from the group consisting of a neutrophil, an eosinophil, a basophil and a lymphocyte; an erythrocyte; a platelet; a macrophage; and a combination thereof.

(Item 43)

The kit according to the above items, wherein the epithelially differentiated cell, tissue or organ is an exocrine gland and a duct thereof.

(Item 44)

The kit according to the above items, wherein the exocrine gland is selected from the group consisting of a perspiratory gland, a sebaceous gland, an intestinal gland, a gastric gland and a salivary gland.

(Item 45)

The kit according to the above items, wherein the epithelially differentiated cell, tissue or organ is a hair, a hair bulb, a hair root or a gland tissue associated with a hair root.

(Item 46)

The kit according to the above items, wherein said NELL-1 is comprised at about 0.01 μg/ml or more.

(Item 47)

The kit according to the above items, wherein said NELL-1 is comprised from about 5 μg/ml to about 50 μg/ml.

(Item 48)

A medical device for inducing differentiation of a cell having been directed to a given differentiation, to a further differentiated cell, tissue or organ directed to the given differentiation, wherein said medical device comprises:

A) NELL-1 or a substance which is altered to function as NELL-1 at the time of said inducing differentiation;
B) a sustained-releasing scaffold; and
C) a container.

(Item 49)

The medical device according to the above items, wherein said medical device is for heterotopically forming a further differentiated cell, tissue or organ directed to the given differentiation in the presence of fat tissue and blood vessels.

(Item 50)

The medical device according to the above items, wherein said sustained-releasing scaffold is an extracellular matrix.

(Item 51)

The medical device according to the above items, wherein said sustained-releasing scaffold is selected from the group consisting of collagen and atelocollagen.

(Item 52)

The medical device according to the above items, wherein the differentiated cell, tissue or organ is differentiated hematopoietically or epithelially.

(Item 53)

The medical device according to the above items, wherein the hematopoietically differentiated cell, tissue or organ is a blood cell differentiated from a hematopoietic stem cell, selected from the group consisting of a leukocyte selected from the group consisting of a neutrophil, an eosinophil, a basophil and a lymphocyte; an erythrocyte; a platelet; a macrophage; and a combination thereof.

(Item 54)

The medical device according to the above items, wherein the epithelially differentiated cell, tissue or organ is an exocrine gland and a duct thereof.

(Item 55)

The medical device according to the above items, wherein the exocrine gland is selected from the group consisting of a perspiratory gland, a sebaceous gland, an intestinal gland, a gastric gland and a salivary gland.

(Item 56)

The medical device according to the above items, wherein the epithelially differentiated cell, tissue or organ is a hair, a hair bulb, a hair root or a gland tissue associated with a hair root.

(Item 57)

The medical device according to the above items, wherein said NELL-1 is comprised at about 0.01 μg/ml or more.

(Item 58)

The medical device according to the above items, wherein said NELL-1 is comprised from about 5 μg/ml to about 50 μg/ml.

(Item 59)

A method for producing a cell, tissue or organ, wherein said method comprises the steps of:

providing a cell having been directed to a given differentiation; and
contacting said cell having been directed to a given differentiation with NELL-1 or a substance which is altered to function as NELL-1 at the time of said inducing differentiation.

(Item 60)

The method according to the above items, wherein said cell having been directed to a given differentiation is provided in the presence of fat tissue and blood vessels supporting the same.

(Item 61)

The method according to the above items, wherein said cell having been directed to a given differentiation is contacted with said NELL-1 on a sustained-releasing scaffold.

(Item 62)

The method according to the above items, wherein said sustained-releasing scaffold is an extracellular matrix.

(Item 63)

The method according to the above items, wherein said sustained-releasing scaffold is selected from the group consisting of collagen and atelocollagen.

(Item 64)

The method according to the above items, wherein said cell having been directed to a given differentiation is a somatic stem cell.

(Item 65)

The method according to the above items, wherein said somatic stem cell is a somatic stem cell present in a tissue selected from the group consisting of hematopoietic tissue, epithelial tissue, connective tissue, muscle tissue and nerve tissue.

(Item 66)

The method according to the above items, wherein said somatic stem cell is a somatic stem cell present in hematopoietic tissue, epithelial tissue or connective tissue.

(Item 67)

The method according to the above items, wherein the somatic stem cell present in the hematopoietic tissue is a hematopoietic stem cell, which is a mother cell of a blood cell having self repair capability and multipotency.

(Item 68)

The method according to the above items, wherein the somatic stem cell present in the epithelial tissue is a stem cell having been directed to differentiation of epithelial tissue, which is a mother cell of an epithelial system cell having self repair capability and multipotency.

(Item 69)

The method according to the above items, wherein the somatic stem cell present in the epithelial cell is an adenoblast or a cell included in a hair root.

(Item 70)

A composition for supporting a differentiated cell, tissue or organ, wherein said composition comprises NELL-1 or a substance which is altered to function as NELL-1 at the time of said inducing differentiation.

(Item 71)

The composition according to the above items, wherein said support is carried out at a location which does not allow presence of said differentiated cell, tissue or organ.

(Item 72)

The composition according to the above items, wherein said differentiated cell, tissue or organ has a function corresponding to that present in nature.

(Item 73)

The composition according to the above items, wherein said differentiated cell, tissue or organ is selected from the group consisting of liver, kidney, pancreas, adrenal gland, thyroid gland, ovary and testis.

(Item 74)

A method for supporting a differentiated cell, tissue or organ, wherein said method comprises the steps of:

providing said differentiated cell, tissue or organ; and
delivering NELL-1 or a substance which is altered to function as NELL-1 at the time of said inducing differentiation to said differentiated cell, tissue or organ.

(Item 75)

Use of NELL-1 or a substance which is altered to function as NELL-1 at the time of inducing differentiation in the manufacture of a medicament for producing a differentiated cell, tissue or organ from a cell having been directed to a given differentiation.

(Item 76)

Use of NELL-1 or a substance which is altered to function as NELL-1 at the time of inducing differentiation in the manufacture of a medicament for supporting a differentiated cell, tissue or organ.

(Item A1)

A method for producing a gland tissue in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing a sustained-releasing scaffold comprising NELL-1 in the container and inserting the pedicle tissue graft thereinto;
D) transplanting the container into the space in a state in which blood stream in the pedicle tissue graft is not blocked;
E) maintaining the container in a state in which blood stream in the pedicle tissue graft is not blocked for a period sufficient to produce said gland tissue.

(Item A2)

A method for producing a gland tissue in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing the pedicle tissue graft into the container and adding NELL-1 thereinto;
D) transplanting the container into the space in a state in which blood stream in the pedicle tissue graft is not blocked;
E) maintaining the container in a state in which blood stream in the pedicle tissue graft is not blocked for a period sufficient to produce said gland tissue, wherein NELL-1 is added to the pedicle tissue graft periodically during the maintenance.

(Item A3)

The method according to the above items, wherein the space has a size such that the space can receive the container.

(Item A4)

The method according to the above items, wherein the space is produced between muscles of the thigh.

(Item A5)

The method according to the above items, wherein said NELL-1 is comprised from about 5 μg/ml to about 50 μg/ml.

(Item A6)

The method according to the above items, wherein said sustained-releasing scaffold is selected from the group consisting of a collagen sponge and an atelocollagen gel.

(Item A7)

A method for producing a gland tissue in a fat tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
C) placing a collagen sponge or an atelocollagen gel comprising NELL-1 (from about 5 μg/ml to about 50 μg/ml) in the container and inserting the pedicle tissue graft thereinto;
D) transplanting the container between muscles of the thigh in a state in which a blood stream in the pedicle tissue graft is not blocked; and
E) maintaining the container in the muscles in a state in which the blood stream in the pedicle tissue graft is not blocked for at least about two weeks.

(Item A8)

A method for producing a gland tissue in a fat tissue transplanted in the thigh of a mammal, wherein said method comprises the steps of:

A) making a skin incision in the thigh of the mammal and ablating the hypogastric artery and the vein branched from femoral artery and vein, and the nerve running in parallel, from surrounding tissues;
B) resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
C) placing the pedicle tissue graft into the container and adding NELL-1 (from about 5 μg/ml to about 50 μg/ml) thereinto;
D) transplanting the container between muscles of the thigh in a state in which a blood stream in the pedicle tissue graft is not blocked; and
E) maintaining the container in the muscles in a state in which the blood stream in the pedicle tissue graft is not blocked for at least about two weeks, wherein NELL-1 (from about 5 μg/ml to about 50 μg/ml) is added to the pedicle tissue graft once a week during the maintenance.

(Item A9)

The method according to the above items, characterized in that 0.1 ml of the NELL-1 in a concentration of 50 μg/1 ml is added once a week over four weeks such that 20 μg of the NELL-1 is added in total.

(Item A10)

A method for producing a gland tissue in a muscle tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting a muscle tissue to a size in accordance with a container to be centered at the branch point of the blood vessel supporting the muscle tissue of the mammal and producing a pedicle tissue graft comprising the blood as pedicle;
C) placing a collagen sponge or an atelocollagen gel comprising NELL-1 (from about 5 μg/ml to about 50 μg/ml) in the container and inserting the pedicle tissue graft thereinto;
D) transplanting the container between muscles of the thigh in a state in which a blood stream in the pedicle tissue graft is not blocked; and
E) maintaining the container in the muscles in a state in which the blood stream in the pedicle tissue graft is not blocked for at least about two weeks.

(Item A11)

A method for producing a gland tissue in a muscle tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting a muscle tissue to a size in accordance with a container to be centered at the branch point of the blood vessel supporting the muscle tissue of the mammal and producing a pedicle tissue graft comprising the blood as pedicle;
C) placing the pedicle tissue graft into the container and adding NELL-1 (from about 5 μg/ml to about 50 μg/ml) thereinto;
D) transplanting the container between muscles of the thigh in a state in which a blood stream in the pedicle tissue graft is not blocked; and
E) maintaining the container in the muscles in a state in which the blood stream in the pedicle tissue graft is not blocked for at least about two weeks, wherein NELL-1 (from about 5 μg/ml to about 50 μg/ml) is added to the pedicle tissue graft periodically during the maintenance.

(Item A12)

The method according to the above items for producing a gland tissue in a fat tissue transplanted in the thigh of a mammal,
wherein said step A) is a step of making the skin incision in the thigh of the mammal and ablating the hypogastric artery and vein branched from the femoral artery and vein, and the nerve running in parallel, from surrounding tissues;
wherein said step B) is a step of resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
wherein in said step C) , said NELL-1 is comprised from about 5 μg/ml to about 50 μg/ml, said sustained-releasing scaffold is a collagen sponge or an atelocollagen gel; and
wherein in said step E), said period sufficient to produce said gland tissue is at least about two weeks.

(Item A13)

The method according to the above items for producing a gland tissue in a muscle tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting a muscle tissue to a size in accordance with a container to be centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml, said sustained-releasing scaffold is a collagen sponge or an atelocollagen gel; and
wherein in said step E), said period sufficient to produce said gland tissue is at least about two weeks.

(Item A14)

The method according to the above items for producing a gland tissue in a fat tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
wherein in said step C) , said NELL-1 is comprised from about 5 $\mu$g/ml to about 50 $\mu$g/ml;
wherein said step D) is a step of transplanting the container between muscles of the thigh in a state in which blood stream in the pedicle tissue graft is not blocked; and
wherein in said step E), said period sufficient to produce said gland tissue is at least about two weeks, NELL-1 is added to the pedicle tissue graft once a week from about 5 $\mu$g/ml to about 50 $\mu$g/ml during the maintenance.

(Item A15)

The method according to the above items for producing a gland tissue in a muscle tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting a muscle tissue to a size in accordance with a container to be centered at the branch point of the blood vessel supporting the muscle tissue of the mammal and producing a pedicle tissue graft comprising the blood as pedicle;
wherein in said step C) , said NELL-1 is comprised from about 5 $\mu$g/ml to about 50 $\mu$g/ml;
wherein said step D) is a step of transplanting the container between muscles of the thigh in a state in which blood stream in the pedicle tissue graft is not blocked; and
wherein in said step E), said period sufficient to produce said gland tissue is at least about two weeks, NELL-1 is added to the pedicle tissue graft once a week from about 5 $\mu$g/ml to about 50 $\mu$g/ml during the maintenance.

(Item B1)

A method for producing a hematopoietic tissue in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing a sustained-releasing scaffold comprising NELL-1 in the container and inserting the pedicle tissue graft thereinto;
D) transplanting the container into the space in a state in which a blood stream in the pedicle tissue graft is not blocked;
E) maintaining the container in a state in which the blood stream in the pedicle tissue graft is not blocked for a period sufficient to produce said hematopoietic tissue.

(Item B2)

A method for producing a hematopoietic tissue in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing the pedicle tissue graft into the container and adding NELL-1 thereinto;
D) transplanting the container into the space in a state in which a blood stream in the pedicle tissue graft is not blocked; and
E) maintaining the container in a state in which the blood stream in the pedicle tissue graft is not blocked for a period

sufficient to produce said hematopoietic tissue, wherein NELL-1 is added to the pedicle tissue graft periodically during the maintenance.

(Item B3)

The method according to the above items, wherein the space has a size such that the space can receive the container.

(Item B4)

The method according to the above items, wherein the space is produced between muscles of the thigh.

(Item B5)

The method according to the above items, wherein said NELL-1 is comprised from about 5 μg/ml to about 50 μg/ml.

(Item B6)

The method according to the above items, wherein said sustained-releasing scaffold is selected from the group consisting of a collagen sponge and an atelocollagen gel.

(Item B7)

A method for producing a hematopoietic tissue in a fat tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
C) placing a collagen sponge or an atelocollagen gel comprising NELL-1 (from about 5 μg/ml to about 50 μg/ml) in the container and inserting the pedicle tissue graft thereinto;
D) transplanting the container between muscles of the thigh in a state in which a blood stream in the pedicle tissue graft is not blocked; and
E) maintaining the container in the muscles in a state in which the blood stream in the pedicle tissue graft is not blocked for at least about two weeks.

(Item B8)

A method for producing a hematopoietic tissue in a fat tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
C) placing a collagen sponge or an atelocollagen gel comprising NELL-1 (from about 5 μg/ml to about 50 μg/ml) in the container and inserting the pedicle tissue graft thereinto;
D) transplanting the container between muscles of the thigh in a state in which a blood stream in the pedicle tissue graft is not blocked; and
E) maintaining the container in the muscles in a state in which the blood stream in the pedicle tissue graft is not blocked for at least about two weeks, wherein NELL-1 (from about 5 μg/ml to about 50 μg/ml) is added to the pedicle tissue graft once a week during the maintenance.

(Item B9)

The method according to the above items, characterized in that 0.1 ml of the NELL-1 in a concentration of 50 μg/1 ml is added once a week over four weeks such that 20 μg of the NELL-1 is added in total.

(Item B10)

A method for producing a hematopoietic tissue in a fat tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting a muscle tissue of the mammal to a size in accordance with a container to be centered at the branch point of the blood vessel supporting the muscle tissue of the mammal and producing a pedicle tissue graft comprising the blood as pedicle;
C) placing a collagen sponge or an atelocollagen gel comprising NELL-1 (from about 5 μg/ml to about 50 μg/ml) in the container and inserting the pedicle tissue graft thereinto;
D) transplanting the container between muscles of the thigh in a state in a which blood stream in the pedicle tissue graft is not blocked; and
E) maintaining the container in the muscles in a state in which the blood stream in the pedicle tissue graft is not blocked for at least about two weeks.

(Item B11)

A method for producing a hematopoietic tissue in a muscle tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting a muscle tissue to a size in accordance with a container to be centered at the branch point of the blood vessel supporting the muscle tissue of the mammal and producing a pedicle tissue graft comprising the blood as pedicle;
C) placing the pedicle tissue graft into the container and adding NELL-1 (from about 5 μg/ml to about 50 μg/ml) thereinto;
D) transplanting the container between muscles of the thigh in a state in which a blood stream in the pedicle tissue graft is not blocked; and
E) maintaining the container in the muscles in a state in which the blood stream in the pedicle tissue graft is not blocked for at least about two weeks, wherein NELL-1 (from about 5 μg/ml to about 50 μg/ml) is added to the pedicle tissue graft periodically during the maintenance.

(Item B12)

The method according to the above items for producing a hematopoietic tissue in a fat tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting the abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
wherein in said step C) , said NELL-1 is comprised from about 5 μg/ml to about 50 μg/ml, said sustained-releasing scaffold is a collagen sponge or an atelocollagen gel; and
wherein in said step E), said period sufficient to produce said gland tissue is at least about two weeks.

(Item B13)

The method according to the above items for producing a hematopoietic tissue in a muscle tissue transplanted in the thigh of a mammal,
wherein said step A) is a step of making a skin incision in the thigh of the mammal and ablating the hypogastric artery

and vein branched from the femoral artery and vein, and the nerve running in parallel, from surrounding tissues;
wherein said step B) is a step of resecting a muscle tissue to a size in accordance with a container to be centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml, said sustained-releasing scaffold is a collagen sponge or an atelocollagen gel; and
wherein in said step E), said period sufficient to produce said hematopoietic tissue is at least about two weeks.

(Item B14)

The method according to the above items for producing a hematopoietic tissue in a fat tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml;
wherein said step D) is a step of transplanting the container between muscles of the thigh in a state in which the blood stream in the pedicle tissue graft is not blocked; and
wherein in said step E), said period sufficient to produce said hematopoietic tissue is at least about two weeks, NELL-1 is added to the pedicle tissue graft once a week from about 5 μg/ml to about 50 μg/ml during the maintenance.

(Item B15)

The method according to the above items for producing a hematopoietic tissue in a muscle tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting a muscle tissue to a size in accordance with a container to be centered at the branch point of the blood vessel supporting the muscle tissue of the mammal and producing a pedicle tissue graft comprising the blood as pedicle;
wherein in said step C) , said NELL-1 is comprised from about 5 μg/ml to about 50 μg/ml;
wherein said step D) is a step of transplanting the container between muscles of the thigh in a state in which blood stream in the pedicle tissue graft is not blocked; and
wherein in said step E), said period sufficient to produce said hematopoietic tissue is at least about two weeks, NELL-1 is added to the pedicle tissue graft once a week from about 5 μg/ml to about 50 μg/ml during the maintenance.

(Item C1)

A method for producing a hair, a hair bulb, a hair root or a gland tissue associated with a hair root in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing a sustained-releasing scaffold comprising NELL-1 in the container;
D) placing a cell derived from a hair root on the sustained-releasing scaffold;
E) inserting the pedicle tissue graft into the container;
F) transplanting the container into the space in a state in which a blood stream in the pedicle tissue graft is not blocked; and
G) maintaining the container in a state in which the blood stream in the pedicle tissue graft is not blocked for a period sufficient to produce said hair, hair bulb, hair root or a gland tissue associated with hair root.

(Item C2)

A method for producing a hair, a hair bulb, a hair root or a gland tissue associated with a hair root in a fat tissue

or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing the pedicle tissue graft into the container and adding a cell contained in hair root and NELL-1 thereinto;
D) transplanting the container into the space in a state in which a blood stream in the pedicle tissue graft is not blocked;
E) maintaining the container in a state in which the blood stream in the pedicle tissue graft is not blocked for a period sufficient to produce said hair, hair bulb, hair root or a gland tissue associated with hair root, wherein NELL-1 is added to the pedicle tissue graft periodically during the maintenance.

(Item C3)

The method according to the above items, wherein the space has a size such that the space can receive the container.

(Item C4)

The method according to the above items, wherein the space is produced between muscles of the thigh.

(Item C5)

The method according to the above items, wherein said NELL-1 is comprised from about 5 μg/ml to about 50 μg/ml.

(Item C6)

The method according to the above items, wherein said sustained-releasing scaffold is selected from the group consisting of a collagen sponge and an atelocollagen gel.

(Item C7)

A method for producing a hair, a hair bulb, a hair root or a gland tissue associated with a hair root in a fat tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
C) placing a collagen sponge or an atelocollagen gel comprising NELL-1 (from about 5 μg/ml to about 50 μg/ml) in the container;
D) placing a cell derived from a hair root on the collagen sponge or the atelocollagen gel;
E) inserting the pedicle tissue graft into the container;
F) transplanting the container between muscles of the thigh in a state in which a blood stream in the pedicle tissue graft is not blocked; and
G) maintaining the container in the muscles in a state in which the blood stream in the pedicle tissue graft is not blocked for at least about two weeks.

(Item C8)

A method for producing a hair, a hair bulb, a hair root or a gland tissue associated with a hair root in a fat tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting abdominal subcutaneous fat tissue of the mammal to a size in accordance with a container to be

centered at the branch point of the hypogastric artery and vein and producing a pedicle tissue graft comprising the hypogastric artery and vein as the blood vessel pedicle;

C) placing the pedicle tissue graft into the container and adding a cell contained in hair root and NELL-1 (from about 5 μg/ml to about 50 μg/ml) thereinto;

D) transplanting the container between muscles of the thigh in a state in which a blood stream in the pedicle tissue graft is not blocked; and

E) maintaining the container in the muscles in a state in which the blood stream in the pedicle tissue graft is not blocked for at least about two weeks, wherein NELL-1 is added to the pedicle tissue graft once a week (from about 5 μg/ml to about 50 μg/ml) during the maintenance.

(Item C9)

The method according to the above items, characterized in that 0.1 ml of the NELL-1 in a concentration of 50 μg/l ml is added once a week over four weeks such that 20 μg of the NELL-1 is added in total.

(Item C10)

A method for producing a hair, a hair bulb, a hair root or a gland tissue associated with a hair root in a muscle tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;

B) resecting a muscle tissue to a size in accordance with a container to be centered at the branch point of the blood vessel supporting the muscle tissue of the mammal and producing a pedicle tissue graft comprising the blood as pedicle;

C) placing a collagen sponge or an atelocollagen gel comprising NELL-1 (from about 5 μg/ml to about 50 μg/ml) in the container;

D) placing a cell derived from a hair root on the collagen sponge or the atelocollagen gel;

E) inserting the pedicle tissue graft into the container;

F) transplanting the container between muscles of the thigh in a state in which a blood stream in the pedicle tissue graft is not blocked; and

G) maintaining the container in the muscles in a state in which the blood stream in the pedicle tissue graft is not blocked for at least about two weeks.

(Item C11)

A method for producing a hair, a hair bulb, a hair root or a gland tissue associated with a hair root in a muscle tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;

B) resecting a muscle tissue to a size in accordance with a container to be centered at the branch point of the blood vessel supporting the muscle tissue of the mammal and producing a pedicle tissue graft comprising the blood as pedicle;

C) placing the pedicle tissue graft into the container and adding a cell contained in hair root and NELL-1 (from about 5 μg/ml to about 50 μg/ml) thereinto;

D) transplanting the container between muscles of the thigh in a state in which a blood stream in the pedicle tissue graft is not blocked; and

E) maintaining the container in the muscles in a state in which the blood stream in the pedicle tissue graft is not blocked for at least about two weeks, wherein NELL-1 (from about 5 μg/ml to about 50 μg/ml) is added to the pedicle tissue graft periodically during the maintenance.

(Item C12)

The method according to the above items for producing a hair, a hair bulb, a hair root or a gland tissue associated with a hair root in a fat tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;

wherein said step B) is a step of resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml, said sustained-releasing scaffold is a collagen sponge or an atelocollagen gel;
wherein said step F) is a step of transplanting the container between muscles of the thigh in a state in which a blood stream in the pedicle tissue graft is not blocked; and
wherein in said step G), said period sufficient to produce said gland tissue is at least about two weeks.

(Item C13)

The method according to the above items for producing a hair, a hair bulb, a hair root or a gland tissue associated with a hair root in a muscle tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting a muscle tissue to a size in accordance with a container to be centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml, said sustained-releasing scaffold is a collagen sponge or an atelocollagen gel; and
wherein said step F) is a step of transplanting the container between muscles of the thigh in a state in which a blood stream in the pedicle tissue graft is not blocked; and
wherein in said step G), said period sufficient to produce said hair, hair bulb, hair root or a gland tissue associated with hair root is at least about two weeks.

(Item C14)

The method according to the above items for producing a hair, a hair bulb, a hair root or a gland tissue associated with a hair root in a fat tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml;
wherein said step D) is a step of transplanting the container between muscles of the thigh in a state in which a blood stream in the pedicle tissue graft is not blocked; and
wherein in said step E), said period sufficient to produce said hair, hair bulb, hair root or a gland tissue associated with hair root is at least about two weeks, NELL-1 is added to the pedicle tissue graft once a week from about 5 μg/ml to about 50 μg/ml during the maintenance.

(Item C15)

The method according to the above items for producing a hair, a hair bulb, a hair root or a gland tissue associated with a hair root in a muscle tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting a muscle tissue to a size in accordance with a container to be centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml;
wherein said step D) is a step of transplanting the container between muscles of the thigh in a state in which blood stream in the pedicle tissue graft is not blocked; and
wherein in said step E), said period sufficient to produce said hair, hair bulb, hair root or a gland tissue is at least about two weeks, NELL-1 is added to the pedicle tissue graft once a week from about 5 μg/ml to about 50 μg/ml during the maintenance.

(Item D1)

A method for supporting a liver tissue graft in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing a sustained-releasing scaffold comprising NELL-1 in the container;
D) placing said liver tissue graft on the sustained-releasing scaffold;
E) inserting the pedicle tissue graft into the container;
F) transplanting the container into the space in a state in which a blood stream in the pedicle tissue graft is not blocked;
G) placing the pedicle tissue graft in a state in which the blood stream thereof is not blocked.

(Item D2)

A method for supporting a liver tissue graft in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing the pedicle tissue graft into the container and adding said liver tissue graft and NELL-1 thereinto;
D) transplanting the container into the space in a state in which a blood stream in the pedicle tissue graft is not blocked; and
E) placing the pedicle tissue graft in a state in which the blood stream thereof is not blocked.

(Item D3)

The method according to the above items, wherein the space has a size such that the space can receive the container.

(Item D4)

The method according to the above items, wherein the space is produced between muscles of the thigh.

(Item D5)

The method according to the above items, said NELL-1 is comprised from about 5 μg/ml to about 50 μg/ml.

(Item D6)

The method according to the above items, wherein said sustained-releasing scaffold is selected from the group consisting of a collagen sponge and an atelocollagen gel.

(Item D7)

A method for supporting a liver tissue graft in a fat tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
C) placing a collagen sponge or an atelocollagen gel comprising NELL-1 (from about 5 μg/ml to about 50 μg/ml) in

the container;
D) placing the liver tissue graft on the collagen sponge or the atelocollagen gel;
E) inserting the pedicle tissue graft into the container;
F) transplanting the container between muscles of the thigh in a state in which a blood stream in the pedicle tissue graft is not blocked; and
G) placing the container in a state in which the blood stream in the pedicle tissue graft is not blocked.

(Item D8)

A method for supporting a liver tissue graft in a fat tissue transplanted in the thigh of a mammal, wherein said method comprises the steps of:

A) making a skin incision in the thigh of the mammal and ablating the hypogastric artery and vein branched from he femoral artery and vein, and the nerve running in parallel, from surrounding tissues;
B) resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
C) placing the pedicle tissue graft into the container and adding the liver tissue graft and NELL-1 (from about 5 μg/ml to about 50 μg/ml) thereinto;
D) transplanting the container between muscles of the thigh in a state in which a blood stream in the pedicle tissue graft is not blocked; and
E) placing the container in the muscles in a state in which the blood stream in the pedicle tissue graft is not blocked, wherein NELL-1 (from about 5 μg/ml to about 50 μg/ml) is added to the pedicle tissue graft once a week during in the state.

(Item D9)

The method according to the above items, characterized in that 0.1 ml of the NELL-1 in a concentration of 50 μg/l ml is added once a week over four weeks such that 20 μg of the NELL-1 is added in total.

(Item D10)

A method for supporting a liver tissue graft in a muscle tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting a muscle tissue to a size in accordance with a container to be centered at the branch point of the blood vessel supporting the muscle tissue of the mammal and producing a pedicle tissue graft comprising the blood as pedicle;
C) placing a collagen sponge or an atelocollagen gel comprising NELL-1 (from about 5 μg/ml to about 50 μg/ml) in the container;
D) placing the liver tissue graft on the collagen sponge or the atelocollagen gel;
E) inserting the pedicle tissue graft into the container;
F) transplanting the container between muscles of the thigh in a state in which a blood stream in the pedicle tissue graft is not blocked; and
G) placing the container in the muscles in a state in which the blood stream in the pedicle tissue graft is not blocked.

(Item D11)

A method for supporting a liver tissue graft in a muscle tissue transplanted in the thigh of a mammal, wherein said method comprises the steps of:

A) making a skin incision in the thigh of the mammal and ablating the hypogastric artery and vein branched from the femoral artery and vein, and the nerve running in parallel, from surrounding tissues;
B) resecting a muscle tissue to a size in accordance with a container to be centered at the branch point of the blood vessel supporting the muscle tissue of the mammal and producing a pedicle tissue graft comprising the blood as pedicle;

C) placing the pedicle tissue graft into the container and adding the liver tissue graft and NELL-1 (from about 5 μg/ml to about 50 μg/ml) thereinto;
D) transplanting the container between muscles of the thigh in a state in which a blood stream in the pedicle tissue graft is not blocked; and
E) placing the container in the muscles in a state in which the blood stream in the pedicle tissue graft is not blocked, wherein NELL-1 (from about 5 μg/ml to about 50 μg/ml) is added to the pedicle tissue graft periodically during the in the state.

(Item D12)

The method according to the above items for supporting a liver tissue graft in a fat tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml, said sustained-releasing scaffold is a collagen sponge or an atelocollagen gel; and
wherein said step F) is a step of transplanting the container between muscles of the thigh in a state in which a blood stream in the pedicle tissue graft is not blocked.

(Item D13)

The method according to the above items for supporting a liver tissue graft in a muscle tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting a muscle tissue to a size in accordance with a container to be centered at the branch point of the blood vessel supporting the muscle tissue of the mammal and producing a pedicle tissue graft comprising the blood as pedicle;
wherein in said step C) , said NELL-1 is comprised from about 5 μg/ml to about 50 μg/ml, said sustained-releasing scaffold is a collagen sponge or an atelocollagen gel; and
wherein said step F) is a step of transplanting the container between muscles of the thigh in a state in which a blood stream in the pedicle tissue graft is not blocked.

(Item D14)

The method according to the above items for supporting a liver tissue graft in a fat tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting abdominal subcutaneous fat tissue of the mammal to a size in accordance with a container to be centered at the branch point of the hypogastric artery and vein and producing a pedicle tissue graft comprising the hypogastric artery and vein as the blood vessel pedicle;
wherein in said step C) , said NELL-1 is comprised from about 5 μg/ml to about 50 μg/ml;
wherein said step D) is a step of transplanting the container between muscles of the thigh in a state in which a blood stream in the pedicle tissue graft is not blocked; and
wherein in said step E), NELL-1 is added to the pedicle tissue graft once a week from about 5 μg/ml to about 50 μg/ml during the maintenance.

(Item D15)

The method according to the above items for supporting a liver tissue graft in a muscle tissue transplanted in the thigh of a mammal,
wherein said step A) is a step of making a skin incision in the thigh of the mammal and ablating the hypogastric artery and vein branched from the femoral artery and vein, and the nerve running in parallel, from surrounding tissues;
wherein said step B) is a step of resecting a muscle tissue to a size in accordance with a container to be centered at

the branch point of the blood vessel supporting the muscle tissue of the mammal and producing a pedicle tissue graft comprising the blood as pedicle;
wherein in said step C) , said NELL-1 is comprised from about 5 μg/ml to about 50 μg/ml;
wherein said step D) is a step of transplanting the container between muscles of the thigh in a state in which a blood stream in the pedicle tissue graft is not blocked; and
wherein in said step E) , NELL-1 is added to the pedicle tissue graft once a week from about 5 μg/ml to about 50 μg/ml during the maintenance.

These and other advantages of the present invention will be apparent to those skilled in the art upon reading and understanding the following detailed description with reference to the attached drawings.

EFFECT OF THE INVENTION

The present invention provides a technique for preparing a differentiated cell, tissue or organ by using NELL-1. In particular, the technique according to the present invention can form a heterotopically differentiated cell, tissue or organ. The cell, tissue or organ differentiated by the present invention has an unexpected significant effect of retaining its inherent functions.

Such a technique of forming a cell, tissue and organ by using NELL-1 is applicable also to recent cell transplantation therapy by using an undifferentiated cell such as ES cell. In other words, it is possible to form an organ functioning in the living body by making an undifferentiated cell such as ES cell differentiate in a desired manner and inducing further differentiation by the action of NELL-1. It is also possible to provide an alternative organ for the patients who cannot enjoy the benefit of organ transplantation and advanced medicine by using the patients' own cell, tissue or organ.

Yet another aspect of the present invention provides a technique of supporting a cell, tissue or organ transplanted in the living body by using NELL-1. According to the techniques of the present invention, the transplanted cell, tissue or organ has an unexpectedly advantageous effect of retaining its shape and size and additionally its inherent function.

These effects are provided for the first time by the present invention.

BRIEF DESCRIPTION OF THE DRAWINGS

[Fig. 1A]
Fig. 1A depicts a schematic flowchart showing the procedure for construction of a human NELL1 plasmid.
[Fig. 1B]
Fig. 1B is a schematic flowchart continued from Fig. 1A.
[Fig. 1C]
Fig. 1C is a schematic flowchart continued from Fig. 1B.
[Fig. 1D]
Fig. 1D depicts a hematopoietic tissue in a silicone mold, the hematopoietic tissue formed in the presence of fat tissue and blood vessels wherein the blood vessels support the fat tissue. The bar in the photograph indicates a length of 2.0 mm.
[Fig. 2]
Fig. 2 depicts a glandular system in a silicone mold, the glandular system formed in the presence of fat tissue and blood vessels wherein the blood vessels support the fat tissue. The bar in the photograph indicates a length of 200 μm. G: colloidal secretion released from epithelial cell (simple columnar epithelium), and P: Duct delivering colloidal secretion surrounded by simple cuboidal epithelium
[Fig. 3]
Fig. 3 depicts a glandular system in a silicone mold, the glandular system formed in the presence of fat tissue and blood vessels wherein the blood vessels support the fat tissue. The bar in the photograph indicates a length of 200 μm. G: There are granular secretions present inside, surrounded by epithelial cells (simple columnar epithelium).
[Fig. 4]
Fig. 4 depicts the transplantation region of a transplant 4 weeks after transplantation, which is obtained by transplanting a liver tissue graft containing added physiological saline in the presence of fat tissue and blood vessels, wherein the blood vessels support the fat tissue.
[Fig. 5]
Fig. 5 depicts the transplantation region of a transplant 4 weeks after transplantation, which is obtained by transplanting a liver tissue graft containing added NELL-1 (5μg) in the presence of fat tissue and blood vessels, wherein the blood vessels support the fat tissue. The region enclosed by a broken line is the transplanted liver graft. The

bar indicates a length of 1.0 mm.
[Fig. 6A]
Fig. 6A depicts the transplantation region of a transplant 2 weeks after transplantation, which is obtained by transplanting an *in vivo* incubator containing only added FGF in the presence of fat tissue and blood vessels, wherein the blood vessels support the fat tissue. The bar indicates a length of 250 μm.
[Fig. 6B]
Fig. 6B depicts an expanded view of Fig. 6A. The bar indicates a length of 500 μm. A: connective tissue, B: blood vessel.
[Fig. 6C]
Fig. 6C depicts the transplantation region of a transplant 2 weeks after transplantation, which is obtained by transplanting an *in vivo* incubator containing added NELL-1 and FGF in the presence of fat tissue and blood vessels, wherein the blood vessels support the fat tissue. The bar indicates a length of 250 μm.
[Fig. 6D]
Fig. 6D depicts an expanded view of Fig. 6C. The bar indicates a length of 500 μm. A: connective tissue, B: differentiated glandular system.
[Fig. 6E]
Fig. 6E depicts a photograph showing the transplantation region of a transplant 2 weeks after transplantation, which is obtained by transplanting an *in vivo* incubator containing only NELL-1 in the presence of fat tissue and blood vessels wherein the blood vessels support the fat tissue. The bar indicates a length of 250 μm.
[Fig. 6F]
Fig. 6F depicts an expanded view of Fig. 6E. The bar indicates a length of 500 μm. The arrows indicates a glandular system.
[Fig. 6G]
Fig. 6G depicts a photograph showing the transplantation region of a transplant 2 weeks after transplantation, which is obtained by transplanting an *in vivo* incubator containing only added physiological saline in the presence of fat tissue and blood vessels wherein the blood vessels support the fat tissue. The bar indicates a length of 250 μm.
[Fig. 6H]
Fig. 6H depicts an expanded view of Fig. 6G. The bar indicates a length of 500 μm. Arrow: connective tissue present surrounding the fat tissue.
[Fig. 7]
Fig. 7 depicts a group in which NELL-1 was administered externally into the mold continuously.
[Fig. 8]
Fig. 8 shows the results of a test examining the effectiveness of the continuous administration of NELL-1 externally into the mold and then injecting a diluted hematoxylin solution externally. The experiment confirmed that the administration of NELL-1 was successful. The site where the diluted hematoxylin solution was injected is stained purple.
[Fig. 9]
Fig. 9 is a photograph after hematoxylin-eosin staining showing the hair, hair bulb, hair root and hair root-associated glandular system formed in the presence of fat tissue and blood vessels, wherein the blood vessels support the fat tissue. The bar indicates a length of 500 μm. A: hair root-associated glandular system, B: hair and hair root (hair bulb).

SEQUENCE LISTING FREE TEXT

SEQ ID NO.: 1 is the nucleic acid sequence of human NELL1. Its signal peptide-coding region corresponds to bases 135 to 182, while the mature peptide-coding region corresponds to bases 183 to 2564.
SEQ ID NO.: 2 is the amino acid sequence of human NELL1. The signal peptide corresponds to residues 1 to 16, while the mature peptide corresponds to residues 17 to 810.
SEQ ID NO.: 3 is the nucleic acid sequence of mouse NELL1. The signal peptide-coding region corresponds to bases 40 to 102, while the mature peptide-coding region corresponds to bases 103 to 2472.
SEQ ID NO. : 4 is the amino acid sequence of mouse NELL1. The signal peptide corresponds to residues 1 to 21, while the mature peptide corresponds to residues 22 to 810.
SEQ ID NO. : 5 is the nucleic acid sequence of rat NELL1. The signal peptide-coding region corresponds to bases 59 to 121, while the mature peptide-coding region corresponds to bases 122 to 2491.
SEQ ID NO.: 6 is the amino acid sequence of rat NELL1. The signal peptide corresponds to residues 1 to 21, while the mature peptide corresponds to residues 22 to 810.
SEQ ID NO.: 7 is the sequence of the vector used in the Examples.
SEQ ID NO.: 8 is the nucleic acid sequences of the V5 tag and the histidine tag (His tag).

SEQ ID NO.: 9 is the amino acid sequences of the V5 tag and the histidine tag (His tag).
SEQ ID NO.: 10 is the amino acid sequence of human NELL1's original signal peptide.
SEQ ID NO.: 11 is the amino acid sequence of honey bee mellitin's signal peptide.
SEQ ID NO.: 12 is the nucleic acid sequence of *Xenopus laevis* NELL1.
SEQ ID NO.: 13 is the amino acid sequence of *Xenopus laevis* NELL1.
SEQ ID NO.: 14 is the amino acid sequence of the hNELL1 fragment.
SEQ ID NO.: 15 is the amino acid sequence of the hLAMA3 fragment.
SEQ ID NO.: 16 is the amino acid sequence of the mLAMA3 fragment.
SEQ ID NO.: 17 is the amino acid sequence of the hCOLA1 fragment.
SEQ ID NO.: 18 is the amino acid sequence of the hTSP1 fragment.
SEQ ID NO.: 19 is the sequence of forward primer #2.
SEQ ID NO.: 20 is the sequence of forward primer #3.
SEQ ID NO.: 21 is the sequence of forward primer #4.
SEQ ID NO.: 22 is the sequence of forward primer #5.
SEQ ID NO.: 23 is the sequence of reverse primer #2.
SEQ ID NO.: 24 is the sequence of reverse primer #3.
SEQ ID NO.: 25 is the sequence of reverse primer #4.
SEQ ID NO.: 26 is the sequence of reverse primer #5.
SEQ ID NO.: 27 is the sequence of the V5 tag.
SEQ ID NO.: 28 is the sequence of the histidine tag.
SEQ ID NO.: 29 is the sequence of the tag region used in the Examples.
SEQ ID NO.: 30 is the sequence of forward primer #1.
SEQ ID NO.: 31 is the sequence of reverse primer #1.

BEST MODES FOR CARRYING OUT THE INVENTION

Hereinafter, the present invention will be described. It should be understood that in the entirety of the present description, a term expressed in the singular form includes the concept of the term in the plural form, unless specified otherwise. Thus, it should be understood that unless specified otherwise, a term with an article in the singular form (such as "a", "an", or "the" in English) has the concept of the term in the plural form. It should also be understood that unless specified otherwise, the terms used in the present description are used in the meaning commonly used in the art. Thus, unless specified otherwise, all professional terms and all scientific and technical terms used in the present description have the meaning generally recognized by those skilled in the art. If there is contradiction, preference is given to the present description (including definitions).

(Definition of terms)

Hereinafter, definition of terms used particularly in the present description will be listed.
The term "NELL-1", as used herein, is a gene found by Ting, K. *et al.*, and the NELL-1 gene was first identified as a gene causing craniosynostosis (Patent Documents 1 to 4, etc.). The inventors had found NELL-1 as a protein by using the Yeast Two Hybrid method (Kuroda, S., Oyasu, M., Kawakami, M., Kanayama, N., Tanizawa, K., Saito, N., Abe, T., Matsuhashi, S. and Ting, K. (1999) Biochem. Biophys. Res. Commun. 265, 79-86). Later, when a mouse expressing the NELL-1 gene in an excess amount was prepared, a craniosynostosis-like phenotype was identified. Furthermore, when the NELL-1 gene was introduced into a mouse precursor osteoblast MC3T3-E1 by using adenovirus, an increase in the activity of intracellular alkali phosphatase, an early-phase bone differentiation marker, was observed, and it is also known that expression of mRNAs of osteopontin and osteocalsin, medium- to late-phase markers, was increased in the differentiated cells and that cells containing the introduced NELL-1 gene finally induced buildup of calcium (Zhang, X., Kuroda, S., Carpenter, D., Nishimura, I., Soo, C., Moats, R., Iida, K., Wisner, E., Hu, F. Y., Miao, S., Beanes, S., Dang, C., Vastardis, H., Longaker, M., Tanizawa, K., Kanayama, N., Saito, N. and Ting, K. (2002) J. Clin. Invest. 18, 2126-2134). As used herein, "NELL-1" and "NELL1" can be used interchangeably.
The "substance which is altered to function as NELL-1", as used herein, is a substance that can produce NELL-1. For example, substances such as plasmids and viral vectors, from which NELL-1 can be expressed by gene engineering technology, may be used in the composition and the like according to the present invention.
As used herein, the term "NELL-1" or "NELL1" generally refers to that of the mature sequence. Thus, in the present invention, care should be given to the fact that "NELL-1" or "NELL1" usually does not contain a signal sequence (leader sequence). The nucleotide sequences of NELL1 can be specifically represented by the following sequences:

(1) amino acid sequences represented by SEQ ID NO.: 2, 4, 6 or 13 (amino acid sequences of NELL1);

(2) amino acid sequences represented by SEQ ID NO.: 2, 4, 6 or 13 (amino acid sequences of NELL1), containing one or more substitutions, additions and/or deletions and showing the action of natural NELL1;
(3) amino acid sequences having a homology of at least 70% with the amino acid sequence represented by SEQ ID NO.: 2, 4, 6 or 13 (amino acid sequences of NELL1);
(4) amino acid sequences coded by a nucleic acid sequence hybridizing with the nucleic acid sequence represented by SEQ ID NO.: 1, 3, 5 or 12 (nucleic acid sequences encoding NELL1) or a nucleic acid sequence complementary thereto under stringent conditions;
(5) amino acid sequences coded by a nucleic acid sequence hybridizing with the nucleic acid sequence represented by SEQ ID NO.: 1, 3, 5 or 12 (nucleic acid sequences encoding NELL1) or a nucleic acid sequence complementary thereto under moderately stringent conditions;
(6) amino acid sequences coded by a nucleic acid sequence having a homology of at least 70% with the nucleic acid sequence represented by SEQ ID NO.: 1, 3, 5 or 12 (nucleic acid sequences encoding NELL1); and
(7) homolog or allelic variants of (1) to (6).

The nucleic acids encoding NELL1 or the NELL1 genes can be represented by the following sequences:

(1) nucleic acid sequences coding the amino acid sequence of SEQ ID NO.: 2, 4, 6 or 13 (amino acid sequences of NELL1);
(2) nucleic acid sequences coding the amino acid sequence represented by SEQ ID NO.: 2, 4, 6 or 13 (amino acid sequences of NELL1) that have one or more substitutions, additions and/or deletions and include the sequence responsible for the activity of natural NELL1;
(3) nucleic acid sequences having an amino acid sequence having a homology of at least 70% with the amino acid sequence represented by SEQ ID NO.: 2, 4, 6 or 13 (amino acid sequences of NELL1);
(4) nucleic acid sequences that hybridize with a nucleic acid sequence represented by SEQ ID NO.: 1, 3, 5 or 12 (nucleic acid sequences encoding NELL1) or a nucleic acid sequence complementary thereto under stringent conditions;
(5) nucleic acid sequences hybridizing with the nucleic acid sequence represented by SEQ ID NO.: 1, 3, 5 or 12 (nucleic acid sequences encoding NELL1) or a nucleic acid sequence complementary thereto under moderately stringent conditions;
(6) nucleic acid sequences having a homology of at least 70% with the nucleic acid sequence represented by SEQ ID NO.: 1, 3, 5 or 12 (nucleic acid sequences encoding NELL1); and
(7) homologs or allelic variants of the sequences (1) to (6).

SEQ ID NO.: 1, among the sequence identification numbers above, represents the nucleic acid sequence of human NELL1. The signal peptide-coding region corresponds to bases 135 to 182, while the mature peptide-coding region corresponds to bases 183 to 2564. SEQ ID NO.: 2 is the amino acid sequence of human NELL1. The signal peptide corresponds to residues 1 to 16, while the mature peptide corresponds to residues 17 to 810.
SEQ ID NO.: 3 is the nucleic acid sequence of mouse NELL1. The signal peptide-coding region corresponds to bases 40 to 102, while the mature peptide-coding region corresponds to bases 103 to 2472. SEQ ID NO.: 4 is the amino acid sequence of mouse NELL1. The signal peptide corresponds to residues 1 to 21, while the mature peptide corresponds to residues 22 to 810.
SEQ ID NO.: 5 is the nucleic acid sequence of rat NELL1. The signal peptide-coding region corresponds to bases 59 to 121, while the mature peptide-coding region corresponds to bases 122 to 2491. SEQ ID NO. : 6 is the amino acid sequence of rat NELL1. The signal peptide corresponds to residues 1 to 21, while the mature peptide corresponds to residues 22 to 810.
Thus, it is understood that it is possible for those skilled in the art to use the information on the signal sequence and mature sequence in the present invention.
Although SEQ ID Nos. 12 and 13 are partial sequences, it is understood that it is possible for those skilled in the art to identify the signal and mature sequences thereof (i.e., polypeptides of the corresponding amino acids in the mature region or nucleic acid sequences coding the same) and use them in the present invention, based on the information above.
The terms "protein", "polypeptide", "oligopeptide" and "peptide" are used in the same meaning in the present description, and mean a polymer of amino acids having an arbitrary length. The polymer may be linear, branched or cyclic. The amino acids may be natural, unnatural or modified. The terms can also include complexes of multiple assembled polypeptide chains. The terms also include natural or artificially modified amino acid polymers. Examples of such modification include disulfide bond formation, glycosylation, lipidation, acetylation, phosphorylation and any other operation or modification (such as binding with a labeling component). The definition also includes, for example, polypeptides containing one or more amino acid analogues (for example, those containing unnatural amino acids), peptide-like

compounds (for example, peptoids) and other modification products known in the art.

As used herein, the "amino acid" may be natural or unnatural, as long as the amino acid allows achievement of the object of the present invention.

As used herein, the "amino acid derivative" or "amino acid analogue" is an amino acid that is different from a natural amino acid but has functions similar to those of the original amino acid. Such amino acid derivatives and amino acid analogues are known in the art. Amino acid derivatives and amino acid analogues can be used as an alternative in the present description, as long as they provide biological functions similar to those of amino acids.

As used herein, "natural amino acids" mean the L-isomers of natural amino acids in the present description. Natural amino acids are glycine, alanine, valine, leucine, isoleucine, serine, methionine, threonine, phenylalanine, tyrosine, tryptophan, cysteine, proline, histidine, aspartic acid, asparagine, glutamic acid, glutamine, γ-carboxyglutamic acid, arginine, ornithine and lysine. All amino acids used in the present description are L-isomers, unless specified otherwise, but polypeptides containing D-amino acids are also included in the scope of the present invention.

As used herein, the "unnatural amino acid" means an amino acid normally not found in natural proteins. Examples of the unnatural amino acids include norleucine, para-nitrophenylalanine, homophenylalanine, para-fluorophenylalanine, 3-amino-2-benzylpropionic acid, D- or L-homoarginine and D-phenylalanine.

As used herein, the "amino acid analogue" is a molecule that is not an amino acid but has physical properties and/or functions similar to those of an amino acid. Examples of the amino acid analogues include ethionine, canavanine, and 2-methylglutamine. An amino acid mimic is a compound having a structure different from the general chemical structure of amino acids but functioning in a manner similar to the case of a natural amino acid.

As used herein, the term "nucleic acid" is used interchangeably with gene, cDNA, mRNA, oligonucleotide, and polynucleotide. A particular nucleic acid sequence includes "splice variants". Similarly, a particular protein coded by a nucleic acid tacitly includes any other proteins coded by the splice variant of the nucleic acid. A "splice variant", as it literally suggests, means a product obtained by alternative splicing of a gene. After transcription, the first nucleic acid transcript can be spliced into a different (another) spliced nucleic acid product coding different polypeptides. The mechanism of producing the splice variant varies, but it includes alternative splicing of the exons. Different polypeptides derived from the same nucleic acid by read-through transcription are also included in the definition. Any products generated by splicing reactions (including splice products in the recombinant form) are included in the definition. Alternatively, allelic variants are also included in this range.

As used herein, the terms "polynucleotide", "oligonucleotide" and "nucleic acid" are used in the same meaning, and mean a nucleotide polymer of any length. The term also includes "oligonucleotide derivatives" or "polynucleotide derivatives". The "oligonucleotide derivatives" or the "polynucleotide derivatives" are oligonucleotides or polynucleotides containing nucleotide derivatives or nucleotide bond(s) different from normal bonds. These terms are used interchangeably. Specific examples of the oligonucleotides include 2'-O-methyl-ribonucleotide, oligonucleotide derivatives in which the phosphate diester bonds in the oligonucleotide are converted to phosphorothioate bonds, oligonucleotide derivatives in which the phosphate diester bonds in the oligonucleotide are converted to the N3'-P5' phosphoramidate bonds, oligonucleotide derivatives in which the ribose-phosphate diester bonds in the oligonucleotide are converted to peptide-nucleic acid bonds, oligonucleotide derivatives in which the urasils of the oligonucleotide are substituted by C-5 propynyluracils, oligonucleotide derivatives in which the urasils of the oligonucleotide are substituted by C-5 thiazole uracils, oligonucleotide derivatives in which the cytosines of the oligonucleotide are substituted by C-5 propynylcytosine, oligonucleotide derivatives in which the cytosines in the oligonucleotide are substituted by phenoxazine-modified cytosines, oligonucleotide derivatives in which the riboses in the DNA are substituted by 2'-O-propylribose, and oligonucleotide derivatives in which the riboses in the oligonucleotide are substituted by 2'-methoxyethoxyriboses. A particular nucleic acid sequence includes, similarly to a sequence explicitly indicated, its conservatively modified variants (for example, degenerate codon substitution derivatives) and sequences complementary thereto, unless specified otherwise. Specifically, degenerate codon substitution derivatives are prepared by forming a sequence in which the 3rd positions of one or more selected (or all) codons are replaced with mixed bases and/or a deoxyinosine residue (Batzer et al., Nucleic Acid Res. 19: 5081 (1991); Ohtsuka et al., J. Biol. Chem. 260: 2605-2608 (1985); Rossolini et al., Mol. Cell. Probes 8: 91-98 (1994)).

As used herein, the "nucleotide" may be natural or unnatural. The "nucleotide derivative" or "nucleotide analogue" is a derivative or analogue that is different from a natural nucleotide but has functions similar to those of the original nucleotide. Such nucleotide derivatives and analogues are known in the art. Examples of the nucleotide derivatives and analogues include, but are not limited to, phosphorothioate, phosphoramidate, methylphosphonate, chiral methylphosphonate, 2'-O-methylribonucleotide and peptide nucleic acids (PNAs).

As used herein, the term "retrieve" means the act of identifying other nucleic acid base sequences having a particular function and/or property by using a nucleic acid base sequence by means of an electronic or biological method or the like. Electronic retrieval methods include, but are not limited to, BLAST (Altschul et al., J. Mol. Biol. 215: 403-410 (1990)), FASTA (Pearson & Lipman, Proc. Natl. Acad. Sci., USA 85: 2444-2448 (1988)), Smith and Waterman method (Smith and Waterman, J. Mol. Biol. 147: 195-197 (1981)), and Needleman and Wunsch method (Needleman and Wunsch,

J. Mol. Biol. 48: 443-453 (1970)). Biological retrieval methods include, but are not limited to, stringent hybridization, macroarray by using, for example, a nylon membrane carrying genome DNA attached thereto or microarray by using a glass plate carrying the same (microarray assay), PCR, and in-situ hybridization. As used herein, it is intended that the corresponding genes identified by the electronic or biological retrieval method are also included in the genes for use in the present invention.

As used herein, the "stringent condition" for hybridization means a condition with which the complementary chain of a nucleotide chain having a similarity or homology with the target sequence can hybridize to the target sequence preferentially and the complementary chain of a nucleotide chain having no similarity or homology does not substantially hybridize. The "complementary chain" of a nucleic acid sequence is a nucleic acid sequence that pairs with the sequence, based on the hydrogen bonds between the bases (for example, T to A and C to G). The stringent condition is dependent on the sequence and varies according to various situations. Longer sequences hybridize specifically at higher temperatures. Generally, the temperature of the stringent condition is selected to be about 5°C lower than the thermal melting temperature (Tm) of a particular sequence at specified ionic strength and pH. $T_m$ is a temperature at which 50% of the nucleotides complementary to a target sequence hybridize to the target sequence at equilibrium under specified ionic strength, pH, and nucleic acid concentration. The "stringent condition" is dependent on the sequence and varies according to various environmental parameters. General indications on nucleic acid hybridization are found in Tijssen (Tijssen (1993), Laboratory Techniques In Biochemistry And Molecular Biology-Hybridization With Nucleic Acid Probes Part I, Chapter 2, "Overview of principles of hybridization and the strategy of nucleic acid probe assay", Elsevier, New York).

Typically, for the stringent condition, the salt concentration is less than about 1.0 M $Na^+$; the $Na^+$ concentration (or other salts) is typically about 0.01 to 1.0 M at a pH of 7.0 to 8.3; and the temperature is at least about 30°C for short nucleotides (for example, with 10 to 50 nucleotides) and at least about 60°C for long nucleotides (for example, nucleotides longer than 50 nucleotides). The stringent condition can also be formed by addition of a destabilizer such as formamide. The stringent condition in the present description is, for example, hybridization in a buffered solution of 50% formamide, 1 M NaCl and 1% SDS (37°C) and washing at 0.1 x SSC at 60°C.

As used herein, the "polynucleotide that hybridizes under stringent conditions" is a condition commonly used in the art. It is possible to obtain such a polynucleotide by using a polynucleotide selected from the polynucleotides according to the present invention as a probe, for example, by a colony-hybridization method, a plaque-hybridization method or a Southern blot hybridization method. Specifically, it means a polynucleotide that can be identified by performing hybridization by using a filter carrying a colony- or plaque-derived DNA immobilized thereon in the presence of 0.7 to 1.0 M NaCl at 65°C and washing the filter by using 0.1x to 2x concentrated SSC (saline-sodium citrate) solution (the composition of the 1x concentrated SSC solution is 150 mM sodium chloride and 15 mM sodium citrate) under the condition of 65°C. The hybridization can be carried out by one of the methods described in experimental books such as Molecular Cloning 2nd ed., Current Protocols in Molecular Biology, Supplement 1 to 38, DNA Cloning 1: Core Techniques, A Practical Approach, Second Edition, Oxford UniversityPress (1995). Preferably, sequences including only A sequence or T sequence are eliminated from the sequence hybridizing under stringent condition. The "hybridizable polynucleotide" is a polynucleotide that can hybridize with another polynucleotide under the above hybridization conditions. Typical examples of the hybridizable polynucleotides include polynucleotides having a homology of 60% or more with the base sequence of the DNA coding the polypeptide having an amino acid sequence specified in the present invention, preferably polynucleotides having a homology of 80% or more, more preferably polynucleotides having a homology of 90% or more, and more preferably polynucleotides having a homology of 95% or more.

As used herein, the "highly stringent conditions" are conditions that are designed to allow hybridization of a nucleic acid sequence with a DNA chain highly complimentary thereto and prohibit hybridization thereof with a DNA having mismatches in a statistically significant amount. The hybridization stringency is determined mainly according to the conditions such as temperature, ionic strength and the modifying agent such as formamide. Examples of the "highly stringent condition" for hybridization and washing include 0.0015 M sodium chloride and 0.0015 M sodium citrate at 65 to 68°C and 0.015 M sodium chloride, 0.0015 M sodium citrate and 50% formamide at 42°C. See, for details of the highly stringent condition, Sambrook et al., Molecular Cloning: A Laboratory Manual, 2nd Ed., Cold Spring Harbor Laboratory (Cold Spring Harbor, N. Y. 1989); and Anderson et al., Nucleic Acid Hybridization: a Practical approach, IV, IRL Press Limited (Oxford, England). Limited, Oxford, England. A more stringent condition (for example, higher temperature, lower ionic strength, higher formamide concentration, or other modifying agents) may be used, as needed. Other agents may be contained in the hybridization buffer solution and the washing buffer solution for reduction of non-specific hybridization and/or background hybridization. Examples of such other agents include 0.1% bovine serum albumin, 0.1% polyvinylpyrrolidone, 0.1% sodium pyrophosphate, 0.1% sodium dodecylsulfate ($NaDodSO_4$ or SDS) , Ficoll, Denhardt solution, ultrasonicated salmon sperm DNA (or other non-complementary DNAs) and dextran sulfate, but other suitable agents can also be used. The concentration and the type of these additives can be varied without substantial influence on the stringency of hybridization conditions. The hybridization experiment is carried out normally at pH 6.8 to 7.4, but the hybridization rate is almost pH-independent under typical ionic strength conditions. See Anderson et al., Nucleic Acid Hybridization: a Practical Approach, Chapter 4, IRL Press Limited (Oxford, England).

Factors influencing the stability of double-stranded DNAs include the composition and length of the bases and the degree of base pair mismatch. The hybridization condition can be adjusted by those skilled in the art, and hybridization of DNAs dependent on different sequences is made possible by modification of these variables. The melting temperature of a completely matched double-stranded DNA can be calculated approximately according to the following Formula:

$$T_m\ (°C) = 81.5+16.6(\log[Na^+])+0.41(\%G+C)-600/N-0.72\ (\%\ formamide)$$

In the formula, N represents the length of the double strand formed; $[Na^+]$ represents the molar concentration of sodium ion in the hybridization solution or washing solution; and %G+C represents the percentage of the (guanine and cytosine) bases in the hybrid. The melting temperature of an incompletely matched hybrid decreases by about 1°C for every 1% content of mismatch.

As used herein, "moderately stringent conditions" are conditions under which double-stranded DNAs having a base pair mismatch greater than that obtained under the "highly stringent condition" can be formed. A typical example of "moderately stringent conditions" is 0.015 M sodium chloride and 0.0015 M sodium citrate at 50 to 65°C, or 0.015 M sodium chloride, 0.0015 M sodium citrate and 20% formamide, at 37 to 50°C. For example, "moderately stringent" conditions of 0.015 M sodium ion at 50°C allows about 21% mismatch.

As used herein, it is known to those skilled in the art that there is no completely distinct difference between the "highly" stringent condition and the "moderately" stringent condition. For example, the melting temperature of a completely matched long DNA is about 71°C at 0.015 M sodium ion (without formamide). Washing at 65°C (at the same ionic strength) allows mismatch in an amount of about 6%. Those skilled in the art can simply lower the temperature or increase the ionic strength, in order to capture more mismatched related sequences.

The approximate melting temperature of an oligonucleotide probe with up to about 20 nucleotides at 1 M NaCl can be calculated as follows:

$$Tm = (2°C\ per\ A\text{-}T\ base\ pair) + (4°C\ per\ G\text{-}C\ base\ pair)$$

The sodium ion concentration of 6x sodium citrate salt (SSC) is 1 M (see Suggs et al., Developmental Biology Using Purified Genes, page 683, Brown and Fox (Ed.) (1981)).

The nucleic acid coding the polypeptide having the amino acid sequence of SEQ ID NO.: * or the variant or fragment thereof can hybridize to one of the sequences shown in the sequence listing or a part thereof, under a low stringent conditions defined by a hybridization buffer solution essentially containing 1% bovine serum albumin (BSA); 500 mM sodium phosphate ($NaPO_4$) 1 mM EDTA; and 7% SDS at a temperature of 42°C and a washing buffer solution essentially containing 2xSSC (600 mM NaCl; 60 mM sodium citrate); and 0.1% SDS at 50°C, more preferably under low stringent conditions defined by a hybridization buffer solution essentially containing 1% bovine serum albumin (BSA); 500 mM sodium phosphate ($NaPO_4$); 15% formamide; 1 mM EDTA; and 7% SDS at a temperature of 50°C and a washing buffer solution essentially containing 1xSSC (300 mM NaCl; 30 mM sodium citrate); and 1% SDS at 50°C, most preferably under low stringent conditions defined by a hybridization buffer solution, essentially containing 1% bovine serum albumin (BSA); 200 mM sodium phosphate ($NaPO_4$); 15% formamide; 1 mM EDTA; and 7% SDS at a temperature of 50°C and a washing buffer solution essentially containing 0.5xSSC (150 mM NaCl; 15 mM sodium citrate); and 0.1% SDS at 65°C.

Amino acids are indicated in the present description by commonly known 3-character symbols or by 1-character symbols recommended by the IUPAC-IUB Biochemical Nomenclature Commission. Nucleotides are also indicated similarly by generally recognized 1-character codes.

As used herein, the "homology" of a gene is the degree of identity between two or more gene sequences. Thus, when the homology of two genes is high, the sequences are more identical or similar to each other. It is possible to determine whether two kinds of genes have homology, by direct comparison of the sequences or by hybridization under a stringent condition in the case of nucleic acids. When two gene sequences are compared directly, if the DNA sequences are identical to each other typically at a rate of at least 50% between the gene sequences, preferably at a rate of at least 70%, more preferably at a rate of at least 80%, 90%, 95%, 96%, 97%, 98% or 99%, these genes have homology.

As used herein, the similarity, identity and homology of amino acid or base sequence is calculated by using a sequence analysis tool BLAST and the default parameters thereof. The identity can be retrieved, for example, by using NCBI's BLAST 2.2.9 (published on May 12, 2004). The identity value as used herein is normally a value obtained by

using BLAST that is aligned under the default condition. However, if the value becomes larger by modification of the parameters, the largest value is used as the value of identity. When the identity is evaluated in multiple regions, the largest value is used as the value of identity.

As used herein, the "corresponding" amino acid means an amino acid in a protein or polypeptide molecule that has or is expected to have an action similar to that of a predetermined amino acid in the protein or polypeptide to be compared.

As used herein, the "corresponding" gene is a gene in a species that has or is expected to have an action similar to that of a predetermined gene in the species to be compared, and if there are multiple genes having such an action, it is a gene having the same evolutionary origin. Thus, the gene corresponding to a gene (for example, NELL1) can be an ortholog of the gene. Thus, the gene corresponding to a human gene can be found in other animals (mouse, rat, pig, rabbit, guinea pig, bovine, sheep and others). Such corresponding genes can be identified by a method known in the art. Thus, for example, the corresponding gene in an animal can be identified by retrieving the databases of the sequences of the animal (for example, mouse, rat, pig, rabbit, guinea pig, bovine, or sheep) by using the sequence of the gene that is the standard for the corresponding gene as a query sequence.

As used herein, when there is a full-length polypeptide or polynucleotide (having a length of n), the "fragment" means a polypeptide or polynucleotide having a sequence length of 1 to n-1. The length of the fragment can be altered properly according to its application, and for example, the shortest length, in the case of a polypeptide, is 3, 4, 5, 6, 7, 8, 9, 10, 15, 20, 25, 30, 40, 50, or more amino acids, and a length not specifically mentioned above represented in an integer (for example, 11) can also be suitable as the shortest length. Furthermore, in the case of a polynucleotide, it is, for example, 5, 6, 7, 8, 9, 10, 15, 20, 25, 30, 40, 50, 75, 100 or more nucleotides, and a length not specifically mentioned above represented in an integer (for example, 11) can also be suitable as the shortest length. As used herein, the length of polypeptide or polynucleotide can be indicated by the number of amino acids or nucleic acids, as described above, but the numbers described above are not absolute, and the number indicated as the longest or shortest value is intended to include the numbers in the range of several-unit shorter and longer (or for example $\pm$10%). In order to indicate such an intension, a term "about" may be used before the number in the present description. However, in the present description, it should be understood that the presence or absence of the term "about" has no influence on interpretation of the number. The length of a fragment useful in the present description can be determined by whether the fragment has at least one function among the functions of the full-length protein, which is the standard of the fragment.

As used herein, the term "homology" means that two or more sequences have the same evolutionary ancestor, when the sequences are compared. It is possible to determine whether two kinds of genes have a homology, based on the similarity obtained after direct comparison of the sequences or by hybridization under stringent conditions. When the homology is to be determined based on the similarity obtained by direct comparison of the sequences, the simplest interpretation of the alignment in the similarity measurement process is that: when the same (or equivalent) character string (e.g. bases or amino acid residues) is preserved in different sequences, these regions remain unchanged from their ancestor sequence, and, when these character strings are not the same (or not equivalent), the character string in one sequence occurred by mutation. A gap (indel) between the sequences in alignment is considered to be the result of insertion or deletion at a site of the sequence. Thus, high identity or similarity between the sequences is considered to strongly suggest homology of the sequences. The homology is also referred to in designing an expression inhibitor.

As used herein, the term "complementary" or "complement" means the sequence of a polynucleotide that can form Watson & Crick base pairs with another particular polynucleotide in the entire complementary region. According to the present invention, when each of the bases in the first polynucleotide forms a base pair with the complementary base, the first polynucleotide is considered to be complementary to the second polynucleotide. Complementary bases are generally A and T (or, A and U) or C and G. As used herein, the term "complementary" is used as a synonym of "complementary polynucleotide", "complementary nucleic acid" and "complementary nucleotide sequence". These terms are used for polynucleotide pairs, based only on their sequences, and not used for a particular set of two polynucleotides practically in the bonded state.

As used herein, a "variant" is a derivative of a polypeptide or a polynucleotide, in which part of the substance is altered. Such variants include substitution variants, addition variants, deletion variants, truncated variants, and allelic variants. Allelic genes (alleles) are genetic variants that belong to the same gene locus but are different from each other. Thus, an "allelic variant" is a variant allele to a gene. The "species homolog or homolog" is a species homologous with a gene at the amino acid or nucleotide level with a homology (of preferably 60% or more, more preferably 80% or more, 85% or more, 90% or more, or 95% or more). The method of obtaining such a species homolog is obvious from the description in the present description. The term "orthologs", which is also called orthologous genes, are two genes generated by species differentiated from a common ancestor. For example, in the case of a hemoglobin gene family having a multiplex gene structure, human and mouse $\alpha$ hemoglobin genes are orthologs, but human $\alpha$ hemoglobin gene and $\beta$ hemoglobin gene are paralogs (genes generated by gene duplication). Orthologs are useful for estimation of molecular phylogenetic tree and thus, orthologs can also be useful in the present invention.

As used herein, the term "conservative (conservatively modified) variant" is used both for amino acid and nucleic

acid sequences. A conservatively modified variant of a particular nucleic acid sequence is a nucleic acid coding the same or essentially the same amino acid sequence, and it is an essentially identical sequence, when the nucleic acid does not code any amino acid sequence. Such methods of modifying a base sequence include cleavage by restriction enzyme or the like, binding by treatment with DNA polymerase, Klenow fragment, DNA ligase or the like, and site-specific base substitution method by using a synthetic oligonucleotide or the like (site-specific mutagenesis; Mark Zoller and Michael Smith, Methods in Enzymology, 100, 468-500 (1983)), but the modification can be made by other methods normally used in the field of molecular biology. Because of degeneration of genetic codes, multiple functionally identical nucleic acids code a certain protein. For example, all of the codons GCA, GCC, GCG and GCU code the amino acid alanine. Thus, the codon can be changed to any other corresponding codon described above at all positions where alanine is specified by the codon without modification of the polypeptide encoded. Such variation of nucleic acid is a "silent modification (mutation)", which is a kind of conservatively modified mutation. All nucleic acid sequences coding a polypeptide in the present description include all possible silent mutation of the nucleic acid. Obviously for those experienced in the art, each codon in a nucleic acid (excluding the only codon AUG normally for methionine and the only codon TGG normally for tryptophan) can be modified for production of a functionally identical molecule. Thus, silent mutations of the nucleic acid encoding a polypeptide is included tacitly in the sequence described. Preferably, such modification is made so as to avoid substitution of cysteine, an amino acid having a significant influence on the higher-order structure of polypeptide.

An amino acid can be replaced with another amino acid in a protein structure such as ligand molecule-binding site without distinct deterioration or loss of interactive bonding ability. The biological function of a protein is governed by the interactive ability and the property of the protein. Thus, even if a particular amino acid is replaced in an amino acid sequence or at the level of a DNA coding sequence, there is a possibility of a protein having the original property even after the replacement. Thus, various modifications can be possibly done without a distinct loss of biological usefulness on the peptides or the corresponding DNAs encoding the peptides disclosed herein.

The hydrophobicity index of amino acids is considered in designing such a modification. Importance of the amino acid hydrophobicity index in interpreting the interactive biological function of a protein is well recognized in the art (Kyte. J and Doolittle, R. F. J. Mol. Biol. 157 (1): 105-132, 1982). The hydrophobic property of amino acids contributes to the secondary structure of the protein generated and then determines the interaction of the protein with other molecules (for example, enzymes, substrates, receptors, DNAs, antibodies, and antigens). Each amino acid has a hydrophobicity index allocated, based on its hydrophobicity and charge property, as shown below: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

It is well known in the art that, replacement with amino acids having a similar hydrophobicity index maintains a similar biological function (for example, a protein equivalent in ligand-binding potential). In such amino acid substitution, the difference in hydrophobicity index is preferably $\pm 2$ or less, more preferably $\pm 1$ or less, and still more preferably $\pm 0.5$ or less. It is known in the art that such amino acid substitution, based on hydrophobicity, is efficient. As described in U.S. Patent No. 4,554,101, the following hydrophilicity indices are allocated to amino acid residues: arginine (+3.0); lysine (+3.0); aspartic acid (+3.0$\pm$1); glutamic acid (+3.0$\pm$1); serine (+0.3); asparagine (+0.2);glutamine (+0.2); glycine (0); threonine (-0.4); proline (-0.5$\pm$1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). Thus, it should be understood that, even if an amino acid is replaced with another amino acid having a similar hydrophilicity index, it is still possible to give a biological equivalent. In such amino acid substitution, the difference in hydrophilicity index is preferably $\pm 2$ or less, more preferably $\pm 1$ or less, and still more preferably $\pm 0.5$ or less.

In the present invention, "conservative substitution" is an amino acid substitution, in which the hydrophilicity indices or/and hydrophobicity indices are similar between original and replacing amino acids, as described above. Examples of the conservative substitution are known to those skilled in the art and include, but are not limited to, substitutions in the following groups: arginine and lysine; glutamic acid and aspartic acid; serine and threonine; glutamine and asparagine; and valine, leucine, and isoleucine.

In the present invention, for production of a functionally equivalent polypeptide, amino acid (s) may not only be substituted but also added, deleted, or modified. Amino acid substitution means substitution of one or more, for example 1 to 10, preferably 1 to 5, more preferably 1 to 3 amino acids of an original peptide. Amino acid addition means addition of one or more, for example 1 to 10, preferably 1 to 5, more preferably 1 to 3 amino acids to an original peptide chain. Amino acid deletion means deletion of one or more, for example 1 to 10, preferably 1 to 5, more preferably 1 to 3 amino acids from an original peptide. Amino acid modifications include, but are not limited to, amidation, carboxylation, sulfation, halogenation, alkylation, phosphorylation, hydroxylation and acylation (for example, acetylation). The amino acids substituted or added may be natural amino acids, unnatural amino acids or amino acid analogues. Natural amino acids are preferable.

These nucleic acids can be prepared by the well known PCR method or by chemical synthesis. These methods

may be combined, for example, with site-specific mutagenesis method, hybridization method or the like.

As used herein, "substitution, addition and/or deletion" of a polypeptide or polynucleotide means substitution, addition and/or deletion of amino acids or alternatives thereof or nucleotides or alternatives thereof from an original polypeptide or polynucleotide. The methods for substitution, addition and/or deletion are known in the art, and examples of the methods include site-specific mutagenesis techniques. These changes in the standard nucleic acid molecule or polypeptide can occur at the 5'- or 3'-terminal of the nucleic acid molecule, the amino-terminal or carboxy-terminal of the amino acid sequence of the polypeptide, or anywhere at the sites between these terminals, as long as the intended function (for example, ossification) is preserved, and these changes are present separately between residues in the standard sequence. The number of the nucleotides or amino acids substituted, added or deleted may be any number of one or more, and the number may be larger as long the variant after substitution, addition or deletion retains the intended function (for example, AGE-recognizing ability). The number may be, for example, one or more, and preferably 20% or less, 15% or less, 10% or less, 5% or less of the entire length, or 150 or less, 100 or less, 50 or less, or 25 or less.

As used herein, the term "cell" is used in the meaning similar to the broadest sense commonly used in the art and it is a basis unit constituting an organism morphologically and/or functionally.

As used herein, the term "tissue" is used in the meaning similar to the broadest sense commonly used in the art and is a group of cells having substantially the same shape and function. In animals, the tissues are categorized into epithelial tissue, connective tissue, muscle tissue, nerve tissue and the like.

As used herein, the term "organ" is used in the meaning similar to the broadest sense commonly used in the art and, in multicellular organisms, it is a region where the tissues form a certain shape together, providing the region with a special function such as respiration, excretion or digestion. As used herein, the terms "organ" and "internal organ" are used interchangeably.

As used herein, the term "heterotopically form(ed)" means that a cell, tissue or organ is formed at a site not found originally. Examples of the heterotopical formation include, but are not limited to, formation of a hematopoietic tissue in fat tissue, formation of an epithelial tissue in fat tissue, formation of a hematopoietic tissue in muscle tissue, and formation of an epithelial tissue in muscle tissue.

As used herein, the term "support" a cell, tissue or organ means preservation of the cell, tissue or organ with its shape and at least part of its function.

As used herein, "in the presence of fat tissue and blood vessels wherein the blood vessels support the fat tissue" means presence of a fat tissue and blood vessels for preservation of the shape and function of the fat tissue. For example, it is an environment surrounding the branching region of inferior epigastric blood vessels in the subcutaneous fat tissue of the abdomen.

As used herein, "in the presence of muscle tissue and blood vessels wherein the blood vessels support the muscle tissue" means presence of a muscle tissue and the blood vessels for preservation of the shape and function of the muscle tissue. It is, for example, an environment of blood vessels in the muscle tissue of the femoral region.

As used herein, the "cell having been directed to a given differentiation" is a cell in the differentiation phase that is to be differentiated into a particular cell. Examples thereof in the present description include, but are not limited to, somatic stem cells present in tissues (such as hematopoietic tissue, epithelial tissue, muscle tissue and nerve tissue), and precursor cells for a cell constituting a tissue.

As used herein, the term "stem cell" is used in the meaning similar to the broadest sense commonly used in the art and it is a cell after cell division retaining the same differentiation potency.

As used herein, the term "somatic stem cell" is used in the meaning similar to the broadest sense commonly used in the art and it is a cell responsible for tissue construction, which was found during remodeling (absorption and reconstruction) of all tissues or organs. As used herein, the terms "somatic stem cell", "tissue stem cell" and "adult stem cell" can be used interchangeably. Examples of the somatic stem cells include, but are not limited to, somatic stem cells present in hematopoietic tissue, epithelial tissue, connective tissue, muscle tissue, nerve tissue and other tissues.

Somatic stem cells are cells differentiated in a particular direction to some extent from a pluripotent stem cell such as ES or iPS cell. Thus, the cells are named differently according to the major tissues, for example, hematopoietic stem cells, mesenchymal stem cells, and nerve stem cells. However, these somatic stem cells are also expected to dedifferentiate (backward differentiation). For example, it is reported that a donor's tissue is detected in a recipient's liver after transplantation of "hematopoietic stem cell" in a bone marrow transplantation clinically practiced (Alison MR, Hepatocytes from non-hepatic adult stem cells., Nature. 2000 Jul 20; 406 (6793): 257). Thus, not only the somatic stem cell, but also the somatic stem cells or pluripotent stem cells can differentiate into "hematopoietic stem cell", which can in turn differentiate, for example, into blood cells. The present invention can use these stem cells as needed.

Examples of somatic stem cells present in hematopoietic tissues include hematopoietic stem cells and bone marrow stem cells. Somatic stem cells present in epithelial tissue are stem cells that are to be differentiated into epithelial tissue and the mother cells for epithelial cells having self repair capability and pluripotency. Examples of the somatic stem cells present in epithelial tissue include adenoblast, and cells included in the hair root. The somatic stem cells present in epithelial tissue can differentiate, for example, into adenocytes or cells included in hair root.

As used herein, the "hematopoietic stem cell" is the mother cell for a blood cell having self repair capability and pluripotency and examples thereof include cells secreted into peripheral blood from bone marrow by activation by a cytokine such as granular colony stimulation factor (G-CSF) and cells that can differentiate into every kind of blood cells (totipotent cells).

Hematopoietic stem cells are used, for example, in treatment of patients with leukemia (for example, hematopoietic stem cell transplantation). Hematopoietic stem cell transplantation began originally with bone marrow transplantation. It was found in the late 1960’s that transplantation of bone marrow form a brother having the same HLA after high-dose chemical therapy resulted in conversion of the patient’s blood cells entirely to donor-derived blood cells, and thus, bone marrow transplantation was invented. It subsequently became clear that administration of G-CSF leads to generation of hematopoietic stem cells in peripheral blood, and peripheral blood stem cell transplantation by using the same has been practiced since the 1990’s. It was also found then that many hematopoietic stem cells are included in cord blood, and cord blood transplantation has been practiced mainly for children. The present invention can use these techniques relating to stem cells as needed.

As used herein, the term "hematopoietic tissue" is used in the meaning similar to the broadest sense commonly used in the art, and it is a tissue forming and developing various blood cells and other blood components.

As used herein, the term "hematopoietic" cell, tissue or organ is used in the meaning similar to the broadest sense commonly used in the art and it is a generic term indicating various cells, tissues or organs constituting a hematopoietic tissue.

As used herein, the term "epithelial tissue" is used in the meaning similar to the broadest sense commonly used in the art and it is a cell layer covering the surface of animal body and the internal surface of the organs of the body.

As used herein, the term "epithelial" cell, tissue or organ is used in the meaning similar to the broadest sense commonly used in the art and it is a generic term indicating epithelium-forming cells, tissues or organs.

As used herein, an "adenoblast" is a stem cell to be differentiated into epithelial tissue, which is present in the aggregate of cells (gland) having a secretory action.

As used herein, the "cells included in hair root" are cells included in the region of hair embedded in a follicle. The cells included in a hair root include cells to be differentiated into a hair root.

As used herein, the term "exocrine gland" is used in the meaning similar to the broadest sense commonly used in the art and it is a gland releasing a secretion through ducts onto the body surface. Examples of the exocrine glands include perspiratory glands, sebaceous glands, intestinal glands, gastric glands, and salivary glands. All glandular systems are formed in the shape of cylindrical or cubic epithelium having a secretion potency, and the secretion varies by tissue (organ). Thus, it is possible to obtain a desired exocrine gland by separating a secretory gland from a desired organ and placing it in the *in vivo* incubator according to the present invention.

As used herein, the term "connective tissue" is used in the meaning similar to the broadest sense commonly used in the art and it is a tissue supporting the animal body and is composed of framework components such as tissues, fibers of various cells and substrates. The connective tissue derives from mesenchyme generated from mesoderm. The connective tissue contains, for example, lymph tissues, cartilages, and bones.

As used herein, the term "muscle tissue" is used in the meaning similar to the broadest sense commonly used in the art and it is a tissue shrinkable by stimulus. Muscle tissues are grouped into skeletal muscle, cardiac muscle, and smooth muscle tissue.

As used herein, the term "nerve tissue" is used in the meaning similar to the broadest sense commonly used in the art and it is a tissue constituting the nervous system. The nerve tissue is made with nerve cells transmitting excitement (signal), neuroglia supporting the same, and others.

As used herein, the "scaffold" is a structure supporting an object, and for example, it is a spatial step structure or a base structure used for a particular action. As used herein, the scaffold is produced from a biocompatible substance, which is a biological or natural substance or a natural or synthetic substance. As used herein, the "scaffolds" are grouped into "functional scaffolds" and "sustained-releasing scaffolds".

As used herein, the "functional scaffold" is a structure used for growth of cells and tissues. In an embodiment, the functional scaffold for use in the present invention may be, for example, a tissue in the living body (for example, fat tissue). It may be called a tissue scaffold.

As used herein, the "sustained-releasing scaffold" means a material containing NELL-1 and releasing it gradually. In an embodiment, the sustained-releasing scaffold for use in the present invention may be, but is not limited to, an extracellular matrix (for example, collagen sponge or atelocollagen gel). The scaffold is not particularly limited, as long as it can hold NELL-1 and provide cells therewith for the period until the cells are converted into differentiated cells, tissue or organ the scaffold is a sustained-releasing scaffold.

As used herein, the term "extracellular matrix (ECM) " is used in the meaning similar to the broadest sense commonly used in the art, and it is a supermolecular structure filling the extracellular space. Examples of the extracellular matrices include, but are not limited to, collagen, atelocollagen, proteoglycans (for example, chondroitin sulfate proteoglycan, heparan sulfate proteoglycan, keratan sulfate proteoglycan, and dermatan sulfate proteoglycan), hyaluronic

acid, fibronectin, laminin, tenascin, entactin, elastin, andfibrillin. As used herein, the terms "extracellular matrix" and "extracellular substrate" can be used interchangeably.

As used herein, the term "collagen" is used in the meaning similar to the broadest sense commonly used in the art and it is the major component of the extracellular matrix of animals. Examples of the collagens for use in the present invention include collagen sponges, collagen gels, and atelocollagen gels, for example, available from Hakuho Corporation (supplier: Hakusui Trading Co., Ltd., Osaka), and collagens from other suppliers are also usable in the present invention.

As used herein, the "medical device" is a device used for the purpose of improving body function or of medical treatment (for example, treatment or operation). The medical device for use in the present invention is not particularly limited, as long as it can contain NELL-1 and make the NELL-1 act on a cell, tissue or organ. The medical device for use in the present invention is preferably produced from a biocompatible material in the shape and size that permit transplantation thereof into the body, and additionally, it is preferably free from immunological rejection reaction.

As used herein, the *"in vivo* incubator" is a structure containing a mold (for example, silicone mold), NELL-1 and a pedicle tissue graft. As used herein, the "pedicle tissue graft" is a tissue containing a tissue graft and the blood vessels supporting the tissue graft, in the shape not inhibiting blood flow. Examples of the pedicle tissue grafts include a fat tissue and the blood vessels supporting the same, and a muscle tissue and the blood vessels supporting the same, but any tissue other than those above may be used, as long as it satisfies the characteristics. Without wishing to be bound by theory, if oxygen and others are supplied to the tissue by blood flow, necrosis is inhibited and cells having been directed to a given differentiation are provided, the characteristics are satisfied and the tissue is a pedicle tissue graft.

The *in vivo* incubator may have the functional scaffold (for example, collagen sponge or atelocollagen gel) releasing NELL-1 gradually. A functional scaffold for the *in vivo* incubator can produce more differentiated cells and others, as long as it can supply NELL-1 to a cell (for example, somatic stem cell) over the period needed for further induction of differentiation of the cell. In other words, the NELL-1-supplying means is not particularly limited, as long as NELL-1 can be supplied to the cell continuously. In an embodiment, NELL-1 can be supplied, by direct addition (for example, by injection) to part of the pedicle tissue graft (for example, fat tissue).

The *in vivo* incubator for use in the present invention is preferably produced from a biocompatible material in the shape and size that permit transplantation thereof into the living body, and additionally, it is preferably free from immunological rejection reaction.

As used herein, the "mold" is a container that can be transplanted in the living body. As used herein, a mold produced from silicone will be called a "silicone mold". The mold can be formed in any shape according to the shape of the organ into which it is transplanted. For example, a silicone mold in the shape of rat bone tip and a clay mold in the shape of pine nut can be used. The mold is not particularly limited, as long as it can contain NELL-1 and make the NELL-1 act on a cell, tissue or organ the mold is mold in the sense of the invention. The mold for use in the present invention is preferably produced from a biocompatible material in the shape and size that permit transplantation thereof into the living body, and additionally, it is preferably free from immunological rejection reaction.

(Preferred Embodiments)

Hereinafter, the preferred embodiments of the present invention will be described. The embodiments below are provided only for better understanding of the present invention and it should be understood that the scope of the present invention is not limited to the description below. It is thus obvious that those skilled in the art can modify the present invention within the scope thereof, based on the description of the present specification.

(Composition used for induced differentiation)

In one aspect, the present invention provides a composition for inducing differentiation of a cell having been directed to a given differentiation, to a further differentiated cell, tissue or organ to the given differentiation. The composition may contain NELL-1 or a substance which is altered to function as NELL-1 at the time of the inducing differentiation. It is possible to produce any organ or the like with the composition according to the present invention. Ogawa K, (Living donor liver transplantation with reduced monosegments for neonates and small infants.: Transplantation. 2007 May 27; 83 (10): 1337-40) describes that there is a possibility of a somatic stem cell to be differentiated into liver being present in the donor or recipient liver after clinically-performed liver transplantation into a child. Without being bound by any theory, it is therefore supposed that, in the heterotopical tissue differentiation or the support and proliferation of a liver tissue graft according to the present invention, the cells present in a tissue (including transplantation graft) in contact with NELL-1 repeats remodeling and then repeats proliferation only by autologous cells or undifferentiated cells contained in the tissue graft proliferates.

In the present specification, the cell having been directed to a given differentiation may be any cell. Without being bound by any theory, NELL-1 for use in the present invention has an action to induce further differentiation of cells

having a predetermined differentiation direction and thus, any cell may be used, if the cell to be subjected has a predetermined differentiation direction.

In an embodiment, NELL-1 or a substance which is altered to function as NELL-1 at the time of the induced differentiation may be, for example, but is not limited to, a substance that can express NELL-1 using genetic engineering technology, such as plasmids or viral vectors. A substance which functions as NELL-1 is one which makes it possible to induce further differentiation of the cell having been directed to a given differentiation, if it is possible to allow NELL-1 to act to the cell in the manner thereof.

In an embodiment, the composition according to the present invention can be a composition for heterotopically forming the further differentiated cell, tissue or organ directed to the given differentiation in the presence of fat tissue and blood vessels supporting the fat tissue.

In a preferred embodiment, the environment in the presence of fat tissue and blood vessels supporting the fat tissue is, but not limited to, the periphery of the branch point of the hypogastric artery and vein in abdominal subcutaneous fat tissue.

Without being bound by theory, the reason that it is preferable in the presence of fat tissue is that differentiation of a fat tissue-derived stem cell (Zuk PA, Multilineage cells from human adipose tissue: implications for cell-based therapies. Tissue Eng. 2001 Apr; 7 (2): 211-28.) is possibly induced by NELL-1.

Somatic stem cells found in fat tissue may differentiate into mesodermal tissue (fat-producing, cartilagenous myogenic and osteogenetic cells). The present invention can be applied to other tissue types in view of the above noted reference which supports the possibility of heterotopical tissue differentiation and the support and proliferation of liver tissue grafts. However, as the range of the fat cell being differentiated is extremely limited according to the paper and thus, it is not possible to expect the formation of epithelial tissue according the present invention from the paper. Alternatively, differentiation is not always desirable in the presence of fat tissue and, for example, a muscular tissue with blood vessels (performed clinically in oral surgery) can also be placed in a silicone mold. In such a case, there are somatic stem cells to be differentiated into muscular tissue and these cells may possibly differentiate into mesodermal cells, as the cells also derive from mesoderm (muscular tissue).

However, as described in the Alison MR' paper below, it is highly probable that other somatic stem cells are fed into a tissue (fat or muscular tissue) consistently nourished through blood vessels by body fluid circulation, leading to generation of a heterotopical tissue in the silicone mold. The present invention was made by mainly embedding blood vessels and a tissue (for example, fat or muscle tissue) completely and efficiently in a capsule, and thus, the series of the papers above are not essential to the present invention and they only provide information needed in considering the "novel results" generated in the test results (tissue image) obtained by the present invention.

Without being bound by theory, the reason that it is preferable in the presence of blood vessels is that differentiation of somatic stem cells present in other sites is possibly induced by NELL-1 through body fluid circulation or the like, because there is reported that donor-derived cells are contained in the patient hepatic cells after bone marrow transplantation (Alison MR, Hepatocytes from non-hepatic adult stem cells., Nature.2000 Jul 20; 406 (6793): 257).

In another embodiment, the cell having been directed to a given differentiation for use in the present invention may be a somatic stem cell, for example a somatic stem cell present in hematopoietic tissue, epithelial tissue, connective tissue, muscular tissue, nerve tissue or the like. Preferably, it can be a somatic stem cell present in hematopoietic tissue, epithelial tissue or connective tissue. The somatic stem cell for use in the present invention can be selected and prepared properly by those skilled in the art with reference to well-known documents or known documents.

In a preferred embodiment, the tissue (organ) to be implanted in the fat tissue in the *in vivo* incubator according to the present invention can be produced, for example, by the following method:

1. Method of inoculating cells derived from own or foreign organs (for example, liver graft)

These organs contain both mature cells and somatic stem cells.

2. Method of providing hematopoietic stem cells (distributed also in peripheral blood) via blood vessels connected to somatic stem cells present in fat tissue. In this way, for example, a hemopoietic tissue, a gland tissue or the like can be formed.

3. Method of inoculating cultured cells having been directed to differentiation or the like. (Such cultured cells are available from organizations such as cell banks or can be prepared).

4. Method of forming an organ from stem cells by inoculating the stem cells and adding a substance directing differentiation thereinto.

In the present invention, it has been shown by an experiment in which an *in vivo* incubator containing fibroblast growth factor (FGF) and an *in vivo* incubator containing both FGF and NELL-1 are compared that NELL-1 can be used with other growth factors cooperatively or competitively. Thus, the cells having been directed to a given differentiation for use in the present invention can, if NELL-1 is added, A) further induce or accelerate differentiation of cells having been directed to differentiation or cells by directing to differentiation, and B) accelerate engraftment of cells higher in

differentiation degree, independently of the methods 1 to 4 exemplified above.

In another embodiment, the stem cell present in the hematopoietic tissue for use in the composition according to the present invention can be a mother cell of a blood cell having self repair capability and multipotency. The stem cell present in the hematopoietic tissue can be differentiated into a blood cell (for example, red blood cell, leukocyte or macrophage), for example, by a cytokine such as granular colony stimulating factor (G-CSF).

In another embodiment, the somatic stem cell present in the epithelial tissue for use in the present invention can be, but is not limited to, an adenoblast cell or a hair root cell.

In another embodiment, the cell, tissue or organ differentiated by the present invention can be, but is not limited to, a hemopoietic or epithelial cell, tissue or organ.

In another embodiment, the cell, tissue or organ hematopoietically differentiated by the present invention can be, but is not limited to, a blood cell such as a leukocyte including a neutrophil, an eosinophil, a basophil and a lymphocyte; an erythrocyte; a platelet; a macrophage; and a combination thereof. Without being bound by theory, although it may be supposed that NELL-1 has induced dedifferentiation of mature cells and redifferentiation thereof into hemopoietic cells, it is likely that the composition according to the present invention acts on hematopoietic stem cells, inducing further differentiation of the hematopoietic stem cell according to the direction of differentiation into hemopoietic cells.

The composition according to the present invention allows heterotopical formation of a hemopoietic cell, tissue and organ. The hemopoietic cell, tissue or organ differentiated by the present invention has unexpected distinctive effect of maintaining its inherent function.

In another embodiment, the cell, tissue or organ epithelially differentiated by the present invention can be, but is not limited to, an exocrine gland and a duct thereof. Examples of the exocrine glands include, but are not limited to, perspiratory glands, sebaceous glands, intestinal glands, gastric glands and salivary glands. Without being bound by theory, although it may be supposed that NELL-1 has induced dedifferentiation of mature cells and redifferentiation thereof into epithelial cells, it is likely that the composition according to the present invention acts on somatic stem cells present in the epithelium, inducing further differentiation of the somatic stem cells according to the direction of differentiation into a gland tissue. In a preferred embodiment, the cell having been directed to a given differentiation is an adenoblast cell, and the differentiated cell, tissue or organ can be an exocrine gland or a duct thereof.

The composition according to the present invention allows heterotopical formation of gland tissue. The gland tissue differentiated by the present invention, has the unexpected distinctive effect of maintaining its inherent function.

In another embodiment, the epithelial tissues according to the present invention include, but are not limited to, hair, hair bulb, hair root, a gland tissue associated with hair root and the like. Without being bound by theory, although it may be supposed that NELL-1 has induced dedifferentiation of mature cells and redifferentiation thereof into epithelial cells, it is likely that the composition according to the present invention acts on somatic stem cells (hair root cells), inducing further differentiation of the hair root cell according to the direction of differentiation into hair, hair bulb, hair root or a gland tissue associated with hair root. In a preferred embodiment, the cell having been directed to a given differentiation is a cell included in a hair root, and the differentiated cell, tissue or organ can be hair, hair bulb, hair root, or a gland tissue associated with hair root.

The composition according to the present invention can heterotopically form hair, hair bulb, hair root, or a gland tissue associated with hair root. These tissues differentiated by the present invention have an unexpected distinctive effect of maintaining their respective inherent function.

In another embodiment, the amount of NELL-1 contained in the composition according to the present invention may be about 0.01 μg/ml or more, about 0.01 μg/ml to about 1000 μg/ml, about 0.1 μg/ml to about 100 μg/ml, preferably about 5 μg/ml to about 50 μg/ml, about 5 μg/ml to about 10 μg/ml, more preferably about 5 μg/ml. The lowest amount of NELL-1 may be, for example, any numerical value in the range of about 0.01 μg/ml to about 1000 μg/ml, such as about 0.01 μg/ml, about 0.1 μg/ml, about 0.2 μg/ml, about 0.3 μg/ml, about 0.4 μg/ml, about 0.5 μg/ml, about 0.6 μg/ml, about 0.7 μg/ml, about 0.8 μg/ml, about 0.9 μg/ml, about 1.0 μ/ml, about 1.5 μg/ml, about 2.0 μg/ml, about 2.5 μg/ml, about 3.0 μg/ml, about 3.5 μg/ml, about 4.0 μg/ml, about 4.5 μg/ml, about 5.0 μg/ml, about 5.5 μg/ml, about 6.0 μg/ml, about 6.5μg/ml, about 7.5 μg/ml, about 8.0 μg/ml, about 8.5 μg/ml, about 9.0 μg/ml, about 9.5 μg/ml, or about 10.0 μg/ml. The highest amount of NELL-1 may be, for example, any numerical value in the range of about 0.01 μg/ml to about 1000 μg/ml, such as about 10.0 μg/ml, about 9.5 μg/ml, about 9.0 μg/ml, about 8.5 μg/ml, about 8.0 μg/ml, about 7.5 μg/ml, about 6.5 μg/ml, about 6.0 μg/ml, about 5.5 μg/ml, about 5.0 μg/ml, about 4.5 μg/ml, about 4.0 μg/ml, about 3.5 μg/ml, about 3.0 μg/ml, about 2.5 μg/ml, about 2.0 μg/ml, about 1.5 μg/ml, about 1.0 μg/ml, about 0.9 μg/ml, about 0.8 μg/ml, about 0.7 μg/ml, about 0.6 μg/ml, about 0.5 μg/ml, about 0.4 μg/ml, about 0.3 μg/ml, about 0.2 μg/ml, about 0.1 μg/ml or about 0.01 μg/ml. These values are common for hemopoietic cells (such as a leukocyte including a neutrophil, an eosinophil, a basophil and a lymphocyte; an erythrocyte; platelet; and a macrophage), epithelial exocrine glands (such as perspiratory glands, sebaceous glands, intestinal glands, gastric glands and salivary glands), hair, hair bulb, hair root, and a gland tissue associated with hair root, and the like. The amount of NELL-1 contained in the composition according to the present invention can be determined properly by those skilled in the art according to the application, and the concentration thereof or the like can also be definitely determined.

In another embodiment, the differentiated cell, tissue or organ has a function corresponding to that present in nature. Examples of the functions include the followings and the functions can be confirmed in the following manner.

Hemopoietic tissue: confirmation of expression of hemopoietic cell markers by immunochemical staining and flow cytometry

Exocrine gland and duct thereof: confirmation of tissue image by tissue staining (hematoxylin-eosin staining or the like) and confirmation of secretion substances listed below

Perspiratory gland: confirmation of secretions such as sodium and apocrine secretion
Sebaceous gland: confirmation of secretions such as lipids
Intestinal gland: confirmation of secretions such as ion peptidase and maltase
Gastric gland: confirmation of pepsinogens such as digestive enzymes, hydrogen ions, chloride ions or the like
Salivary gland: confirmation of secretion of amylase, ptyalin or peroxydase

Hair, hair bulb, hair root or a gland tissue associated with hair root: observation with naked eyes and confirmation of tissue image by tissue staining (hematoxylin-eosin staining or the like)

The composition, material and the like according to the present invention can contain, as needed, suitable formulation materials or pharmaceutically acceptable carriers. Examples of the suitable formulation materials or the pharmaceutically acceptable carriers include, but are not limited to, antioxidants, preservatives, colorants, fluorescent pigments, flavoring agents, diluents, emulsifiers, suspending agents, solvents, fillers, bulking agents, buffer agents, delivery vehicles and/or pharmaceutical adjuvants. Typically, the composition and the like according to the present invention are administered in the form of a composition or a material containing NELL-1, and as needed other active ingredients, with at least one physiologically acceptable carrier, excipient or diluent. For example, a suitable vehicle can be a micelle, an injection solution, a physiological solution, or synthetic cerebrospinal fluid, and other substances commonly used in compositions for parenteral delivery can be added thereto.

The acceptable carrier, diluent or stabilizer for use in the present specification is preferably non-toxic to the recipient and preferably inactive at the administration amount and concentration used, and preferred examples thereof include phosphate salts, citrate salts and other organic acids; ascorbic acid, α-tocopherol; low-molecular-weight polypeptides; proteins (such as serum albumin, gelatin or immunoglobulin); hydrophilic polymers (such as polyvinylpyrrolidone); amino acids (such as glycine, glutamine, asparagine, arginine or lysine); monosaccharides, disaccharides and other carbohydrates (glucose, mannose or dextrin); chelating agents (such as EDTA); sugar alcohols (such as mannitol or sorbitol); salt-forming counter ions (such as sodium); and/or nonionic surfactants (such as Tween, pluronic or polyethylene glycol (PEG)).

Suitable examples of carriers include neutrally buffered physiological saline and physiological saline mixed with serum albumin. Preferably, the product is formulated as a freeze-dried preparation with a suitable diluent (such as sucrose). Other standard carriers, diluents and excipients can be contained, if necessary. Other exemplary compositions contain a Tris buffer having a pH of about 7.0 to 8.5 or an acetate buffer having a pH of about 4.0 to 5.5, and may additionally contain sorbitol or a suitable alternative thereof.

Hereinafter, a general method for preparing the composition according to the present invention will be described. It should be noted that animal drug compositions, quasi drugs, aquaculture drug compositions, food compositions and cosmetic compositions and the like can also be prepared by known preparation methods.

The composition and the like according to the present invention can be administered parenterally, as it is blended with a pharmaceutically acceptable carrier as needed. Examples of the pharmaceutically acceptable carriers include diluents, lubricants, binders, disintegrants, decay inhibitors, absorption accelerators, absorbents, wetting agents, solvents, solubilizing agents, suspending agents, isotonic agents, buffer agents and soothing agents. In addition, formulation additives such as antiseptics, antioxidants, colorants and sweeteners can also be used, as needed. Substances other than NELL-1 may be blended with the composition and the like according to the present invention. Examples of the parenteral administration routes include, but are not limited to, intravenous administration, intramuscular administration, subcutaneous administration, intracutaneous administration, mucosal administration, endorectal administration, intravaginal administration, local administration and skin administration. Consideration of pH, isotonicity, stability and the like in preparation of such a pharmaceutically acceptable composition is within the technical scope of those skilled in the art.

The composition according to the present invention may contain additionally: colorants, preservatives, flavoring agents, corrective agents, sweeteners and other drugs.

(Material)

In an aspect, the present invention provides a material for inducing differentiation of a cell having been directed to a given differentiation, to a further differentiated cell, tissue or organ directed to the given differentiation. The material

can contain

A) a sustained-releasing scaffold, and
B) NELL-1 or a substance which is altered to function as NELL-1 at the time of the induced differentiation.

In an embodiment, the sustained-releasing scaffold for use in the present invention may be an extracellular matrix. Examples of the extracellular matrices include, but are not limited to, collagen and atelocollagen. In the present invention, the sustained-releasing scaffold is not particularly limited, if it can consistently provide NELL-1 to a targeted cell or the like the scaffold is a sustained-releasing scaffold. Collagen and the like are available, for example, from Hakuho Corporation (supplier: Hakusui Trading Co., Ltd., Osaka) and collagens from other suppliers can also be used in the present invention. It should be understood that, in other preferred embodiments, any preferred form similar to that described for the composition in the present specification may be adopted.

(Kit)

In other aspects, the present invention provides a kit for inducing differentiation of a cell having been directed to a given differentiation, to a further differentiated cell, tissue or organ directed to the given differentiation. The kit can contain NELL-1 or a substance which is altered to function as NELL-1 at the time of the induced differentiation. It should be understood that, in other preferred embodiments, any preferred form similar to that described for the composition, material and method in the present specification may be adopted.

(Medical device)

In other aspects, the present invention provides a medical device for inducing differentiation of a cell having been directed to a given differentiation, to a further differentiated cell, tissue or organ directed to the given differentiation. The medical device can contain

A) NELL-1 or a substance which is altered to function as NELL-1 at the time of the induced differentiation;
B) a sustained-releasing scaffold; and
C) a container.

It should be understood that, in other preferred embodiments, any preferred form similar to that described for the composition, material and kit in the present specification may be adopted.

(Production method)

In other aspects, the present invention provides a method for producing a cell, tissue or organ. The method can include the steps of:

providing a cell having been directed to a given differentiation; and
contacting the cell having been directed to a given differentiation with NELL-1 or a substance which is altered to function as NELL-1 at the time of the induced differentiation.

In an embodiment, the cell having been directed to a given differentiation can be provided in the presence of fat tissue and blood vessels supporting the same in the production method according to the present invention.
In another embodiment of the differentiation method according to the present invention, the cell having been directed to a given differentiation is contacted with the NELL-1 on a sustained-releasing scaffold, but it is not limited thereto. Without being bound by theory, any means by which NELL-1 may be contacted with the cells to be differentiated for a period when NELL-1 resulting in action is encompassed. It should be understood that, in other preferred embodiments, any preferred form similar to that described for the composition, material, method, kit and the like in the present specification may be adopted.
Products produced by the production method according to the present invention are also included in the scope of the present invention.

(Composition for support application)

In another aspect, the present invention provides a composition for supporting a differentiated cell, tissue or organ. The composition can include NELL-1 or a substance which is altered to function as NELL-1 at the time of the

support.

The composition for support application according to the present invention has an unexpected advantageous effect of maintaining the form and size of the transplanted cell, tissue or organ and allowing it to maintain its inherent function.

In an embodiment, the support of the cell, tissue or organ differentiated by the composition for support application according to the present invention can be carried out at a location which does not allow presence of the differentiated cell, tissue or organ. Examples of the location which does not allow presence of the differentiated cell, tissue or organ include, but are not limited to, a location where the cell, tissue or organ is normally absent (for example, fat tissue for liver tissue graft). The composition for support application according to the present invention can suppress an immunological rejection reaction and be maintained in the living body, independently of whether it is transplanted into the living body or formed or induced in the living body. When transplanted into the living body, the cell, tissue and organ to be transplanted may be differentiated naturally or artificially.

In a preferred embodiment, the composition for support application according to the present invention can support, for example, liver, kidney, pancreas, adrenal gland, thyroid gland, ovary, testis and the like, but the organs supported are not limited thereto.

The reason that heterotopical transplantation of organ and the like was not possible is believed to be due to the presence of the foreign material-recognizing and removing mechanism, which is generally called rejection reaction. Herein, the fact that NELL-1 has a hemopoietic cell-forming potency and a heteroblastic tissue-forming potency has been found by the present invention. Without being bound by theory, when these findings are taken into consideration, the reason for the lack of rejection reaction of the transplanted organ may be that an own tissue that is not rejected by the immune system is newly formed or is in the course of formation by NELL-1.

The cell, tissue or organ supported by the composition according to the present invention maintains a function corresponding to that present in nature. Examples of the functions will be described below.

Liver:

1) metabolic function: producing proteins by storing and using sugars and fats as energy
2) detoxicating function: detoxicating hazardous substances by metabolism
3) excretion function: secreting bile

In particular, the liver function can be confirmed by measuring albumin production and an increase in the amounts of hepatic cell function markers (tryptophan oxygenase and the like).

Pancreas: Pancreatic function can be confirmed by measuring an increase in production of insulin and its precursor substances internally secreted from pancreatic β cells or the like;
Adrenal gland: Adrenal function can be confirmed by confirming secretion of steroid hormones;
Thyroid gland: Thyroid gland function can be confirmed by confirming secretion of thyroid hormones (such as triiodothyronine and thyroxin);
Ovary: Ovarian function can be confirmed by confirming secretion of estrogen, progesterone and the like;
Testis: Testoid function can be confirmed by confirming secretion of testosterone, dihydrotestosterone and dehydroepiandrosterone; and
Kidney: Kidney function can be confirmed by confirming secretion of erythropoietin.
In another embodiment, the amount of NELL-1 contained in the composition for support application according to the present invention can be about 0.01 μg/ml or more, about 0.01 μg/ml to about 1000 μg/ml, about 0.1 μg/ml to about 100 μg/ml, preferably about 5 μg/ml to about 50 μg/ml, about 5 μg/ml to about 10 μg/ml, and more preferably about 5 μg/ml. The lowest amount of NELL-1 can be any numerical value in the range of about 0.01 μg/ml to about 1000 μg/ml, such as about 0.01 μg/ml, about 0.1 μg/ml, about 0.2 μg/ml, about 0.3 μg/ml, about 0.4 μg/ml, about 0.5 μg/ml, about 0.6 μg/ml, about 0.7 μg/ml, about 0.8 μg/ml, about 0.9 μg/ml, about 1.0 μg/ml, about 1.5 μg/ml, about 2.0 μg/ml, about 2.5 μg/ml, about 3.0 μg/ml, about 3.5 μg/ml, about 4.0 μg/ml, about 4.5 μg/ml, about 5.0 μg/ml, about 5.5 μg/ml, about 6.0 μg/ml, about 6.5 μg/ml, about 7.5 μg/ml, about 8.0 μg/ml, about 8.5 μg/ml, about 9.0 μg/ml, about 9.5 μg/ml or about 10.0 μg/ml. The highest amount of NELL-1 can be any numerical value in the range of about 0.01 μg/ml to about 1000 μg/ml, such as about 10.0 μg/ml, about 9.5 μg/ml, about 9.0 μg/ml, about 8.5 μg/ml, about 8.0 μg/ml, about 7.5 μg/ml, about 6.5 μg/ml, about 6.0 μg/ml, about 5.5 μg/ml, about 5.0 μg/ml, about 4.5 μg/ml, about 4.0 μg/ml, about 3.5 μg/ml, about 3.0 μg/ml, about 2.5 μg/ml, about 2.0 μg/ml, about 1.5 μg/ml, about 1.0 μg/ml, about 0.9 μg/ml, about 0.8 μg/ml, about 0.7 μg/ml, about 0.6 μg/ml, about 0.5 μg/ml, about 0.4 μg/ml, about 0.3 μg/ml, about 0.2 μg/ml, about 0.1 μg/ml or about 0.01 μg/ml. The numerical values are common for transplanted organs (such as liver, kidney, pancreas, adrenal gland, thyroid gland, ovary and testis), and internally formed organs (hemopoietic cells (such as a leukocytes

including neutrophils, eosinophils, basophils and lymphocytes; erythrocytes; platelets; and macrophages), epithelial exocrine glands (such as perspiratory glands, sebaceous glands, intestinal glands, gastric glands and salivary glands), and hair, hair bulb, hair root, a gland tissue associated with hair root and the like). The amount of NELL-1 contained in the composition according to the present invention can be determined properly by those skilled in the art according to the application, and the concentration thereof or the like can also be definitely determined.

It should be understood that, in other preferred embodiments, any preferred form similar to that described for the composition, material, method and kit in the present specification may be adopted.

(Support method)

In another aspect, the present invention provides a method for supporting a differentiated cell, tissue or organ. The method can include the steps of:

providing the differentiated cell, tissue or organ; and

giving NELL-1 or a substance which is altered to function as NELL-1 at the time of the support to the differentiated cell, tissue or organ.

It should be understood that, in other preferred embodiments, any preferred form similar to that described for the composition, material, method and kit in the present specification may be adopted.

(Use)

In another aspect, the present invention provides the use of NELL-1 or a substance which is altered to function as NELL-1 at the time of formation in the manufacture of a medicament for producing a differentiated cell, tissue or organ from a cell having been directed to a given differentiation.

In another aspect, the present invention provides use of NELL-1 or a substance which is altered to function as NELL-1 at the time of support in the manufacture of a medicament for supporting a differentiated cell, tissue or organ.

It should be understood that, in other preferred embodiments, any preferred form similar to that described for the composition, material, method and kit in the present specification may be adopted.

The reference documents such as scientific documents, patents, patent applications cited in the present specification are fully incorporated in their entirety by reference in the present specification to a degree identical to such that each of the documents is specifically described.

According to the above description, the present invention has been described with reference to preferred embodiments for easy understanding. Hereinafter, the present invention will be described with reference to examples, however, it should be understood that the description above and the following examples are provided only as examples and are not provided for restriction of the present invention. Therefore, the scope of the present invention is not restricted by the embodiments or the examples specifically described in the present specification, but is restricted only by the claims.

EXAMPLES

Hereinafter, the present invention will be described more specifically with reference to examples, but it should be understood that the present invention is not restricted by these examples. Reagents and others used in examples were obtained from general reagent makers and NELL-1 obtained from Katayama Chemical, Ltd. was used unless specified otherwise. Animal tests were carried out according to the ethical code specified by Showa University. Human tests, when performed, are carried out after informed consent has been obtained.

(Preparation of NELL-1)

hNELL1 protein was produced, purified and used as NELL-1 in the following examples.

Used for production and purification of the hNELL1 protein were reagents purchased or available from Sigma-Aldrich Co., GE Healthcare Japan (formerly, Amersham Bioscience Corporation), Invitrogen Corporation, Funakoshi Corporation, Cosmo Bio Co., Ltd., Bio-Rad Laboratories, Inc., Qiagen, Promega K.K., Takara Bio Inc., Toyobo Co., Ltd., Daiichi Pure Chemicals Co., Ltd., Greiner Bio-One Co. , Ltd., DS Pharma Biomedical Co., Ltd. (formerly, Laboratory Products Department of Dainippon Pharmaceutical Co., Ltd.), Becton, Dickinson and Company, Merck & Co., Ltd., Hydrus Chemical Inc., PerkinElmer Co., Ltd., Millipore Corporation, Nihon Waters K.K., Tosoh Corporation, GL Science Inc., Shiseido Co., Ltd., Taitec Co., Ltd., Affymetrix, Inc., AS ONE Corporation, TGK, IKA, PerkinElmer Japan Co., Ltd., Roche Diagnostics K.K., Oriental Yeast Co., Ltd., Medical & Biological Laboratories Co., Ltd., Immuno-Biological Laboratories Co., Ltd., Peptide Institute, Inc., Toyobo Engineering Co., Ltd., Sanko Junyaku Co., Ltd., Veritas Corporation, TEFCO, Sartorius K.K., Seikagaku Corporation and Biomedical Department of Kurabo Industries Ltd.

hNELL1 protein was produced and purified in the present example.

(Isolation of hNELL cDNA)

First, 2448-bp hNELL1 cDNA was isolated from human brain cDNA library by PCR according to the method described below.

(Construction of plasmid)

A schematic procedure for the construction of a plasmid is shown (Figs. 1A to 1C).
(1) Human brain cDNA was prepared from human brain whole RNA by reverse transcription reaction.
(2) For preparation of human NELL-1 cDNA by PCR method, four fragments were produced from the obtained human brain cDNA by using respective primers.
(3) Two of the four fragments obtained were further linked to each other by PCR method, to give two fragments.
(4) The two fragments thus obtained were further linked to each other by PCR method, to give a full-length cDNA.
(5) The full-length cDNA obtained was introduced into a vector for pGEM-T cloning by TA cloning, to give a human NELL1 plasmid.

(Detailed procedure)

Human NELL1 cDNA was produced from human brain mRNA by reverse transcription.
To a 1.5 mL tube containing 13.5 μL of diethyl pyrocarbonate-treated ultrapure water (DEPC-UPW) were added 2 μL of human brain whole RNA (2.5 mg/mL, Clontech) and 1.5 μL of Oligo $(dT)_{12-18}$ (Invitrogen) for mixing, and the mixture was left at room temperature for 20 minutes. Then, 3 μL of a 10 x PCR buffer (GIBCO), 1.5 μL of 25 mM $MgCl_2$, 3 μL of 0.1 M DTT and 2 μL of 25 mM dNTP were added to the tube; the resultant was mixed thoroughly and spud down; 1.5 μL of Superscript II (Invitrogen) was added thereto; the mixture was allowed to react in reverse transcription reaction at 42°C for 60 minutes, heated at 72°C for 15 minutes and then stored at -20°C.
Subsequently, the following four fragments 1 to 4 (f1 to f4) were amplified by PCR.
Fragment 1 (f1) 527 bp
Fragment 2 (f2) 624 bp
Fragment 3 (f3) 612 bp
Fragment 4 (f4) 841 bp

The primers and the reaction solutions used were as follows:
[Table 1]

| Primer number | Sequence, 5'-3' | Location |
|---|---|---|
| F#2 | GGAAGCTTCGGAGCGATGCCGATGGATTTGATT (SEQ ID No.19) | 87-120 |
| F#3 | TCAGTTAGCGCCTCTCATCTC (SEQ ID No.20) | 564-584 |
| F#4 | GCGGATTTTAACCAAGAGCTG (SEQ ID No.21) | 1139-1159 |
| F#5 | GTGTCTGTCCATCTGGATTCAC (SEQ ID No.22) | 1705-1726 |
| R#2 | GTAACCGGTTCAATTATTTTGAAGACACTCAAAATCC (SEQ ID No.23) | 2544-2508 |
| R#3 | ATCTTTCTCGCAGTGGCTTCC (SEQ ID No.24) | 1749-1729 |
| R#4 | CTAAAACTCCACCTCGGCATT (SEQ ID No.25) | 1185-1165 |
| R#5 | ATCCTGTTACAGTCGACATGG (SEQ ID No. 26) | 610-590 |

| | |
|---|---|
| Plasmid DNA (10-fold dilution) | 1 μL |
| 10 x Thermopol. buffer solution | 5 μL |
| 10 mM dNTP (SIGMA) | 1 μL |
| 10 μL primer (forward direction) | 1 μL |
| 10 μL primer (reverse direction) | 1 μL |

(continued)

| | |
|---|---|
| VENT DNA pol. (NEB) | 1 μL |
| Ultrapure water (UPW) | 40 μL |
| | Total 50 μL |

f1 used was a combination of a forward primer F#2 (SEQ ID NO.: 19) and a reverse primer R#5 (SEQ ID NO.: 26); f2 used was a combination of a forward primer F#3 (SEQ ID NO.: 20) and a reverse primer R#4 (SEQ ID NO.: 25); f3 used was a combination of a forward primer F#4 (SEQ ID NO.: 21) and a reverse primer R#3 (SEQ ID NO.: 24); and f4 used was a combination of a forward primer F#5 (SEQ ID NO.: 22) and a reverse primer R#2 (SEQ ID NO.: 23).

In the PCR reaction, the mixture was treated at 95°C for 2 minutes, and then treated at 95°C for 30 seconds, at 55°C for 30 seconds and at 72°C for 1 minute repeatedly in 25 cycles; and then, treated at 72°C for 7 minutes and kept at 4°C.

Subsequently for linkage of f1 and f2 or f3 and f4, the following reaction solution was prepared and subjected to PCR reaction.

| | |
|---|---|
| PCR reaction solution (f1 and f2 or f3 and f4) | each 1 μL |
| 10 x Pfu buffer solution | 5 μL |
| 10 mM dNTP (SIGMA) | 2 μL |
| 10 μM primer (forward direction) #2 or #4 | 1 μL |
| 10 μM primer (reverse direction) #4 or #2 | 1 μL |
| Pfu turbo DNA pol. (STRATAGENE) | 1 μL |
| Ultrapure water (UPW) | 38 μL |
| | Total 50 μL |

In the PCR reaction, the mixture was treated at 95°C for 2 minutes, and then treated at 95°C for 1 minute, at 60°C for 1 minute and at 72°C for 2 minutes repeatedly in 3 cycles. A primer was then added to the reaction solution. The mixture was further treated at 95°C for 1 minute, at 55°C for 1 minute and at 72°C for 2 minutes repeatedly in 27 cycles, and then treated at 72°C for 10 minutes and kept at 4°C.

Further, the two fragments produced by linkage of f1 and f2 or of f3 and f4 were linked to each other by PCR, to give a full-length human NELL1 cDNA. A reaction solution was prepared as follows:

| | |
|---|---|
| PCR reaction solution (f1-2, f3-4) | each 1 μL |
| 10 x Pfu buffer solution | 5 μL |
| 10 mM dNTP (SIGMA) | 2 μL |
| 10 μM primer (forward direction) #2 | 1 μL |
| 10 μM primer (reverse direction) #2 | 1 μL |
| Pfu turbo DNA pol. (STRATAGENE) | 1 μL |
| Ultrapure water (UPW) | 38 μL |
| | Total 50 μL |

In the PCR reaction, the mixture was treated at 95°C for 2 minutes, and then treated at 95°C for 1 minute, at 60°C for 1 minute and at 72°C for 2 minutes repeatedly in 3 cycles. A primer was then added to the reaction solution (see the table below). The mixture was additionally treated at 95°C for 1 minute, at 55°C for 1 minute and at 72°C for 2 minutes repeatedly in 27 cycles and then allowed to react at 72°C for 10 minutes and kept at 4°C. To the reaction solution was added 1 μL of Dpn I (NEB) and the mixture was allowed to react at 37°C for 1 hour. The reaction solution obtained was precipitated with ethanol and the precipitate was dissolved in ultrapure water (UPW). To the resultant were added 0.5 μL of 10 mM dATP and 2.5 μL of a 10 x Taq DNA polymerase buffer. To the resultant was added 0.5 μL of Taq DNA polymerase and the mixture was allowed to react at 72°C for 20 minutes.

After addition of 1 μL of pGEM-T vector, 5 μL of 2 x Rapid Ligation Buffer, 1 μL of the full-length human NELL1 obtained by PCR, 1 μL of T4 DNA Ligase and ultrapure water (UPW) to a 0.5 mL tube to make the total amount 10 μL, the nucleic acid obtained was subjected to TA cloning, as the mixture was incubated at room temperature for 1 hour.

The following table shows the information used in primer design.

[Table 2]

Candidate primer

```
hNELL1 forward #1: 5'-GGCTCATTTGCTTCCACCTAG-3'  (21-mer) (SEQ ID No.30)
       forward #2: 5'-GGAAGCTTCGAGAGCGATGCCGATGGATTTGATT-3'  (34-mer) (SEQ ID No.19)
       forward #3: 5'-TCAGTTAGCGCCTCTCATCTC-3'  (21-mer) (SEQ ID No.20) #564 -#584
Tm :  64  GC : 52.38
       forward #4: confirming primer                                   #1139 -#1159
       forward #5: 5'-gtgtctgtccatctggattcac-3' (21-mer) (SEQ ID No.22) #1705-#1726

       reverse #1: 5'-GTCATTTCGTCCATTCTTCTG-3'  (21-mer) (SEQ ID No.31)
       reverse #2: 5'-GTAACCGGTTCAATTATTTTGAAGACACTCAAAATCC-3'  (37-mer) (SEQ ID No.23)
       reverse #3: confirming primer                                   #1749 -#1729
       reverse #4: 5'-CTAAAACTCCACCTCGGCATT-3'  (21-mer) (SEQ ID No.25) #1185 -#1165
Tm :  62  GC : 47.62
       reverse #5: 5'-atcctgttacagtcgacatgg-3'  (21-mer) (SEQ ID No.26) #610-#590
```

Size of fragment

```
f2-r5: 527bp (f1)
f3-r4: 624bp (f2)
f4-r3: 612bp (f3)
f5-r2: 841bp (f4)
```

(Production and purification of hNELL1 protein)

(Summary)

The hNELL1 cDNA fragment used was one obtained in the present example.

The hNELL1 cDNA fragment was inserted to a position downstream of the OpIE2 promotor of an expression vector pIZT/V5-His contained in InsectSelect Glow System (Invitrogen, Carlsbad, CA), to give C-terminal V5- and Hisx6-tagged protein (hNELL1-VH). Then, Trichoplusia ni-derived High Five™ cells (Invitrogen) were transfected with the obtained plasmid pIZT/V5-His-NELL1 by using FuGene 6 (Roche, Mannheim, Germany) and incubated in a serum-free medium Express Five SFM (Invitrogen) for 48 hours. An antibiotic Zeocin (Nakalai Tesque, Kyoto, Japan) was added to the medium at a concentration of 400 μg/ml and the medium was exchanged every 3 to 4 days, for selection of Zeocin-resistant cells. For monitoring extracellular production of the hNELL1-VE protein, the culture medium (6 ml) was incubated with anti-V5 tag antibody (1 μg; Nakalai Tesque) and the sediment was subjected to sodium dodecylsulfate-polyacrylamide gel electrophoresis (SDS-PAGE) in 6% gel and also to Western blotting on PVDF membrane by using the antibody. For large-scale affinity purification of the hNELL1-VE protein, the culture medium on the third day (adjusted to 500 ml) was applied to a plastic column (ϕ 1.5 x 10 cm) packed with Ni Sepharose 6 Fast Flow (GE Healthcare UK, Buckinghamshire, UK), and the column was washed thoroughly with phosphate-buffered saline (PBS) and then eluted with 500 mM imidazole solution in PBS. The eluate (about 10 ml) was dialyzed thoroughly against PBS at 4°C overnight and concentrated to about 900 μg/ml by ultrafiltration. The purity of the hNELL1-VH protein was confirmed by SDS-PAGE and by staining with Coomassie Brilliant Blue R-250. More specifically, the purity was confirmed in the following manner:

(Material and method)

(Expression vector)

An insect cell expression vector was constructed by using human NELL1 (hNELL1) cDNA (ORF 2433 bp) synthesized and cloned from human brain mRNA. The expression vector used was pIZT/V5-His (Invitrogen). The multicloning sites SpeI and SacII in the vector were used for insertion of hNell1. The DNA fragment to be inserted was

amplified by PCR method and cleaved and ligated by the same restriction enzymes. The stop codon of hNELL1 was removed for a fusion tag expression vector, while the stop codon of hNELL1 was used for a non-fusion tag expression vector. Gene and protein information was retrieved from databases via the Internet.

Gene information: NCBI
http://www.ncbi.nlm.nih.gov/entrez/viewer.fcgi?db= nucleotide&val=1827482
Protein information: Swiss-Prot
http://au.expasy.org/uniprot/Q92832
Tag region: PRFEGKPIPNPLLGLDSTRTGHHHHHH (SEQ ID NO.: 29)
The SacII cloning site region is PR in the tag region.

The V5 tag region in the tag region corresponds to the nucleic acids coding PIPNPLLGLDST (SEQ ID NO.: 27).

The histidine tag region in the tag region corresponds to the nucleic acid coding HHHHHH (SEQ ID NO.: 28). There is stop codon downstream thereof.

(Establishment of transduced cell strain)

(Cell and medium)

The insect cell used was HiFive (Invitrogen), and the medium used was a mixture of Express Five SFM (Invitrogen, #10486-025, 1L), 90 mL of 200 mM L-glutamine and suitable antibiotics (penicillin and streptomycin).

(Transduction)

Transduction was done by electroporation method using Gene Pulser (Bio-Rad). To a cuvette (Bio-Rad) with a slit having a width of 4 mm were added 10 mg of DNA and cells ($1x10^6$ pieces) and the mixture was suspended gently, cooled on ice for 10 minutes and then subjected to pulses at 125 μF and 300 V. The mixture was then cooled on ice for 10 minutes and added back to the medium for culture.

(Selection of expression cell (zeocin-resistant))

Because the vector shows resistance to zeocin (Invitrogen), zeocin was added to the medium to a final concentration of 0.5 mg/mL and three-day culture was repeated twice. In addition, because the vector co-expresses GFP, transformed cells were identified by confirming expression of GFP under epifluorescence microscope (CKX41 Olympus).

(Small-scale culture)

The zeocin-resistant cells were inoculated as expression strain on a 10 cm dish (BD Falcon) at 20% confluency and the culture supernatant was collected after culturing for 4 days by high-speed centrifugation (5000 rpm, 20 minutes). The culture temperature was 27°C.

(Protein purification)

Nickel Sepharose FF6 medium (Amersham Biosciences) was used for purification. EconoColumn (Bio-Rad) was packed with 1 mL of the medium and equilibrated with an equilibration buffer (10 mM Tris-HCl, 20 mM Imidazole, 150 mM NaCl, pH 7.6) and the culture supernatant obtained by centrifugation was applied directly on the column and fed through by gravity, while affinity bonding was allowed. The column was then washed with a washing buffer (10 mM Tris-HCl, 20 mM Imidazole, 150 mM NaCl, pH 7.6) and eluted with an elution buffer (10 mM Tris-HCl, Imidazole, 0.5 mM PMSF, pH 7.6). The Nickel Sepharose contains nitrilotriacetic acid (NTA) immobilized on the Sepharose surface and can hold nickel ions by bonding of NTA and nickel. It is possible to purify only proteins having fused His tag by using bonding of nickel and histidine. Desired proteins were isolated using imidazole, which is similar in structure to histidine but a less expensive alternative (see the following Chemical Formula), for elution and thus making it replace the histidine residues in the desired protein captured.

[Formula 1]

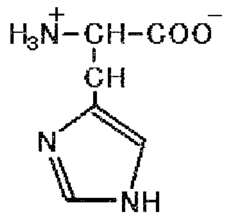 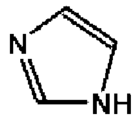

Histidine Imidazole

A dialysis tube (trade name: Spectra/Por2; model number: 132680: Spectrum Laboratories) was cut to a length of about 10 cm and washed in boiling water for 5 minutes. The tube after boiling was washed with pure water; one end was sealed with a specially designed clip; the collected NELL-1 protein solution was placed in the tube; and the other end was also sealed with a clip, as the tube was deaerated. The tube was connected to a float and immersed in 5 L of external dialysate PBS (-) and the solution was agitated gently at 4°C. The dialysis was continued, as the external dialysate was exchanged every hour for four times. After dialysis, the solution in the tube was collected. After collection, the solution was filtered through a φ0.22 μm filter (trade name: Millex GV filter unit; model number: SLGV033RB: Millipore Corporation), to give prepared NELL-1 protein.

| 20xPBS (-) | |
|---|---|
| NaCl | 480 g |
| $NaHPO_4$-$12H_2O$ | 174 g |
| KCl | 12 g |
| $KH_2PO_4$ | 12 g |

Diluted to 1 L with ultrapure water PBS (-)
20xPBS (-) was diluted 20 times with ultrapure water.

(Example 1. Formation of hemopoietic tissue)

(Production of silicone mold)

A silicone rubber impression material (vinylpolysiloxane impression material Exafine, controlled medical device 23BZ0035, G. C. Dental Products Co., Ltd., Tokyo, Japan) was used for production of a silicone mold. A cylindrical Teflon (registered trade name) rotor (1-4206-07) (diameter: 4 mm, length: 8 mm; AS ONE Corporation, Tokyo, Japan) was covered with the thin layer of the silicone rubber. After hardening of the silicone, the layer was cut in halves lengthwise and the Teflon (registered trade name) rotor was removed. For securing the space for a vascular flap, a long linear dent was formed from one terminal of the silicone mold in the region cut half.

The silicone mold can be designed according to the shape of the organ to which it is transplanted. For example, a silicone mold in the shape of rat bone tip and a clay mold in the shape of pine nut can be prepared and used.

(Production of *in vivo* incubator)

Rats (25 male Wistar rats 8 weeks of age, purchased from Saitama Experimental Animals Supply Co., Ltd.) were anesthetized with ether and pentobarbital (0.1 ml/100 gw) was administered intraperitoneally.

Skin incision was conducted in the thigh of a rat, and nerve/vessel bundles including the hypogastric artery and vein branched from the femoral artery and vein, and the nerve running in parallel were ablated from surrounding tissues.

The abdominal subcutaneous fat tissue was resected to a size in accordance with the silicone mold centered at the branch point of the hypogastric artery and vein and a pedicle tissue graft containing the hypogastric artery and vein was produced as the blood vessel pedicle.

Collagen sponges (Helistat, Hakuho Corporation, product code: 4300010, highly controlled medical device 20700BZG00024000) containing NELL-1 protein (5 μg/ml, 10 μg/ml or 50 μg/ml) added dropwise thereto were inserted into the silicone mold (from both sides). The distal end of the fat tissue was sutured and fixed to one side of the silicone mold with 5-0 nylon yarn (Hasegawa Medical BRAK-K15A05H). Another silicone mold was placed thereon and two sites on the side faces were sutured and fixed to each other with 5-0 nylon yarn (Hasegawa Medical BRAK-K15A05H).

(Production of a pocket in the muscles in the femoral region and transplantation)

A pocket extending to the gluteal skin was produced by blunt dissection of rat muscles in the femoral region at the site suitable for embedding the silicone mold without application of excessive pressure on the blood vessel pedicle. Specifically, the region between the adductor muscles and the gracilis muscles at the root of the lower extremities was ablated, to produce a pocket in the muscle.

The silicone mold was embedded in the pocket thus produced. The opening of the pocket was then sutured and closed with 5-0 nylon yarn (Hasegawa Medical BRAK-K15A05H). The thigh was closed after it was confirmed that the blood flow in the blood vessel pedicle was not inhibited.

Rats were anesthetized with diethylether 2 or 4 weeks after transplantation and the transplantation site was excised. The region excised was fixed with a 10% neutral formaldehyde solution, to give a paraffin sample. The sample was cut to a slice of 5 μm, stained with hematoxylin-eosin (HE) and observed in bright field under optical microscope.

(Confirmation of function of tissue formed)

The following test is performed for confirmation of the function as a hemopoietic tissue of the tissue formed in the present example.

The function of the hemopoietic tissue *in vivo* is confirmed by examining the expression of the hemopoietic cell marker with immunochemical staining and flow cytometry.

Localization of the hemopoietic cell marker in a tissue can be examined by confirmation of the texture of the tissue after three-dimensional labeling by immunochemical staining.

It is possible by flow cytometry to measure the cell count by labeling targeted cells and extracting cells by cell marker and dividing the cells into groups by various differentiation phases for examination.

(Results)

It was possible to induce a hemopoietic tissue by optimizing a method of adding the NELL-1 protein at the transplantation site (in the presence of fat tissue and blood vessels supporting the same) (Fig. 1D). It is supposed that NELL-1 induced dedifferentiation of fat cells and re-differentiation of the cells into hemopoietic cells. However, because blood flow is maintained in the fat tissue placed in the silicone mold, it is supposed that differentiation of hematopoietic stem cells (undifferentiated somatic stem cell) present in peripheral blood was further induced in the presence of NELL-1. It is also supposed that the fat tissue-derived stem cells present in the fat tissue differentiated into hemopoietic cells.

There were observed several clumpings of hemopoietic tissues in the *in vivo* incubator. Its tissue image showed the presence of undeveloped blood cells (leukocytes) therein and also support of the blood vessels communicating with the blood vessels connected.

(Comparative Example 1. Formation of tissue when BMP, TGFβ or FGF is used)

In the present comparative example, an *in vivo* incubator is produced by a method similar to that in Example 1, except that BMP, TGFβ or FGF was used in place of NELL-1. (1) An *in vivo* incubator containing only a buffer solution and (2) an *in vivo* incubator not containing collagen sponges but containing NELL-1 are produced as negative controls. Each of these *in vivo* incubators is transplanted similarly to Example 1, such that whether or not formation of a hemopoietic tissue can be examined.

(Example 2A. Formation of gland tissue)

(Production of *in vivo* incubator)

An *in vivo* incubator was produced by a method similar to that in Example 1. The amount of NELL-1 protein added to the silicone mold (both sides) is 5 μg/ml, 10 μg/ml or 50 μg/ml.

(Rats used in test)

The rats used were 25 male Wistar rats, 8 weeks of age (supplier: Saitama Experimental Animals Supply Co., Ltd.).

(Production of pocket in muscles in thigh)

A pocket was produced in the muscles in the thigh of the rats and the *in vivo* incubator was transplanted into the pocket produced by a method similar to that in Example 1. Two to four weeks after transplantation, the shape of the tissue formed at the transplantation site was examined by hematoxylin-eosin staining.

(Confirmation of function of tissue formed)

The following tests are performed for confirmation of the function as a gland tissue of the tissue formed in the present example.
Perspiratory gland: confirmation of secretions such as sodium and apocrine secretion
Sebaceous gland: confirmation of secretions such as lipids
Intestinal gland: confirmation of secretions such as ion peptidase and maltase
Gastric gland: confirmation of pepsinogens such as digestive enzymes, hydrogen ion, chloride ion or the like
Salivary gland: confirmation of secretion of amylase, ptyalin or peroxydase.

(Results)

In the presence of fat tissue and blood vessels supporting the same, it was possible to induce epithelial cells in the fat tissue of pedicle tissue graft by optimizing a method of adding the NELL-1 protein (Figs. 2 and 3). It is supposed that NELL-1 induced dedifferentiation of fat cells and re-differentiation of the cells into epithelial cells. However, it is unlikely that the fat tissue, which derives from mesoderm, differentiates into an ectoderm-derived epithelial tissue. Because blood flow is maintained in the fat tissue placed in the silicone mold, it is likely that previously differentiation-oriented cells present in peripheral blood accumulated locally in the presence of NELL-1 and differentiated into a gland tissue of epithelial cells. The newly formed epithelial tissue was an exocrine gland and a duct thereof (Figs. 2 and 3). It can be said that confirmation of the production of secretions and a duct to transport the secretions outside the body indicates that it is possible in the present example to form and support an epithelial tissue in the fat tissue and make it function.
Accordingly, the results demonstrated an epithelial gland tissue in the fat tissue of the NELL-1-added group. It can be said that the results also demonstrated an epithelial (epidermal) tissue containing hair roots.
There may be two kinds of inoculation routes, demonstrated by the fact that fat tissue is a connective tissue (mesoderm embryologically) and hair roots and gland tissues are generally epithelial tissues (ectoderm embryologically). Therefore, in order to carry out the present example, in the case of hair roots or hairs and the accompanying gland tissues, the procedure above is possible by cutting out a portion of a region of 5 mm square and having a thickness of about 2 mm from the cut-open rear surface of the skin and inoculated.
It is also possible to test whether a somatic stem cell or a fat tissue (mesoderm embryologically), which corresponds to a blood stem cell, can be differentiated into an epithelial tissue. It is also possible to obtain an epithelial tissue by cutting out a tissue from the rear surface of skin and inoculating it.

(Comparative Example 2. Formation of tissue when BMP, TGFβ or FGF is used)

In the present comparative example, an *in vivo* incubator is produced by a method similar to that in Example 2, except that BMP or TGFβ is used in place of NELL-1. (1) An *in vivo* incubator containing only a buffer solution and (2) an *in vivo* incubator not containing collagen sponges but containing NELL-1 are prepared as negative controls. Each of these *in vivo* incubators is transplanted similarly to Example 1 and whether or not formation of a gland tissue occurs can be examined.

(Example 2B. Influence by FGF (fibroblast growth factor))

In the present example, rats and *in vivo* incubators similar to those in Example 2A were used.
(1) An *in vivo* incubator containing only FGF, (2) an *in vivo* incubator containing NELL-1 and FGF, (3) an *in*

*vivo* incubator containing only NELL-1, and (4) an *in vivo* incubator containing only physiological saline were produced. Each of them was transplanted into a pocket in the muscles in the thigh of a rat. The transplantation site was excised, 2 weeks after transplantation, and a hematoxylin-eosin staining sample was produced and observed by a routine method.

As a result, connective tissues and blood vessels are formed, but no fat tissue was formed in the group of (1) an *in vivo* incubator containing only FGF (Figs. 6A and B) . Formation of a gland tissue was observed 2 weeks after transplantation in the group of (2) an *in vivo* incubator containing NELL-1 and FGF (Figs. 6C and D). The gland tissue had multiple branchings, compared to the case where only NELL1 was added, and the periphery of the gland tissue was covered with connective tissues. Administration of FGF to the dermis under the epidermis, which is known to activate a subcutaneous tissue, resulted in accelerated formation of a subcutaneous tissue (gland tissue) that was induced by NELL-1 in the *in vivo* incubator.

In the group of (3) an *in vivo* incubator containing only NELL-1, a gland tissue was observed, 2 weeks after transplantation, but formation of a connective tissue was slower (Figs. 6E and F). The image of the gland tissue was more developed, four weeks after transplantation. In the group of (4) an *in vivo* incubator containing only physiological saline, only a connective tissue was observed around the fat tissue (Figs. 6G and H). The connective tissue surrounding the fat tissue was observed in all groups.

(Example 3. Formation of hairs and hair bulbs)

(Production of *in vivo* incubator)

An *in vivo* incubator was produced by a method similar to that in Example 1. The amount of the NELL-1 protein added to the silicone mold (both sides) is 5 μg/ml, 10 μg/ml or 50 μg/ml.

(Rats used in test)

The rats used were 25 male Wistar rats, 8 weeks of age (supplier: Saitama Experimental Animals Supply Co., Ltd.).

(Collection and inoculation of hairs containing hair roots)

Hairs and epidermis containing hair roots, a region of 5 mm square having a thickness of about 2 mm, were cut out and collected from the cut-open rear surface of the skin of a Wistar rat. This was inoculated on the collagen sponge of the *in vivo* incubator.

(A. Production of pocket in muscles in thigh and transplantation (autologous transplantation))

A pocket was produced in the muscles in the thigh of a rat by a method similar to that in Example 1 and the *in vivo* incubator produced in the present example was transplanted thereinto.

Two to four weeks after transplantation, the shape of the tissue formed at the transplantation site was examined by hematoxylin-eosin staining.

(Confirmation of function of tissue formed)

The following test is performed for confirmation of the function as a hair-generating tissue of the tissue formed in the present example.

The state of the transplantation site was examined by visual observation with the naked eye and also by tissue staining (hematoxylin-eosin staining).

(Results)

In the presence of fat tissue and blood vessels supporting the same, it was possible to induce hairs and hair bulbs in the fat tissue of pedicle tissue graft by optimizing a method of adding the NELL-1 protein (Fig 9). It is supposed that NELL-1 induced dedifferentiation of fat cells and re-differentiation of the cells into hairs and hair bulbs. However, it is unlikely that the fat tissue, which derives from mesoderm, differentiates into hairs and hair bulbs, which are ectoderm-derived epithelial tissues. Observation of the transplanted hair roots showed the presence of hairs, hair bulbs, hair roots and gland tissue associated with hair roots (perspiratory gland). It can be said that the results above indicate that it is possible to form and support hairs in the fat tissue and make them function in the present example.

(Comparative Example 3. Formation of tissue when BMP, TGFβ or FGF is used)

In the present comparative example, an *in vivo* incubator is produced by a method similar to that in Example 3, except that BMP, TGFβ or FGF is used in place of NELL-1. (1) An *in vivo* incubator containing only a buffer solution and (2) an *in vivo* incubator not containing collagen sheets but containing NELL-1 are prepared as negative controls. Each of these *in vivo* incubators is transplanted similarly to Example 1, and whether or not formation of hairs and hair bulbs occurs can be examined.

(Example 4. Transplantation of liver tissue graft)

(Production of *in vivo* incubator)

An *in vivo* incubator was produced by a method similar to that in Example 1. The amount of the NELL-1 protein added to the silicone mold (both sides) is 5 μg/ml, 10 μg/ml or 50 μg/ml.

(Rats used in test)

The rats used were 25 male Wistar rats, 8 weeks of age (supplier: Saitama Experimental Animals Supply Co., Ltd.).

(Collection and inoculation of liver tissue graft)

The xiphisternal region of an animal identical to the rat, into which the *in vivo* incubator is transplanted, was cut open to a length of 1 cm, and part of the liver of 1 cm in length from the end (left lobe) was cut out. The liver graft after removal was cut into 3 to 5 pieces (3 mm square) in physiological saline and the liver sample was placed in presence of fat tissue and blood vessels supporting the same, as it is inoculated in the silicone mold. The dissection site was sutured after hemostasis is confirmed.

(Transplantation)

The *in vivo* incubator produced in the present example was transplanted into a pocket produced by a method similar to that in Example 1. At this time, care was given not to stop blood flow in the pedicle tissue graft.

The rat was anesthetized with diethylether, 2 or 4 weeks after transplantation, and the transplantation site was excised. The region excised was fixed with a 10% neutral formaldehyde solution, to give a paraffin sample. The sample was cut into a slice of 5 μm in thickness and observed in the bright field using an optical microscope after hematoxylin-eosin (HE) staining.

(Confirmation of function of transplanted tissue graft)

The following test is performed to examine that the transplanted tissue supported its function as liver. For regeneration of the functions of large-sized organs and organs demanding an efferent vessel or the like other than blood vessels, such as kidney, ducts and afferent vessels are essential and for that reason, the endocrine system is mainly studied.

The functions of liver are grossly divided into the following three factions:

1) metabolic function: producing proteins by storing and using sugars and fats as energy
2) detoxicating function: detoxicating hazardous substances by metabolism
3) secretion function: secreting bile.

It is possible to examine whether or not liver is functioning *in vivo,* particularly by measuring the amount of albumin production and an increase in the amounts of hepatic cell functional markers (tryptophan oxygenase and the like).

(Results)

In the group of containing 5 μg of NELL-1, the transplanted liver graft supported the shape of its hepatic lobule (Fig. 5).

In the presence of fat tissue and blood vessels supporting the same, addition of a liver tissue graft and NELL-1 protein enabled support of the liver tissue graft, of which the ectopical presence is not allowed *in vivo.*

(B. Heterologous transplantation)

A liver tissue graft was collected from male Wistar rat, 8 weeks of age by a method similar to that in the present example. It was then transplanted similarly to the present example into another rat in the same species. The transplanted liver graft was supported.

(Comparative Example 4A. Influence by use of physiological saline)

In the present comparative example, a method similar to that in Example 4 was used, except that physiological saline was used in place of NELL-1.

In the group containing physiological saline, no transplanted liver graft was observed 4 weeks after transplantation and only the image of fat tissues, macrophages that have absorbed the transplanted liver graft, and the residue after absorption and decomposition were observed (Fig. 4: control group).

(Summary)

When an autologous or heterologous collected liver tissue graft was transplanted with NELL-1 into the *in vivo* incubator produced in the abdomen of a rat, engraftment of the liver tissue graft succeeded 4 weeks after transplantation. There was no growth of the tissue graft observed.

In the group containing physiological saline in place of NELL-1, the liver tissue graft was necrotized or absorbed. It is supposed that the *in vivo* incubator and NELL-1 in combination function as an *"in vivo* incubator". There has been no report on a method effective for transplantation of cells, tissues and organs so far. It is supposed that the method according to the present invention can be a method effective in engraftment of a transplant to the living body without causing an immunological rejection reaction (for example, liver transplantation), regarding the transplantation of cells, tissues or organs.

(Comparative Example 4B. Formation of tissue when BMP, TGFβ or FGF is used)

In the present comparative example, the influence by BMP, TGFβ or FGF on a liver graft can be examined by using a method similar to that in Example 4, except that BMP, TGFβ or FGF was used in place of NELL-1.

(Example 5. Transplantation of ovarian tissue graft)

(Production of *in vivo* incubator)

An *in vivo* incubator was produced by a method similar to that in Example 1. The amount of the NELL-1 protein added to the silicone mold (both sides) is 5 μg/ml, 10 μg/ml or 50 μg/ml.

(Rats used in test)

The rats used were 25 male Wistar rats, 8 weeks of age (supplier: Saitama Experimental Animals Supply Co., Ltd.).

(Collection of ovarian tissue)

The lower abdomen of a rat was cut open from side to side from the rear side. The opening was widened from the back to the abdominal cavity, and the fallopian tube and the fat tissue surrounding the ovary at the end of the fallopian tube were tied up and the right and left ovaries were collected. The excised ovaries were spherical in shape having a diameter of about 5 mm.

(Inoculation of ovarian tissue in *in vivo* incubator)

The ovary was placed in the presence of fat tissue and blood vessels supporting the same, as the ovary collected in the present example was inoculated in an *in vivo* incubator therein.

(Transplantation)

The *in vivo* incubator produced in the present example is transplanted into a pocket produced similarly to

Example 1. At this time, care is given not to stop blood flow. It is cultured in the presence of NELL-1 for 12 weeks.

(Confirmation of functions of transplanted tissue graft)

It is possible to confirm whether or not the transplanted tissue maintains its functions as an ovary by examining the secretion of estrogen, progesterone or the like.

(Results)

In the control group, there was observed a distinct decrease in the amount of bone, because of experimental osteoporosis caused under the influence of ovary excision (Mayahara M, Anat Rec A Discov Mol Cell Evol Biol 2003 Sep; 274 (1) : 817-26).

It is possible to confirm whether or not the ovary-inoculated *in vivo* incubator can prevent a decrease in bone amount after excision of ovary by three-dimensionally measuring the amount of the cancellous bones below the epiphyseal plate, such as thigh bones in the rats used in the test by pCT.

(Comparative Example 5. Formation of tissue when BMP, TGFβ or FGF is used)

In the present comparative example, a method similar to that in Example 5 is used, except that BMP, TGFβ or FGF is used in place of NELL-1. The negative control is a sample containing only a buffer solution. It is possible in this way to examine whether or not an ovarian tissue graft can be supported similarly to Example 5.

(Example 6. Transplantation of tissue grafts of pancreas, adrenal gland, thyroid gland and testis)

(Protocol for collecting tissue)

In the present example, each tissue graft is collected from a rat similar to that used in Example 4.

(Pancreas)

The lower abdominal midline is cut open, and the stomach is pushed upward for exclusion. A tissue graft of about 5 mm square in size is collected from the pancreas, which is found behind the stomach.

(Adrenal gland)

The lower abdomen midline is cut open and the small intestine and duodenum are pushed away for exclusion. A tissue graft of about 5 mm square in size is collected from the adrenal gland, which is found above the kidney.

(Thyroid gland)

The cervical part midline is cut open, and one of the thyroid glands attached to both sides of tracheal cartilage, which is found under the muscle layer, is collected.

(Testis)

The lower abdomen midline is cut open and the testis is excised, as the epithelium is folded in the direction from the pelvis periphery to the testis.

(Transplantation)

Each tissue graft is placed in the presence of fat tissue and blood vessels supporting the same, as each tissue graft collected in the present example is added to a NELL-1-containing *in vivo* incubator by a method similar to that in Example 4. Each *in vivo* incubator is transplanted into the pocket produced by a method similar to that in Example 1. It is possible to confirm whether or not each tissue is supported after transplantation, by observation with the naked eye, histological observation and the like.

(Confirmation of functions of transplanted tissue grafts)

Pancreas: Pancreatic function can be confirmed by measuring an increase in the production of insulin and its precursor substances internally secreted from pancreatic β cell or the like;

Adrenal gland: Adrenal function can be confirmed by confirming secretion of steroid hormones;

Thyroid gland: Thyroid gland function can be confirmed by confirming secretion of thyroid hormones (such as triiodothyronine and thyroxin);

Testis: Testoid function can be confirmed by confirming secretion of testosterone, dihydrotestosterone and dehydroepiandrosterone; and

Kidney: Kidney function can be confirmed by confirming secretion of erythropoietin.

(Comparative Example 6. Formation of tissue when BMP, TGFβ or FGF is used)

In the present comparative example, a method similar to that in Example 6 is used, except that BMP, TGFβ or FGF is used in place of NELL-1. The negative control is a sample containing only a buffer solution. It is possible in this way to examine whether or not the tissue grafts of pancreas, adrenal gland, thyroid gland and testis can be supported in a manner similar to Example 5.

(Example 7. Influence of NELL-1 on cells)

In the present example, the influence of NELL-1 on cells will be examined. The cells used include fat tissue-derived stem cells, hematopoietic stem cells, mesenchymal stem cells, undifferentiated cells contained in organ grafts, somatic stem cells, fat cells and the like. These cells are prepared or available, as described in the following literatures.

Fat tissue-derived stem cells: Zuk PA, Multilineage cells from human adipose tissue: implications for cell-based therapies. Tissue Eng. 2001 Apr; 7 (2): 211-28;

Hematopoietic stem cells and mesenchymal stem cells: Alison MR, Hepatocytes from non-hepatic adult stem cells., Nature. 2000 Jul 20; 406 (6793): 257; and

Undifferentiated cells contained in organ grafts: Ogawa K, Living donor liver transplantation with reduced monosegments for neonates and small infants.: Transplantation. 2007 May 27; 83 (10): 1337-40.

In the present example, it is also possible to examine the influence of NELL-1 on cells by obtaining the cells contained in epithelial or fat tissue.

It is possible to observe the influence of NELL-1 on each cell by adding NELL-1 to these cells at various concentrations (for example, 5 μg/ml, 10 μg/ml and 50 μg/ml).

(Comparative Example 7. Formation of tissue when BMP, TGFβ or FGF is used)

In the present comparative example, it is possible to examine the influence on each cell by using a method similar to that in Example 7, except that BMP, TGFβ or FGF is used in place of NELL-1.

(Example 8. Transplantation of tissues and the like formed to living body)

In the present example, it is possible to examine whether or not the transplanted tissue is supported *in vivo* and whether or not it has a function that the corresponding natural tissue originally has by transplanting the tissue or the like formed in Examples 1 to 3 into an animal and observing the growth of the tissue or the like.

(Example 9. Induction of differentiation to hepatocytes and support thereof)

In the present example, differentiation from stem cells to hepatocytes is induced by using hepatocyte growth factor (HGF), dexamethasone and oncostatin. The hepatocyte growth factor is prepared according to Kamiya A, Oncostatin M and hepatocyte growth factor induce hepatic maturation via distinct signaling pathways. FEBS Lett. 2001 Mar 9; 492 (1-2): 90-4. It is possible to confirm the functions of liver by inoculating hepatocytes in an *in vivo* incubator and measuring 1) metabolic function (of producing proteins by storing and using sugars and fats as energy), 2) detoxicating function (of detoxicating hazardous substances by metabolism), and 3) excretion function (of secreting bile or the like) ,

in particular by measuring the amount of albumin production and an increase in the amounts of hepatic cell function markers (tryptophan oxygenase and the like).

(Example 10. Induction of differentiation to pancreatic β cells and support thereof)

In the present example, differentiation from stem cells to pancreatic β cells is induced by using betacellulin, conophylline or the like. Betacellulin, conophylline or the like is prepared according to Kojima I, Conophylline: a novel differentiation inducer for pancreatic beta cells. Int J Biochem Cell Biol. 2006; 38 (5-6): 923-30.

It is possible to examine the function of the pancreas cells by inoculating cultured cells in an *in vivo* incubator and measuring an increase in the productions of insulin and its precursor substances and the like internally secreted from the pancreatic β cells, or the like.

(Example 11. Comparison with Isogai's method)

The present method is compared with the Isogai's method (Noritaka Isogai, Hirohisa Kusuhara, Yoshito Ikada, Hitoshi Ohtani, Robin Jacquet, Jennifer Hillyer, Elizabeth Lowder, William J. Landis. Tissue Engineering. April 1, 2006, 12 (4): 691-703. doi: 10. 1089/ten. 2006.12. 691), as the Isogai's method is performed.

(Example 12. Production of *in vivo* incubator containing atelocollagen gel and transplantation thereof)

The silicone mold used was a mold produced by a method similar to that in Example 1.

(Production of atelocollagen gel and administration method 1 ml)

The atelocollagen gel was obtained by purchasing a highly controlled medical device, approval number: 16100BZZ01355000.

It was diluted with sterilized PBS so as to have NELL-1 concentrations of 500 μg/ml and 50 μg/ml. The diluted NELL-1 solutions were added respectively to two sterilized collagen gel syringe-shaped package (3%) and the two syringes were connected and pressed to each other alternately for about 15 times for mixing. During use, the dosages of NELL-1 were 50 μg and 5 μg in order to administer the solution in an amount of 0.1 ml.

(Production of *in vivo* incubator)

An *in vivo* incubator was produced by a method similar to that in Example 1, except that the atelocollagen gel prepared in the present example was used in place of using the collagen sponges with NELL-1 protein added dropwise.

(A. Formation of hemopoietic tissue)

A pocket was produced in the muscles in the thigh and the *in vivo* incubator was transplanted in a manner similar to Example 1. The transplantation site was excised 2 or 4 weeks after transplantation and a paraffin sample was produced for hematoxylin-eosin staining. As a result, the formation of hemopoietic tissue was confirmed.

(B. Formation of gland tissue)

A pocket was produced in the muscles in the thigh and the *in vivo* incubator was transplanted in a manner similar to Example 2. The transplantation site was excised 2 or 4 weeks after transplantation and a paraffin sample was produced for hematoxylin-eosin staining. As a result, formation of gland tissue was confirmed.

(C. Formation of gland tissue)

In the present example, an *in vivo* incubator was produced by a method similar to that in Example 3, except that the atelocollagen gel produced in the present example was used in place of using the collagen sponge with NELL-1 protein added dropwise. Hairs or epidermis containing hair roots were collected similarly to Example 3.

The hairs or epidermis containing hair roots collected was inoculated on the atelocollagen gel. A pocket was produced in the muscles in the thigh and an *in vivo* incubator was transplanted in a manner similar to Example 3. The transplantation site was excised 2 or 4 weeks after transplantation and a paraffin sample was produced for hematoxylin-eosin staining. As a result, formations of hairs, hair bulbs, hair roots and gland tissues associated with hair roots (perspiratory glands) were confirmed.

(D. Support of liver tissue graft)

When a liver tissue graft was transplanted to an *in vivo* incubator similarly to Example 4, the transplanted liver tissue graft was supported.

(Example 13. Test by continuous injection of NELL-1)

In the present example, an *in vivo* incubator containing no sustained-releasing scaffold in a collagen sponge, atelocollagen gel and the like was used. The *in vivo* incubator used in the present example was produced in a manner similar to Example 1, except that no sustained-releasing scaffold was contained.

A solution of NELL-1 diluted with physiological saline was administered into the *in vivo* incubator containing no NELL-1 by using a 30G injection needle (common injection needle). The solution was administered by inserting the injection needle into the previously embedded mold through the lower abdomen of a rat under ether anesthesia, as the internal cavity therein was identified with the hardness of the internal mold of the *in vivo* incubator.

In order to confirm secure administration of NELL-1 by the method, a test in which a 50-fold diluted purple staining solution (Carazzi's hematoxylin No, 30020-2, Muto Pure Chemicals Co., Ltd.) was injected before excision is performed for confirmation of secure administration of NELL-1 (Figs. 7 and 8).

It was possible by the administration method to keep the concentration of NELL-1high in the mold by administering NELL-1 not only once but several times during operation.

In the present example, a total of 20 μg/ml was administered every week for four weeks (50 μg/l ml NELL1 solution, 0.1 ml). The transplantation site was then excised, a paraffin sample was produced, and the tissue image was confirmed by hematoxylin-eosin staining according to a method similar to that in Example 1. As a result, the hemopoietic or gland tissue was confirmed. The result was the same as that when collagen sponge was used.

Similarly to Example 3, hairs, hair bulbs, hair roots and gland tissues associated with hair roots (perspiratory glands) were confirmed, when hairs and epidermis containing hair roots was transplanted into an *in vivo* incubator.

These results indicate that formation of each tissue is not influenced by the presence or absence of sustained-releasing scaffold and formation of the tissue and the like could be confirmed when the concentration of NELL-1 in the mold was kept constant.

In addition, when a liver tissue graft was transplanted into an *in vivo* incubator similarly to Example 4, the transplanted liver tissue graft was supported.

(Example 14. Production in the presence of muscular tissue and blood vessels supporting the same in *in vivo* incubator and transplantation thereof)

In the present example, a pedicle tissue graft containing a muscular tissue and blood vessels supporting the muscular tissue was used in place of the pedicle tissue graft containing a fat tissue and hypogastric artery and vein. *In vivo* incubators (*in vivo* incubator containing a collagen sponge, *in vivo* incubator containing an atelocollagen gel, and *in vivo* incubator not containing sustained-releasing scaffold) were produced by a method similar to those in Examples 1, 12 and 13, except the method above. When the *in vivo* incubator not containing sustained-releasing scaffold was transplanted, NELL-1 was injected externally, similarly to Example 13.

(A. Formation of hemopoietic tissue)

A pocket was produced in the muscles in the thigh and each *in vivo* incubator was transplanted, similarly to Example 1. The transplantation site was excised 2 or 4 weeks after transplantation and a paraffin sample was produced for hematoxylin-eosin staining. As a result, formation of hemopoietic tissue was confirmed.

(B. Formation of gland tissue)

A pocket was produced in the muscles in the thigh and each *in vivo* incubator was transplanted, similarly to Example 2. The transplantation site was excised 2 or 4 weeks after transplantation and a paraffin sample was produced for hematoxylin-eosin staining. As a result, formation of gland tissue was confirmed.

(C. Formation of gland tissue)

In the present example, each *in vivo* incubator was produced by a method similar to that in Example 3, except that the atelocollagen gel prepared in the present example was used in place of the collagen sponge containing NELL-1 protein added dropwise. Hairs or epidermis containing hair roots were collected, similarly to Example 3.

The hairs or epidermis containing hair roots collected were inoculated on the atelocollagen gel. A pocket was produced in the muscles in the thigh and each *in vivo* incubator was transplanted, similarly to Example 3. The transplantation site was excised 2 or 4 weeks after transplantation and a paraffin sample was produced for hematoxylin-eosin staining. As a result, formations of hairs, hair bulbs, hair roots and gland tissues associated with hair roots (perspiratory glands) were confirmed.

(D. Support of liver tissue graft)

When a liver tissue graft was transplanted into an *in vivo* incubator similarly to Example 4, the transplanted liver tissue graft was supported.

INDUSTRIAL APPLICABILITY

The present invention has usefulness in that it provides a technique for forming and/or supporting a differentiated cell, tissue and organ.
The present invention provides usefulness such that it is possible to protect a differentiated cell, tissue and organ transplanted into the living body from an immunological rejection reaction.

SEQUENCE LISTING

## Claims

1. A composition for inducing differentiation of a cell having been directed to a given differentiation, to a further differentiated cell, tissue or organ directed to the given differentiation, wherein the composition comprises NELL-1 or a substance which is altered to function as NELL-1 at the time of said inducing differentiation.

2. The composition according to claim 1, wherein the composition is for heterotopically forming said further differentiated cell, tissue or organ directed to the given differentiation in the presence of fat tissue and blood vessels wherein the blood vessels support the fat tissue.

3. The composition according to claim 1, wherein said cell having been directed to a given differentiation is a somatic stem cell.

4. The composition according to claim 3, wherein said somatic stem cell is a somatic stem cell present in a tissue selected from the group consisting of hematopoietic tissue, epithelial tissue, connective tissue, muscle tissue and nerve tissue.

5. The composition according to claim 4, wherein said somatic stem cell is a stem cell having been directed to differentiation of hematopoietic tissue, epithelial tissue or connective tissue.

6. The composition according to claim 5, wherein the somatic stem cell present in the hematopoietic tissue is a hematopoietic stem cell, which is a mother cell of a blood cell having self repair capability and multipotency.

7. The composition according to claim 5, wherein the somatic stem cell present in the epithelial tissue is a stem cell having been directed to differentiation of epithelial tissue, which is a mother cell of epithelial system cell having self repair capability and multipotency.

8. The composition according to claim 5, wherein the somatic stem cell present in the epithelial cell is adenoblast or a cell included in a hair root.

9. The composition according to claim 1, wherein the differentiated cell, tissue or organ is differentiated hematopoietically or epithelially.

10. The composition according to claim 9, wherein the hematopoietically differentiated cell, tissue or organ is a blood cell differentiated from hematopoietic stem cell, selected from the group consisting of a leukocyte selected from the group consisting of neutrophil, eosinophil, basophil and lymphocyte; erythrocyte; platelet; macrophage; and a combination thereof.

**11.** The composition according to claim 9, wherein the epithelially differentiated cell, tissue or organ is exocrine gland and a duct thereof.

**12.** The composition according to claim 11, wherein the exocrine gland is selected from the group consisting of perspiratory gland, sebaceous gland, intestinal gland, gastric gland and salivary gland.

**13.** The composition according to claim 9, wherein the epithelially differentiated cell, tissue or organ is hair, hair bulb, hair root or a gland tissue associated with hair root.

**14.** The composition according to claim 1, wherein said cell having been directed to a given differentiation is adenoblast, and said differentiated cell, tissue or organ is exocrine gland and a duct thereof.

**15.** The composition according to claim 1, wherein said cell having been directed to a given differentiation is a cell included in a hair root, and said differentiated cell, tissue or organ is hair, hair bulb, hair root or a gland tissue associated with hair root.

**16.** The composition according to claim 1, which comprises said NELL-1 at about 0.01 μg/ml or more.

**17.** The composition according to claim 16, which comprises said NELL-1 from about 5 μg/ml to about 50 μg/ml.

**18.** The composition according to claim 1, wherein said differentiated cell, tissue or organ has a function corresponding to that present in nature.

**19.** A material for inducing differentiation of a cell having been directed to a given differentiation, to a further differentiated cell, tissue or organ directed to the given differentiation, said material comprises:

A) a sustained-releasing scaffold; and
B) NELL-1 or a substance which is altered to function as NELL-1 at the time of said inducing differentiation.

**20.** The material according to claim 19, wherein the material is for heterotopically forming a further differentiated cell, tissue or organ directed to the given differentiation in the presence of fat tissue and blood vessels wherein the blood vessels support the fat tissue.

**21.** The material according to claim 19, wherein said sustained-releasing scaffold is an extracellular matrix.

**22.** The material according to claim 21, wherein said sustained-releasing scaffold is selected from the group consisting of collagen and atelocollagen.

**23.** The material according to claim 19, wherein said cell having been directed to a given differentiation is a somatic stem cell.

**24.** The material according to claim 23, wherein said somatic stem cell is a somatic stem cell present in a tissue selected from the group consisting of hematopoietic tissue, epithelial tissue, connective tissue, muscle tissue and nerve tissue.

**25.** The material according to claim 24, wherein said somatic stem cell is a somatic stem cell present in hematopoietic tissue, epithelial tissue or connective tissue.

**26.** The material according to claim 25, wherein the somatic stem cell present in the hematopoietic tissue is a hematopoietic stem cell, which is a mother cell of a blood cell having self repair capability and multipotency.

**27.** The material according to claim 25, wherein the somatic stem cell present in the epithelial tissue is a stem cell having been directed to differentiation of epithelial tissue, which is a mother cell of epithelial system cell having self repair capability and multipotency.

**28.** The material according to claim 25, wherein the somatic stem cell present in the epithelial cell is adenoblast or a cell included in a hair root.

**29.** The material according to claim 25, wherein the differentiated cell, tissue or organ is differentiated hematopoietically

or epithelially.

**30.** The material according to claim 29, wherein the hematopoietically differentiated cell, tissue or organ is a blood cell differentiated from hematopoietic stem cell, selected from the group consisting of a leukocyte selected from the group consisting of neutrophil, eosinophil, basophil and lymphocyte; erythrocyte; platelet; macrophage; and a combination thereof.

**31.** The material according to claim 29, wherein the epithelially differentiated cell, tissue or organ is exocrine gland and a duct thereof.

**32.** The material according to claim 31, wherein the exocrine gland is selected from the group consisting of perspiratory gland, sebaceous gland, intestinal gland, gastric gland and salivary gland.

**33.** The material according to claim 29, wherein the epithelially differentiated cell, tissue or organ is hair, hair bulb, hair root or a gland tissue associated with hair root.

**34.** The material according to claim 19, wherein said cell having been directed to a given differentiation is adenoblast, and said differentiated cell, tissue or organ is exocrine gland and a duct thereof.

**35.** The material according to claim 19, wherein said cell having been directed to a given differentiation is a cell included in a hair root, and said differentiated cell, tissue or organ is hair, hair bulb, hair root or a gland tissue associated with hair root.

**36.** The material according to claim 19, wherein said NELL-1 is comprised at about 0.01 μg/ml or more.

**37.** The material according to claim 36, wherein said NELL-1 is comprised from about 5 μg/ml to about 50 μg/ml.

**38.** The material according to claim 19, wherein said differentiated cell, tissue or organ has a function corresponding to that present in nature.

**39.** A kit for inducing differentiation of a cell having been directed to a given differentiation, to a further differentiated cell, tissue or organ directed to the given differentiation, wherein said kit comprises NELL-1 or a substance which is altered to function as NELL-1 at the time of said inducing differentiation.

**40.** The kit according to claim 39, wherein the kit is for heterotopically forming a further differentiated cell, tissue or organ directed to the given differentiation in the presence of fat tissue and blood vessels wherein the blood vessels support the fat tissue.

**41.** The kit according to claim 40, wherein the differentiated cell, tissue or organ is differentiated hematopoietically or epithelially.

**42.** The kit according to claim 41, wherein the hematopoietically differentiated cell, tissue or organ is a blood cell differentiated from hematopoietic stem cell, selected from the group consisting of a leukocyte selected from the group consisting of neutrophil, eosinophil, basophil and lymphocyte; erythrocyte; platelet; macrophage; and a combination thereof.

**43.** The kit according to claim 41, wherein the epithelially differentiated cell, tissue or organ is exocrine gland and a duct thereof.

**44.** The kit according to claim 43, wherein the exocrine gland is selected from the group consisting of perspiratory gland, sebaceous gland, intestinal gland, gastric gland and salivary gland.

**45.** The kit according to claim 41, the epithelially differentiated cell, tissue or organ is hair, hair bulb, hair root or a gland tissue associated with hair root.

**46.** The kit according to claim 39, wherein said NELL-1 is comprised at about 0.01 μg/ml or more.

**47.** The kit according to claim 46, wherein said NELL-1 is comprised from about 5 μg/ml to about 50 μg/ml.

**48.** A medical device for inducing differentiation of a cell having been directed to a given differentiation, to a further differentiated cell, tissue or organ directed to the given differentiation, wherein said medical device comprises:

A) NELL-1 or a substance which is altered to function as NELL-1 at the time of said inducing differentiation;
B) a sustained-releasing scaffold; and
C) a container.

**49.** The medical device according to claim 48, wherein
said medical device is for heterotopically forming a further differentiated cell, tissue or organ directed to the given differentiation in the presence of fat tissue and blood vessels.

**50.** The medical device according to claim 48, wherein
said sustained-releasing scaffold is an extracellular matrix.

**51.** The medical device according to claim 48, wherein said sustained-releasing scaffold is selected from the group consisting of collagen and atelocollagen.

**52.** The medical device according to claim 48, wherein the differentiated cell, tissue or organ is differentiated hematopoietically or epithelially.

**53.** The medical device according to claim 52, wherein the hematopoietically differentiated cell, tissue or organ is a blood cell differentiated from hematopoietic stem cell, selected from the group consisting of a leukocyte selected from the group consisting of neutrophil, eosinophil, basophil and lymphocyte; erythrocyte; platelet; macrophage; and a combination thereof.

**54.** The medical device according to claim 52, wherein the epithelially differentiated cell, tissue or organ is exocrine gland and a duct thereof.

**55.** The medical device according to claim 54, wherein the exocrine gland is selected from the group consisting of perspiratory gland, sebaceous gland, intestinal gland, gastric gland and salivary gland.

**56.** The medical device according to claim 52, wherein the epithelially differentiated cell, tissue or organ is hair, hair bulb, hair root or a gland tissue associated with hair root.

**57.** The medical device according to claim 48, wherein said NELL-1 is comprised at about 0.01 μg/ml or more.

**58.** The medical device according to claim 55, wherein said NELL-1 is comprised from about 5 μg/ml to about 50 μg/ml.

**59.** A method for producing a cell, tissue or organ, wherein said method comprises the steps of:

providing a cell having been directed to a given differentiation; and
contacting said cell having been directed to a given differentiation with NELL-1 or a substance which is altered to function as NELL-1 at the time of said inducing differentiation.

**60.** The method according to claim 59, wherein said cell having been directed to a given differentiation is provided in the presence of fat tissue and blood vessels supporting the same.

**61.** The method according to claim 59, wherein said cell having been directed to a given differentiation is contacted with said NELL-1 on a sustained-releasing scaffold.

**62.** The method according to claim 61, wherein said sustained-releasing scaffold is an extracellular matrix.

**63.** The method according to claim 62, wherein said sustained-releasing scaffold is selected from the group consisting of collagen and atelocollagen.

**64.** The method according to claim 59, wherein said cell having been directed to a given differentiation is a somatic stem cell.

**65.** The method according to claim 62, wherein said somatic stem cell is a somatic stem cell present in a tissue selected from the group consisting of hematopoietic tissue, epithelial tissue, connective tissue, muscle tissue and nerve tissue.

**66.** The method according to claim 65, wherein said somatic stem cell is a somatic stem cell present in hematopoietic tissue, epithelial tissue or connective tissue.

**67.** The method according to claim 66, wherein the somatic stem cell present in the hematopoietic tissue is a hematopoietic stem cell, which is a mother cell of a blood cell having self repair capability and multipotency.

**68.** The method according to claim 66, wherein the somatic stem cell present in the epithelial tissue is a stem cell having been directed to differentiation of epithelial tissue, which is a mother cell of epithelial system cell having self repair capability and multipotency.

**69.** The method according to claim 66, wherein the somatic stem cell present in the epithelial cell is adenoblast or a cell included in a hair root.

**70.** A composition for supporting a differentiated cell, tissue or organ, wherein said composition comprises NELL-1 or a substance which is altered to function as NELL-1 at the time of said inducing differentiation.

**71.** The composition according to claim 70, wherein said support is carried out at a location which does not allow presence of said differentiated cell, tissue or organ.

**72.** The composition according to claim 70, wherein said differentiated cell, tissue or organ has a function corresponding to that present in nature.

**73.** The composition according to claim 70, wherein said differentiated cell, tissue or organ is selected from the group consisting of liver, kidney, pancreas, adrenal gland, thyroid gland, ovary and testis.

**74.** A method for supporting a differentiated cell, tissue or organ, wherein said method comprises the steps of:

providing said differentiated cell, tissue or organ; and
giving NELL-1 or a substance which is altered to function as NELL-1 at the time of said inducing differentiation to said differentiated cell, tissue or organ.

**75.** Use of NELL-1 or a substance which is altered to function as NELL-1 at the time of inducing differentiation in the manufacture of a medicament for producing a differentiated cell, tissue or organ from a cell having been directed to a given differentiation.

**76.** Use of NELL-1 or a substance which is altered to function as NELL-1 at the time of inducing differentiation in the manufacture of a medicament for supporting a differentiated cell, tissue or organ.

**77.** A method for producing a gland tissue in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing a sustained-releasing scaffold comprising NELL-1 in the container and inserting the pedicle tissue graft thereinto;
D) transplanting the container into the space in a state in which blood stream in the pedicle tissue graft is not blocked;
E) maintaining the container in a state in which blood stream in the pedicle tissue graft is not blocked for a period sufficient to produce said gland tissue.

**78.** The method according to claim 77 for producing a gland tissue in a fat tissue transplanted in the thigh of a mammal, wherein said step A) is a step of conducting skin incision in the thigh of the mammal and ablating hypogastric artery and vein branched from femoral artery and vein, and nerve running in parallel, from surrounding tissues;

wherein said step B) is a step of resecting abdominal subcutaneous fat tissue of the mammal to a size in accordance with the container centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml, said sustained-releasing scaffold is a collagen sponge or an atelocollagen gel; and
wherein in said step E), said period sufficient to produce said gland tissue is at least about two weeks.

**79.** The method according to claim 77 for producing a gland tissue in a muscle tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting a muscle tissue to a size in accordance with the container centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml, said sustained-releasing scaffold is a collagen sponge or an atelocollagen gel; and
wherein in said step E), said period sufficient to produce said gland tissue is at least about two weeks.

**80.** A method for producing a gland tissue in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing the pedicle tissue graft into the container and adding NELL-1 thereinto;
D) transplanting the container into the space in a state in which blood stream in the pedicle tissue graft is not blocked;
E) maintaining the container in a state in which blood stream in the pedicle tissue graft is not blocked for a period sufficient to produce said gland tissue, wherein NELL-1 is added to the pedicle tissue graft periodically during the maintenance.

**81.** The method according to claim 80 for producing a gland tissue in a fat tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting abdominal subcutaneous fat tissue of the mammal to a size in accordance with the container centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml;
wherein said step D) is a step of transplanting the container between muscles of the thigh in a state in which blood stream in the pedicle tissue graft is not blocked; and
wherein in said step E), said period sufficient to produce said gland tissue is at least about two weeks, NELL-1 is added to the pedicle tissue graft once a week from about 5 μg/ml to about 50 μg/ml during the maintenance.

**82.** The method according to claim 80 for producing a gland tissue in a muscle tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting a muscle tissue to a size in accordance with the container centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml;
wherein said step D) is a step of transplanting the container between muscles of the thigh in a state in which blood stream in the pedicle tissue graft is not blocked; and
wherein in said step E), said period sufficient to produce said gland tissue is at least about two weeks, NELL-1 is added to the pedicle tissue graft once a week from about 5 μg/ml to about 50 μg/ml during the maintenance.

**83.** A method for producing a hematopoietic tissue in a fat tissue or muscle tissue having been transplanted in the living

body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing a sustained-releasing scaffold comprising NELL-1 in the container and inserting the pedicle tissue graft thereinto;
D) transplanting the container into the space in a state in which blood stream in the pedicle tissue graft is not blocked;
E) maintaining the container in a state in which blood stream in the pedicle tissue graft is not blocked for a period sufficient to produce said hematopoietic tissue.

**84.** The method according to claim 83 for producing a hematopoietic tissue in a fat tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting abdominal subcutaneous fat tissue of the mammal to a size in accordance with the container centered at the branch point of the hypogastric artery and vein and producing a pedicle tissue graft comprising the hypogastric artery and vein as the blood vessel pedicle;
wherein in said step C), said NELL-1 is comprised from about 5 $\mu$g/ml to about 50 $\mu$g/ml, said sustained-releasing scaffold is a collagen sponge or an atelocollagen gel; and
wherein in said step E), said period sufficient to produce said hematopoietic tissue is at least about two weeks.

**85.** The method according to claim 83 for producing a hematopoietic tissue in a muscle tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting a muscle tissue to a size in accordance with the container centered at the branch point of the blood vessel supporting the muscle tissue of the mammal and producing a pedicle tissue graft comprising the blood as pedicle;
wherein in said step C), said NELL-1 is comprised from about 5 $\mu$g/ml to about 50 $\mu$g/ml, said sustained-releasing scaffold is a collagen sponge or an atelocollagen gel; and
wherein in said step E), said period sufficient to produce said hematopoietic tissue is at least about two weeks.

**86.** A method for producing a hematopoietic tissue in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing the pedicle tissue graft into the container and adding NELL-1 thereinto;
D) transplanting the container into the space in a state in which blood stream in the pedicle tissue graft is not blocked; and
E) maintaining the container in a state in which blood stream in the pedicle tissue graft is not blocked for a period sufficient to produce said hematopoietic tissue, wherein NELL-1 is added to the pedicle tissue graft periodically during the maintenance.

**87.** The method according to claim 86 for producing a hematopoietic tissue in a fat tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting abdominal subcutaneous fat tissue of the mammal to a size in accordance with the container centered at the branch point of the hypogastric artery and vein and producing a pedicle tissue graft comprising the hypogastric artery and vein as the blood vessel pedicle;
wherein in said step C), said NELL-1 is comprised from about 5 $\mu$g/ml to about 50 $\mu$g/ml;
wherein said step D) is a step of transplanting the container between muscles of the thigh in a state in which blood

stream in the pedicle tissue graft is not blocked; and
wherein in said step E), said period sufficient to produce said hematopoietic tissue is at least about two weeks, NELL-1 is added to the pedicle tissue graft once a week from about 5 μg/ml to about 50 μg/ml during the maintenance.

**88.** The method according to claim 86 for producing a hematopoietic tissue in a muscle tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting a muscle tissue to a size in accordance with the container centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml;
wherein said step D) is a step of transplanting the container between muscles of the thigh in a state in which blood stream in the pedicle tissue graft is not blocked; and
wherein in said step E), said period sufficient to produce said hematopoietic tissue is at least about two weeks, NELL-1 is added to the pedicle tissue graft once a week from about 5 μg/ml to about 50 μg/ml during the maintenance.

**89.** A method for producing hair, hair bulb, hair root or a gland tissue associated with hair root in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing a sustained-releasing scaffold comprising NELL-1 in the container;
D) placing a cell included in hair root on the sustained-releasing scaffold;
E) inserting the pedicle tissue graft into the container;
F) transplanting the container into the space in a state in which blood stream in the pedicle tissue graft is not blocked; and
G) maintaining the container in a state in which blood stream in the pedicle tissue graft is not blocked for a period sufficient to produce said hair, hair bulb, hair root or a gland tissue associated with hair root.

**90.** The method according to claim 89 for producing hair, hair bulb, hair root or a gland tissue associated with hair root in a fat tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting abdominal subcutaneous fat tissue of the mammal to a size in accordance with the container centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml, said sustained-releasing scaffold is a collagen sponge or an atelocollagen gel;
wherein said step F) is a step of transplanting the container between muscles of the thigh in a state in which blood stream in the pedicle tissue graft is not blocked; and
wherein in said step G), said period sufficient to produce said hair, hair bulb, hair root or a gland tissue associated with hair root is at least about two weeks.

**91.** The method according to claim 89 for producing a hair, hair bulb, hair root or a gland tissue associated with hair root in a muscle tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting a muscle tissue to a size in accordance with the container centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml, said sustained-releasing scaffold is a collagen sponge or an atelocollagen gel;
wherein said step F) is a step of transplanting the container between muscles of the thigh in a state in which blood stream in the pedicle tissue graft is not blocked; and
wherein in said step G), said period sufficient to produce said hair, hair bulb, hair root or a gland tissue associated

with hair root is at least about two weeks.

**92.** A method for producing hair, hair bulb, hair root or a gland tissue associated with hair root in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing the pedicle tissue graft into the container and adding a cell contained in hair root and NELL-1 thereinto;
D) transplanting the container into the space in a state in which blood stream in the pedicle tissue graft is not blocked;
E) maintaining the container in a state in which blood stream in the pedicle tissue graft is not blocked for a period sufficient to produce said hair, hair bulb, hair root or a gland tissue associated with hair root, wherein NELL-1 is added to the pedicle tissue graft periodically during the maintenance.

**93.** The method according to claim 92 for producing hair, hair bulb, hair root or a gland tissue associated with hair root in a fat tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting abdominal subcutaneous fat tissue of the mammal to a size in accordance with the container centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml;
wherein said step D) is a step of transplanting the container between muscles of the thigh in a state in which blood stream in the pedicle tissue graft is not blocked; and
wherein in said step E), said period sufficient to produce said hair, hair bulb, hair root or a gland tissue associated with hair root is at least about two weeks, NELL-1 is added to the pedicle tissue graft once a week from about 5 μg/ml to about 50 μg/ml during the maintenance.

**94.** The method according to claim 92 for producing hair, hair bulb, hair root or a gland tissue associated with hair root in a muscle tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting a muscle tissue to a size in accordance with the container centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml;
wherein said step D) is a step of transplanting the container between muscles of the thigh in a state in which blood stream in the pedicle tissue graft is not blocked; and
wherein in said step E), said period sufficient to produce said hair, hair bulb, hair root or a gland tissue is at least about two weeks, NELL-1 is added to the pedicle tissue graft once a week from about 5 μg/ml to about 50 μg/ml during the maintenance.

**95.** A method for supporting a liver tissue graft in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing a sustained-releasing scaffold comprising NELL-1 in the container;
D) placing said liver tissue graft on the sustained-releasing scaffold;
E) inserting the pedicle tissue graft into the container;
F) transplanting the container into the space in a state in which blood stream in the pedicle tissue graft is not blocked;
G) placing the pedicle tissue graft in a state in which blood stream thereof is not blocked.

**96.** The method according to claim 95 for supporting a liver tissue graft in a fat tissue transplanted in the thigh of a mammal, wherein said step A) is a step of conducting skin incision in the thigh of the mammal and ablating hypogastric artery and vein branched from femoral artery and vein, and nerve running in parallel, from surrounding tissues;
wherein said step B) is a step of resecting abdominal subcutaneous fat tissue of the mammal to a size in accordance with the container centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml, said sustained-releasing scaffold is a collagen sponge or an atelocollagen gel; and
wherein said step F) is a step of transplanting the container between muscles of the thigh in a state in which blood stream in the pedicle tissue graft is not blocked.

**97.** The method according to claim 95 for supporting a liver tissue graft in a muscle tissue transplanted in the thigh of a mammal,
wherein said step A) is a step of conducting skin incision in the thigh of the mammal and ablating hypogastric artery and vein branched from femoral artery and vein, and nerve running in parallel, from surrounding tissues;
wherein said step B) is a step of resecting a muscle tissue to a size in accordance with the container centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml, said sustained-releasing scaffold is a collagen sponge or an atelocollagen gel; and
wherein said step F) is a step of transplanting the container between muscles of the thigh in a state in which blood stream in the pedicle tissue graft is not blocked.

**98.** A method for supporting a liver tissue graft in a fat tissue or muscle tissue having been transplanted in the living body, wherein said method comprises the steps of:

A) producing a space for transplanting a container in the living body;
B) resecting a fat tissue or a muscle tissue together with blood vessels supporting the fat tissue or the muscle tissue from the living body in accordance with the size of the container to produce a pedicle tissue graft comprising a blood vessel pedicle;
C) placing the pedicle tissue graft into the container and adding a liver tissue graft and NELL-1 thereinto;
D) transplanting the container into the space in a state in which blood stream in the pedicle tissue graft is not blocked; and
E) placing the pedicle tissue graft in a state in which blood stream thereof is not blocked.

**99.** The method according to claim 98 for supporting a liver tissue graft in a fat tissue transplanted in the thigh of a mammal, wherein said step A) is a step of conducting skin incision in the thigh of the mammal and ablating hypogastric artery and vein branched from femoral artery and vein, and nerve running in parallel, from surrounding tissues;
wherein said step B) is a step of resecting abdominal subcutaneous fat tissue of the mammal to a size in accordance with the container centered at the branch point of the hypogastric artery and vein and producing a pedicle tissue graft comprising the hypogastric artery and vein as the blood vessel pedicle;
wherein in said step C), said NELL-1 is comprised from about 5 μg/ml to about 50 μg/ml;
wherein said step D) is a step of transplanting the container between muscles of the thigh in a state in which blood stream in the pedicle tissue graft is not blocked; and
wherein in said step E), NELL-1 is added to the pedicle tissue graft once a week from about 5 μg/ml to about 50μg/ml during the maintenance.

**100.** The method according to claim 98 for supporting a liver tissue graft in a muscle tissue transplanted in the thigh of a mammal, wherein said step A) is a step of conducting skin incision in the thigh of the mammal and ablating hypogastric artery and vein branched from femoral artery and vein, and nerve running in parallel, from surrounding tissues;
wherein said step B) is a step of resecting a muscle tissue to a size in accordance with the container centered at the branch point of the blood vessel supporting the muscle tissue of the mammal and producing a pedicle tissue graft comprising the blood as pedicle;
wherein in said step C), said NELL-1 is comprised from about 5 μg/ml to about 50 μg/ml;
wherein said step D) is a step of transplanting the container between muscles of the thigh in a state in which blood stream in the pedicle tissue graft is not blocked; and
wherein in said step E), NELL-1 is added to the pedicle tissue graft once a week from about 5 μg/ml to about 50 μg/ml during the maintenance.

**Fig. 1A**

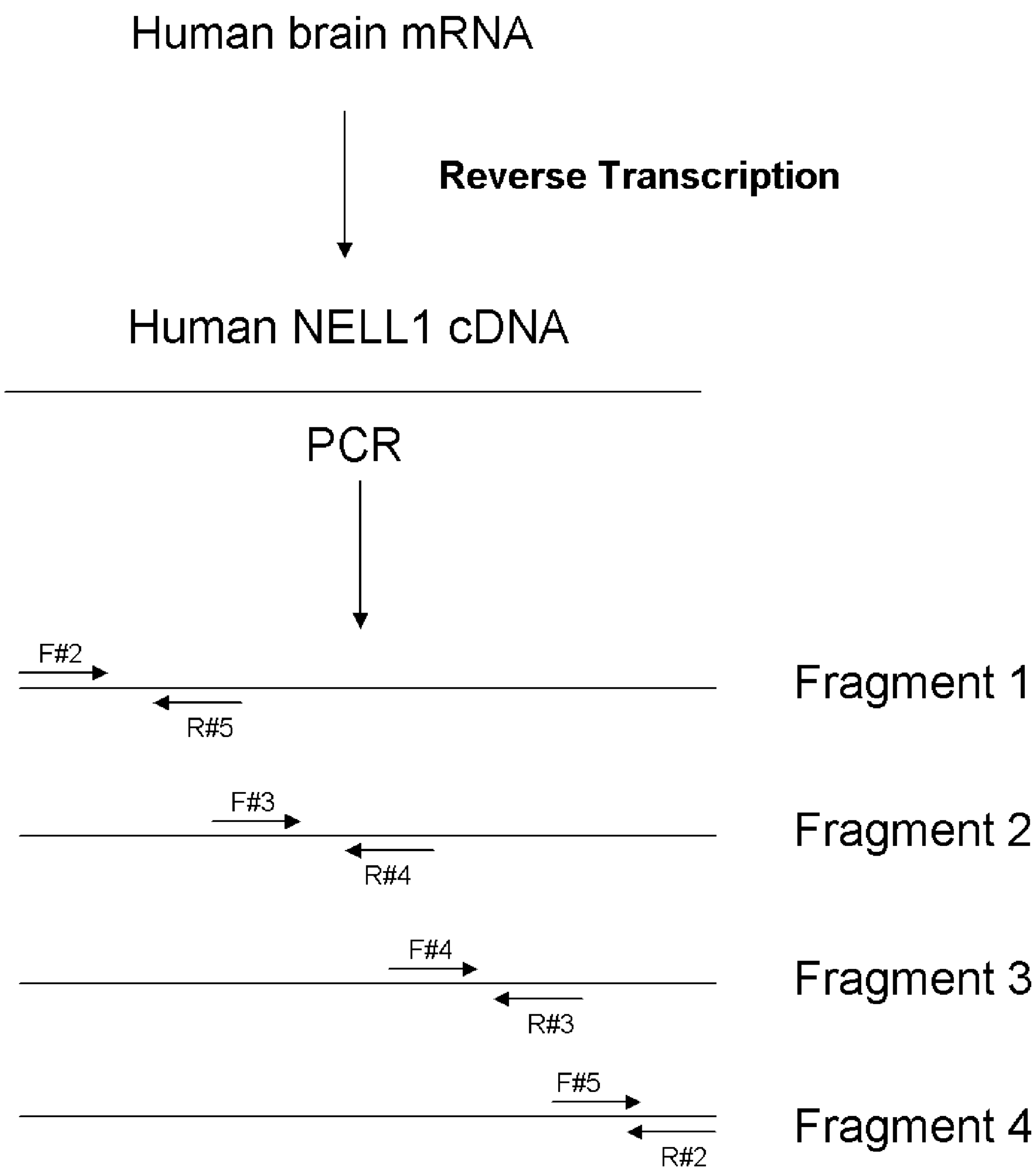
Human brain mRNA
Reverse Transcription
Human NELL1 cDNA
PCR
F#2
R#5
Fragment 1
F#3
R#4
Fragment 2
F#4
R#3
Fragment 3
F#5
R#2
Fragment 4

**Fig. 1B**

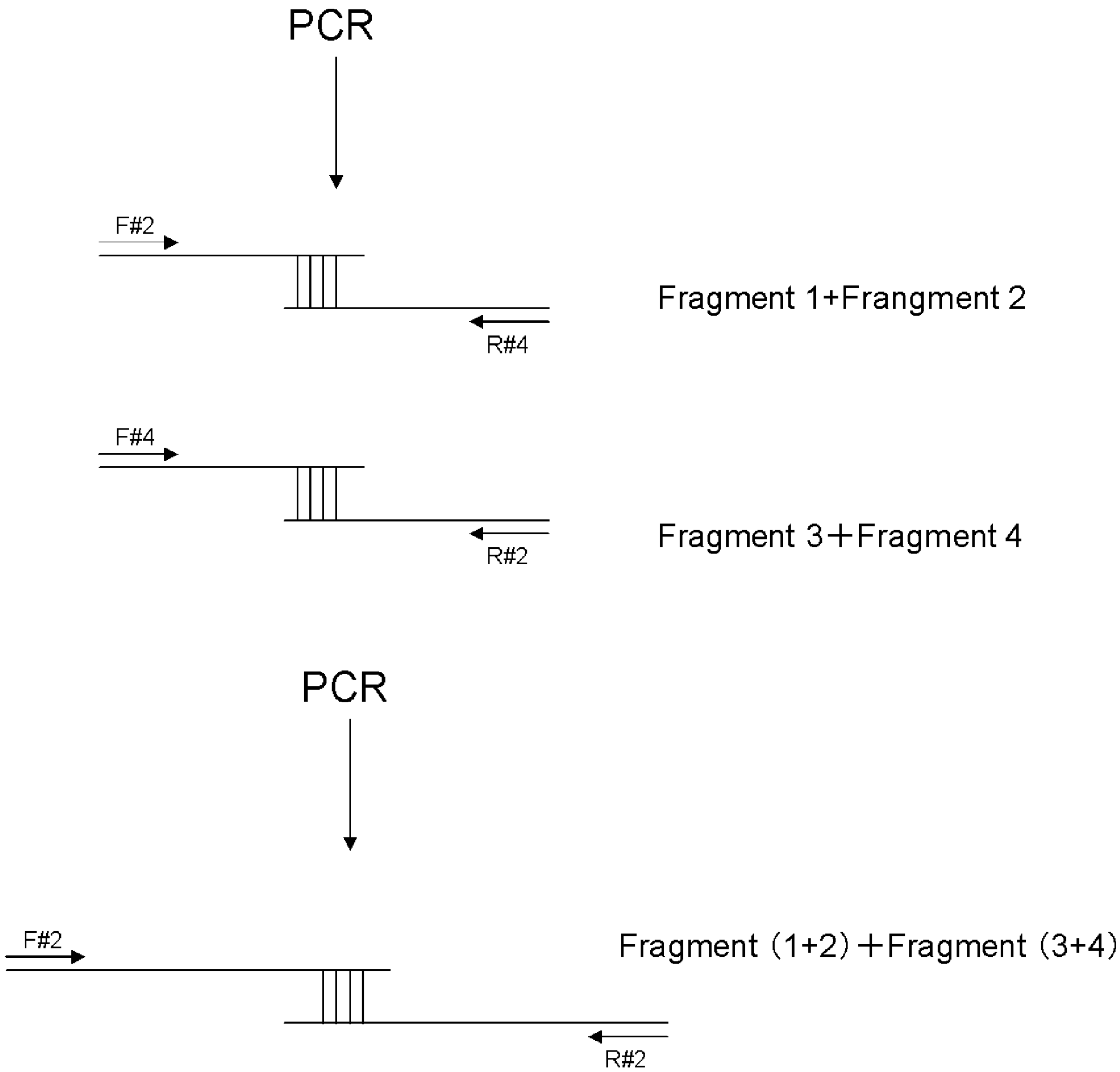
PCR
F#2
R#4
Fragment 1+Frangment 2
F#4
R#2
Fragment 3＋Fragment 4
PCR
F#2
R#2
Fragment (1+2)＋Fragment (3+4)

Fig. 1C

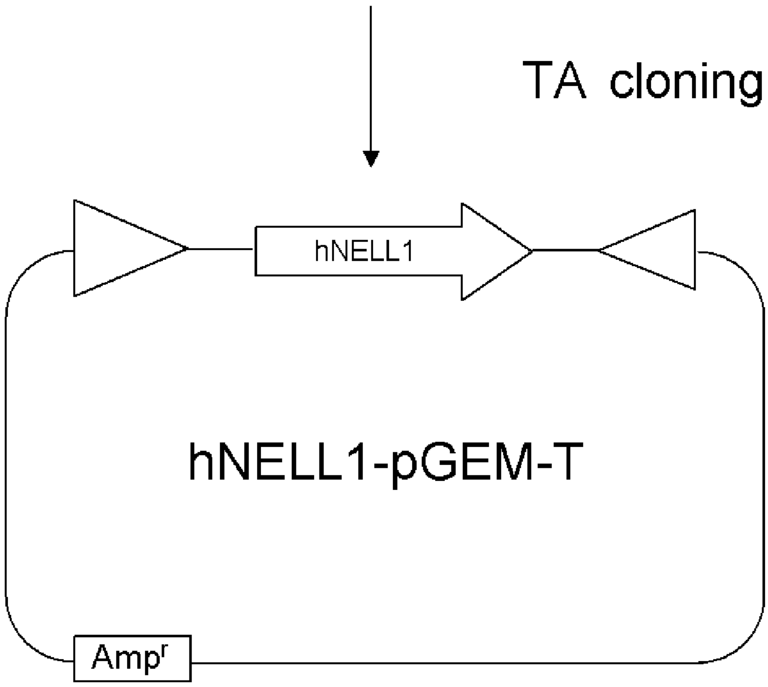

Fig. 1D

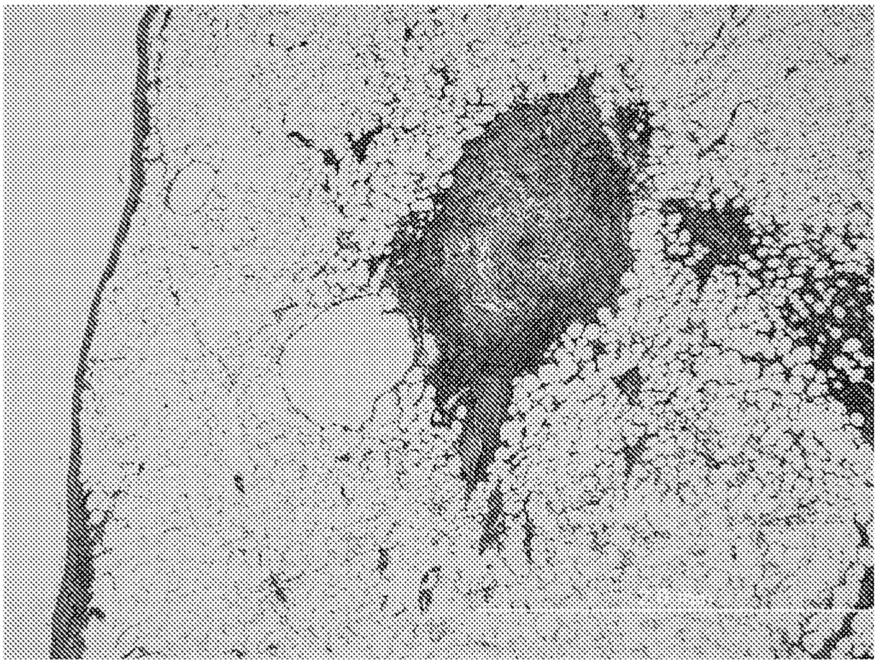

Fig. 2

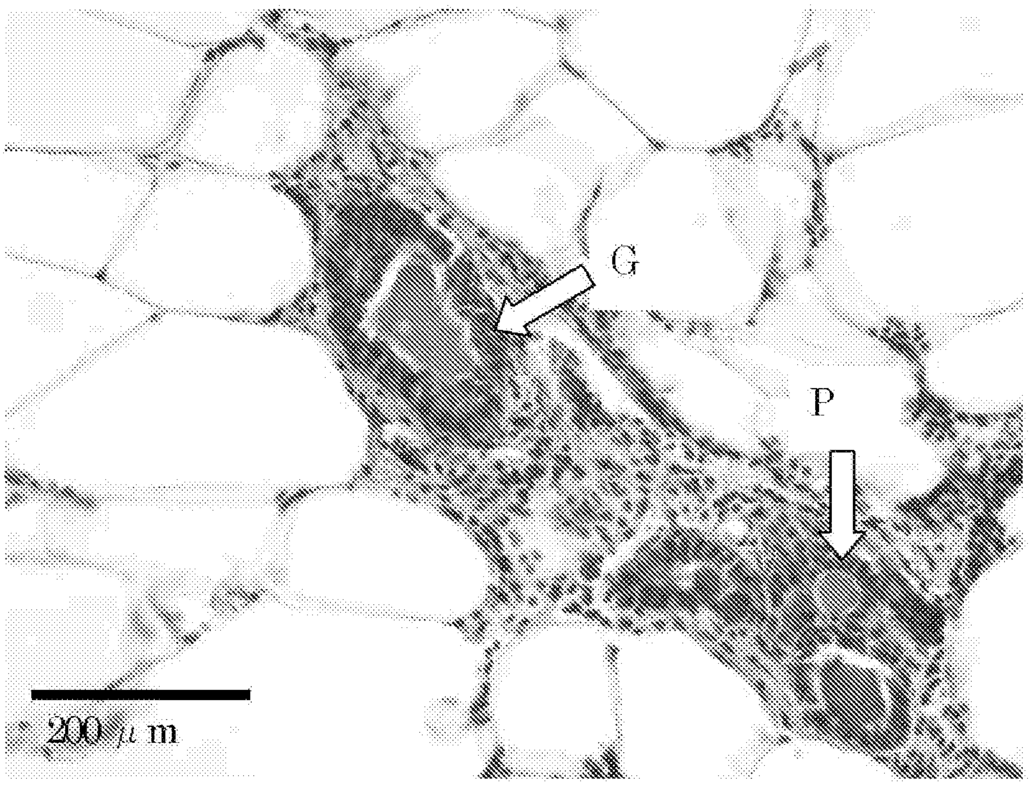

Fig. 3

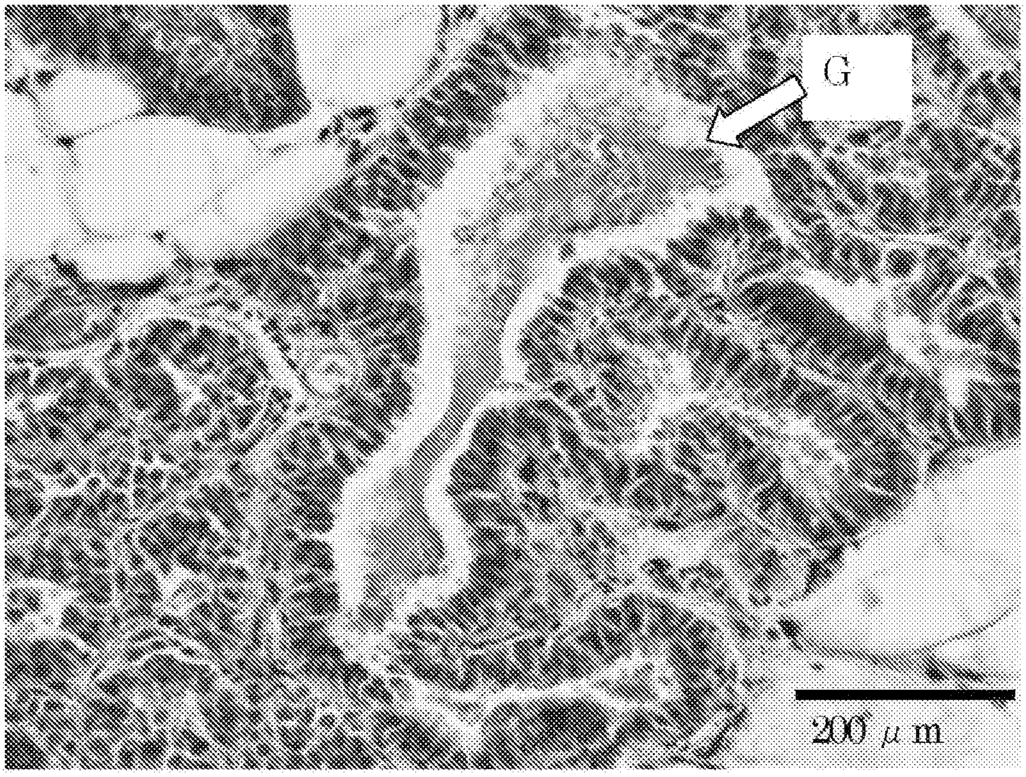

Fig. 4

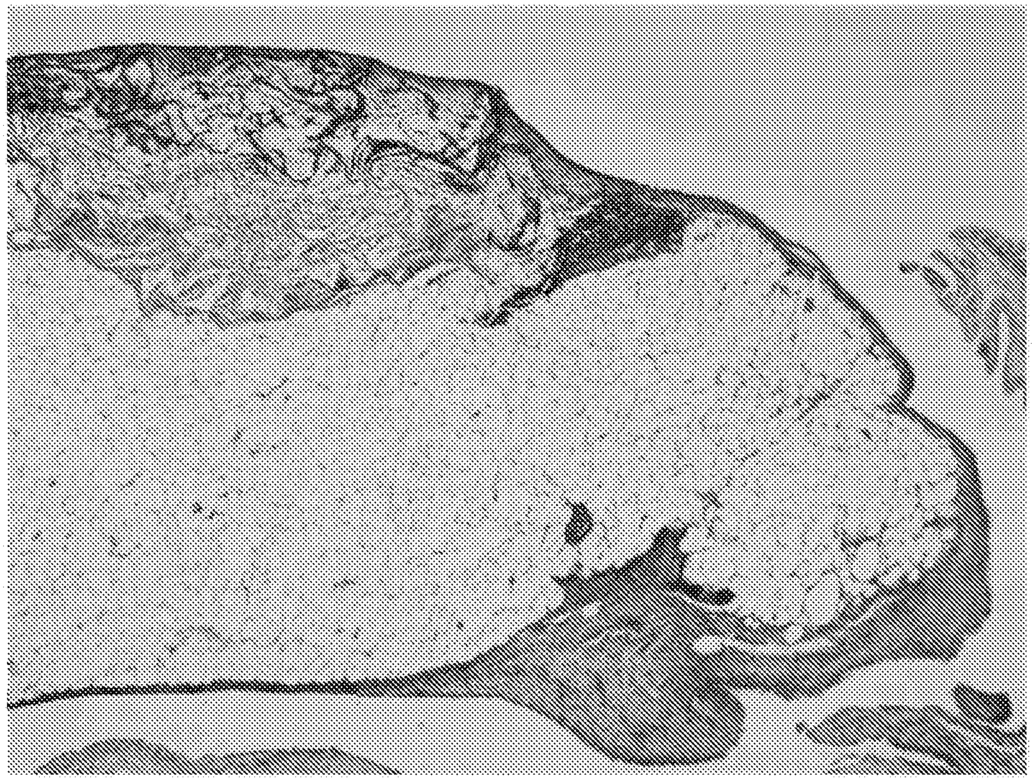

Fig. 5

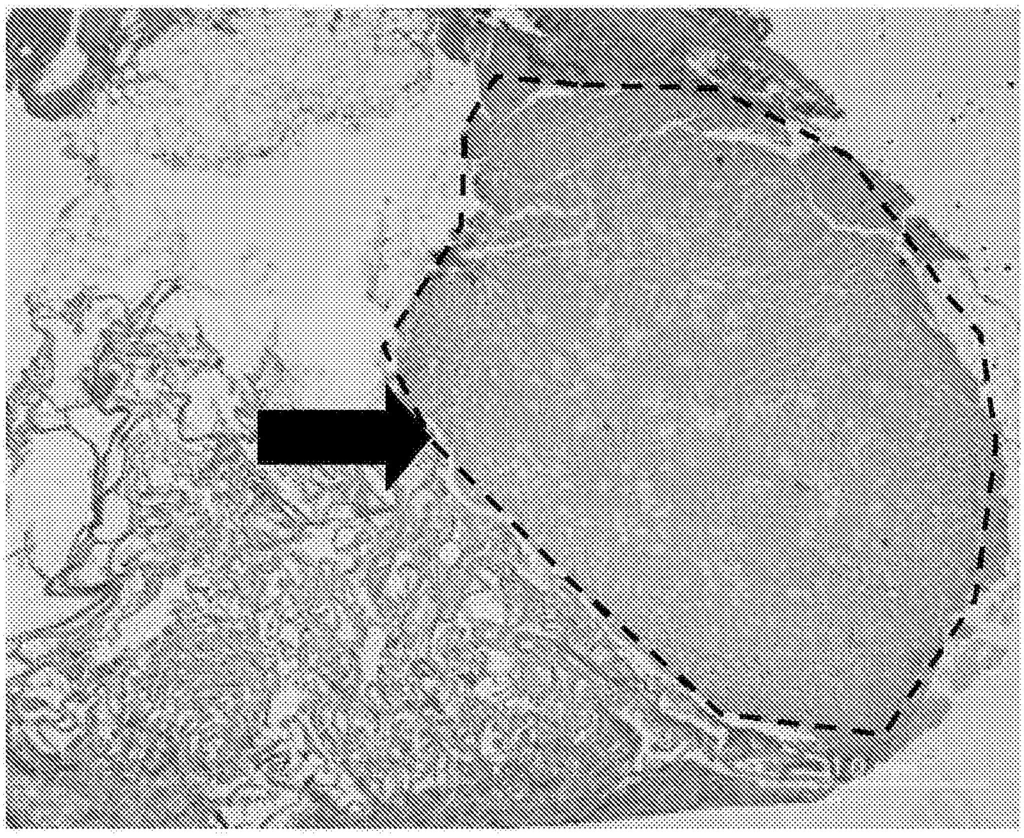

Fig. 6A

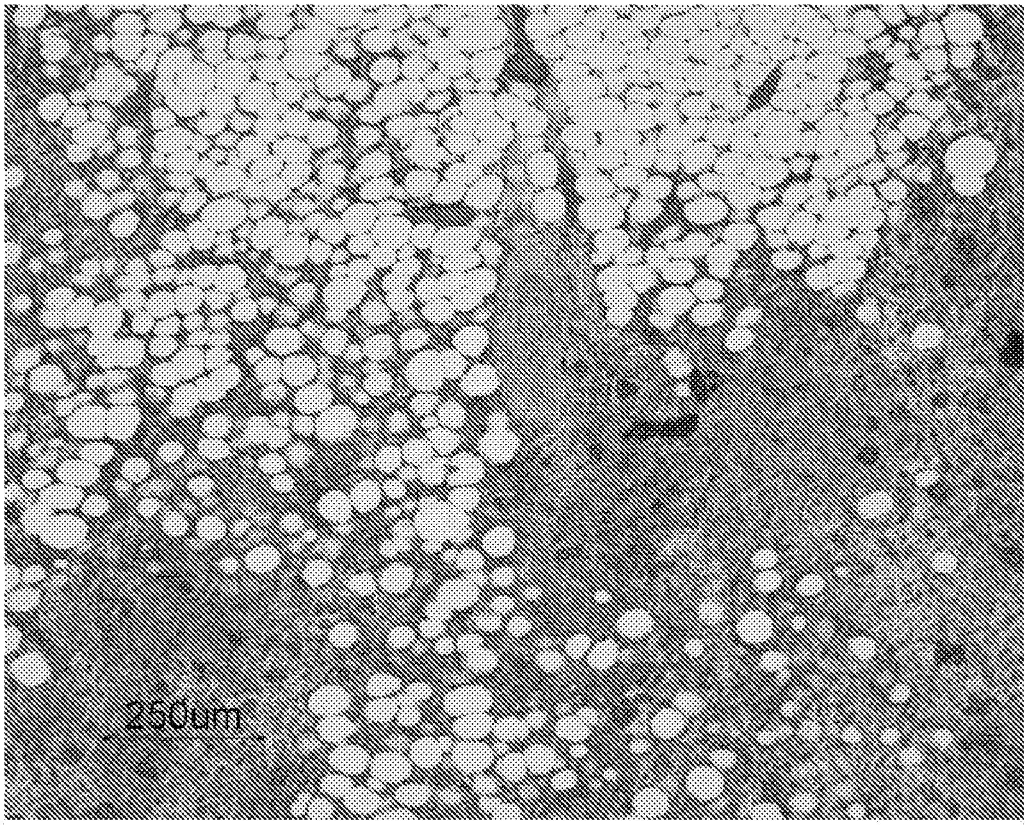

Fig. 6B

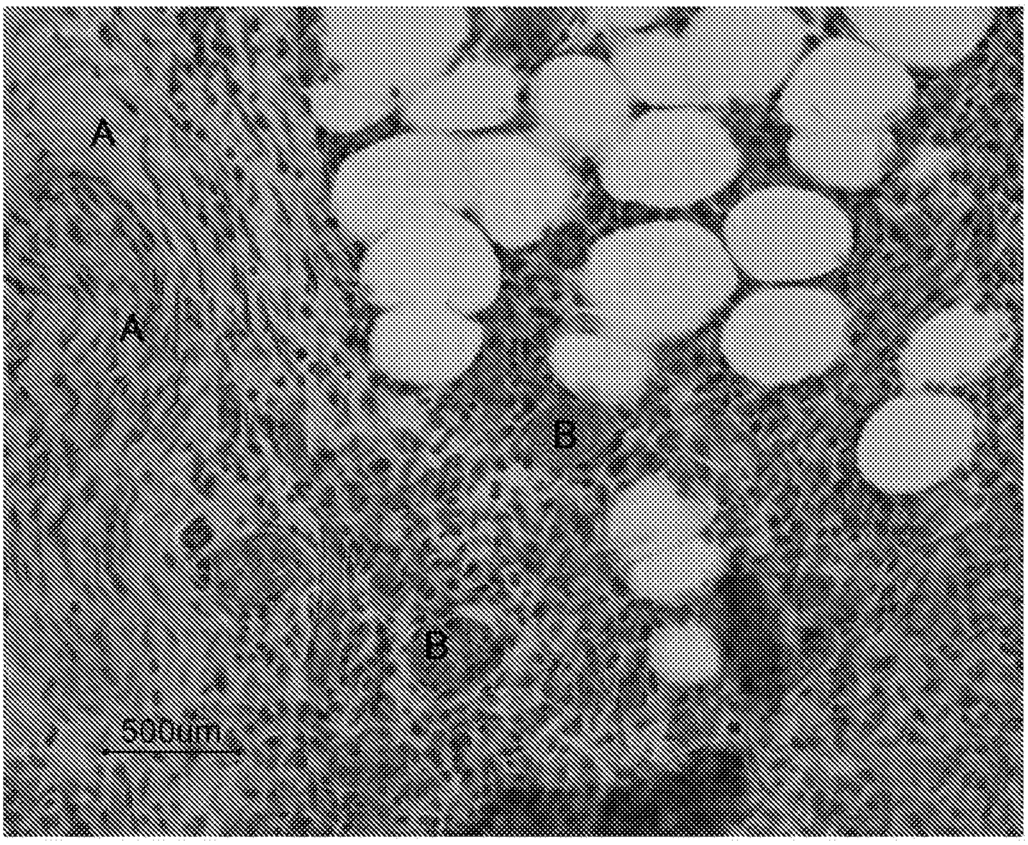

Fig. 6C

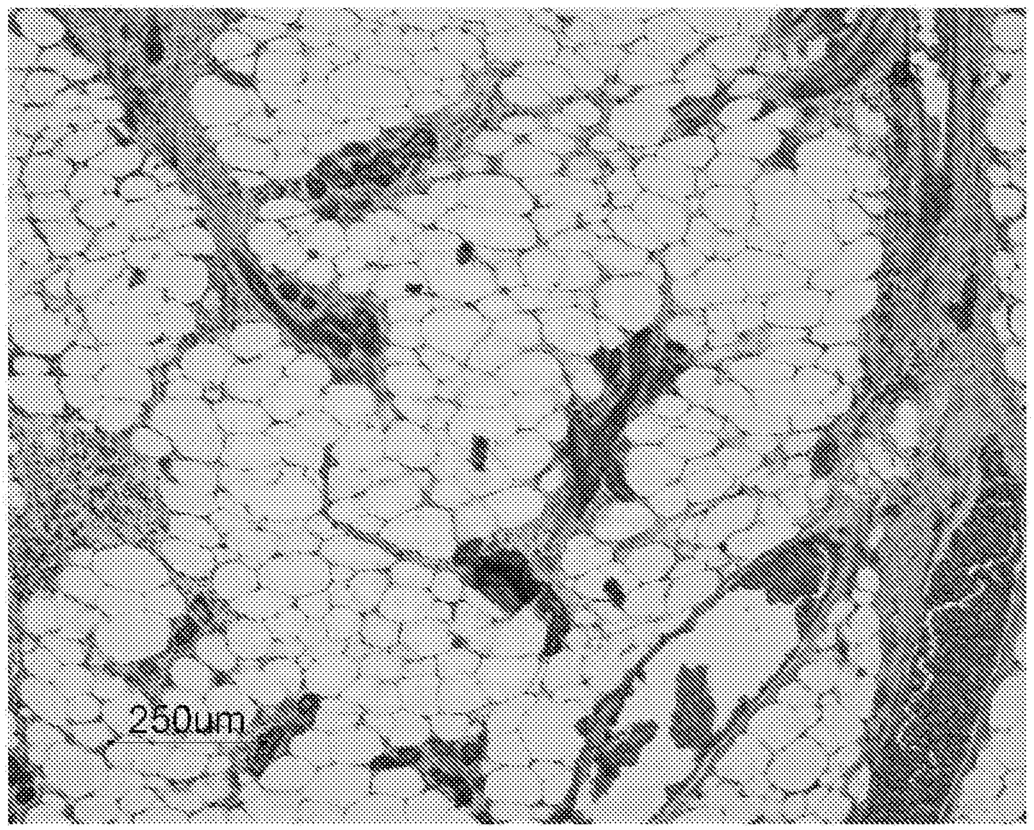

**Fig. 6D**

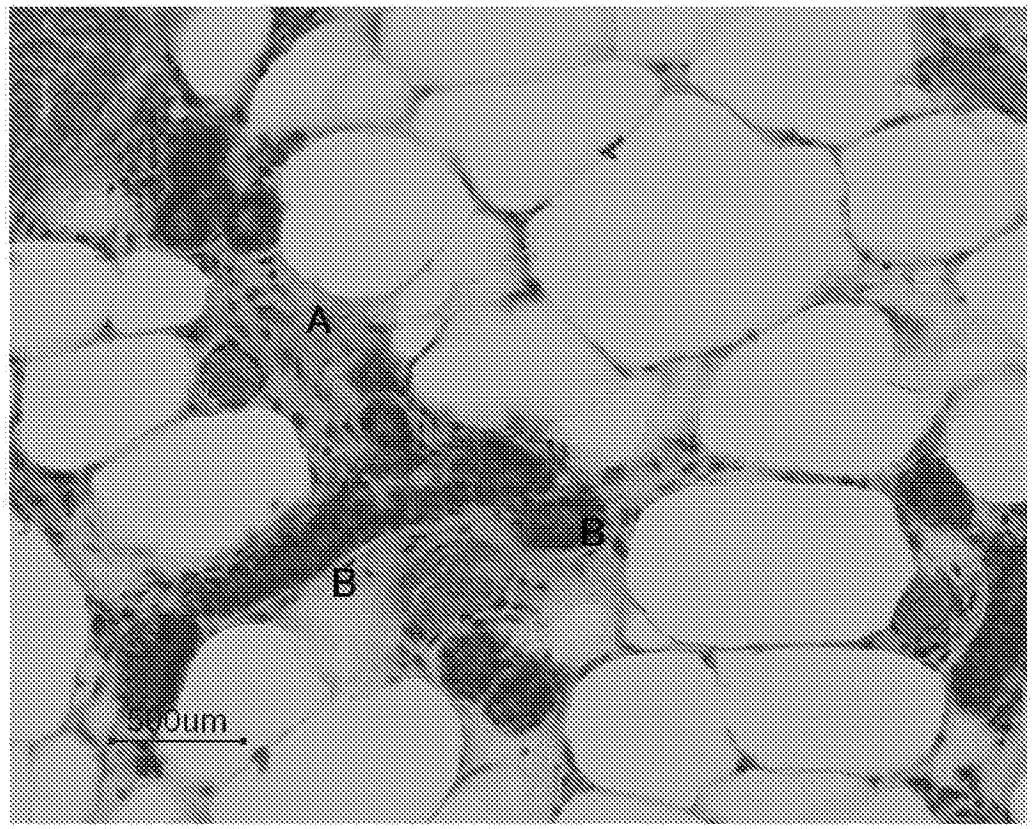

Fig. 6E

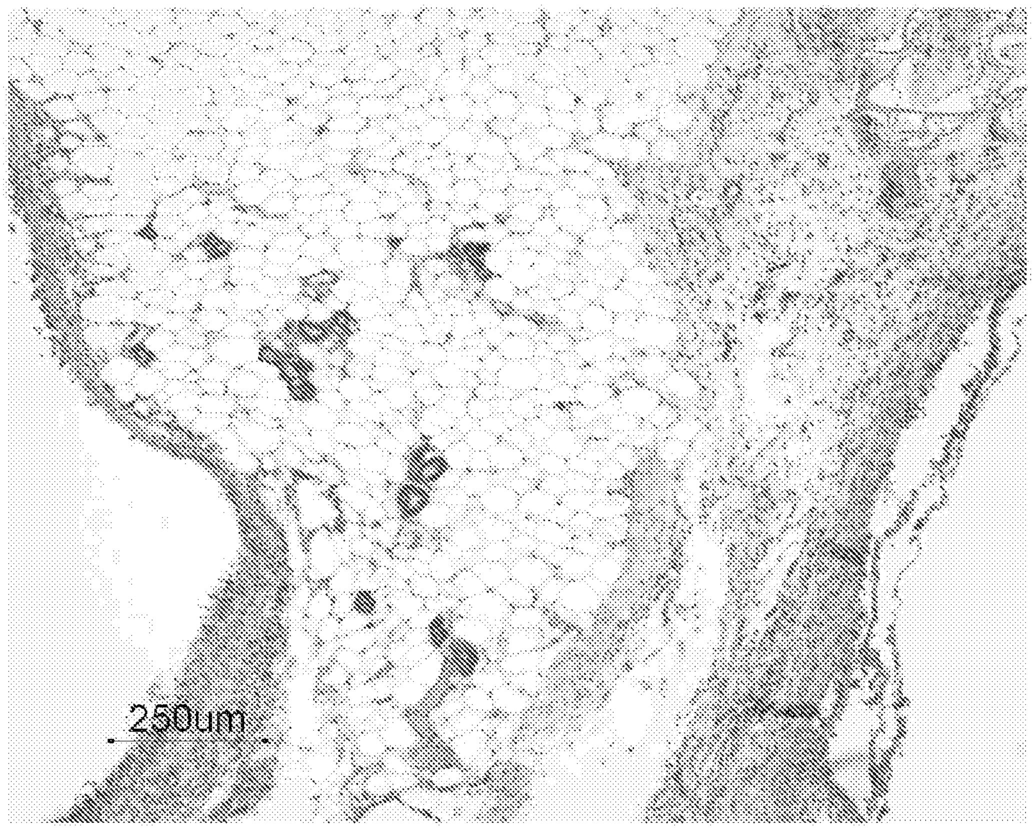

Fig. 6F

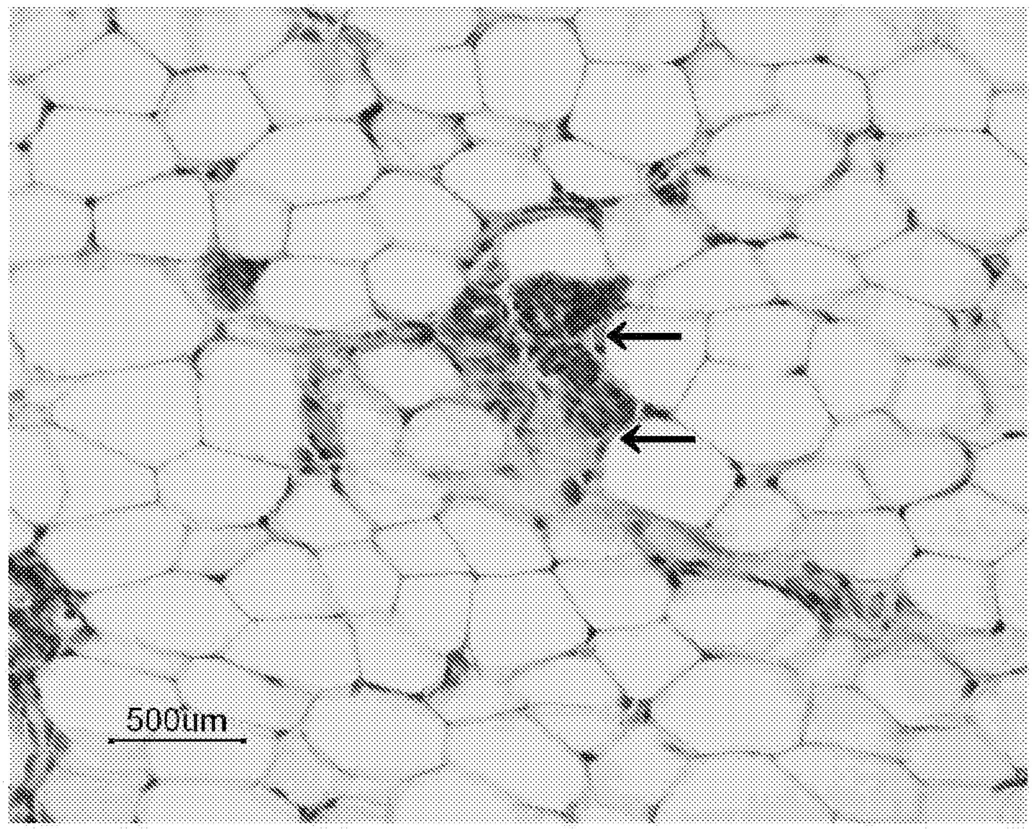

Fig. 6G

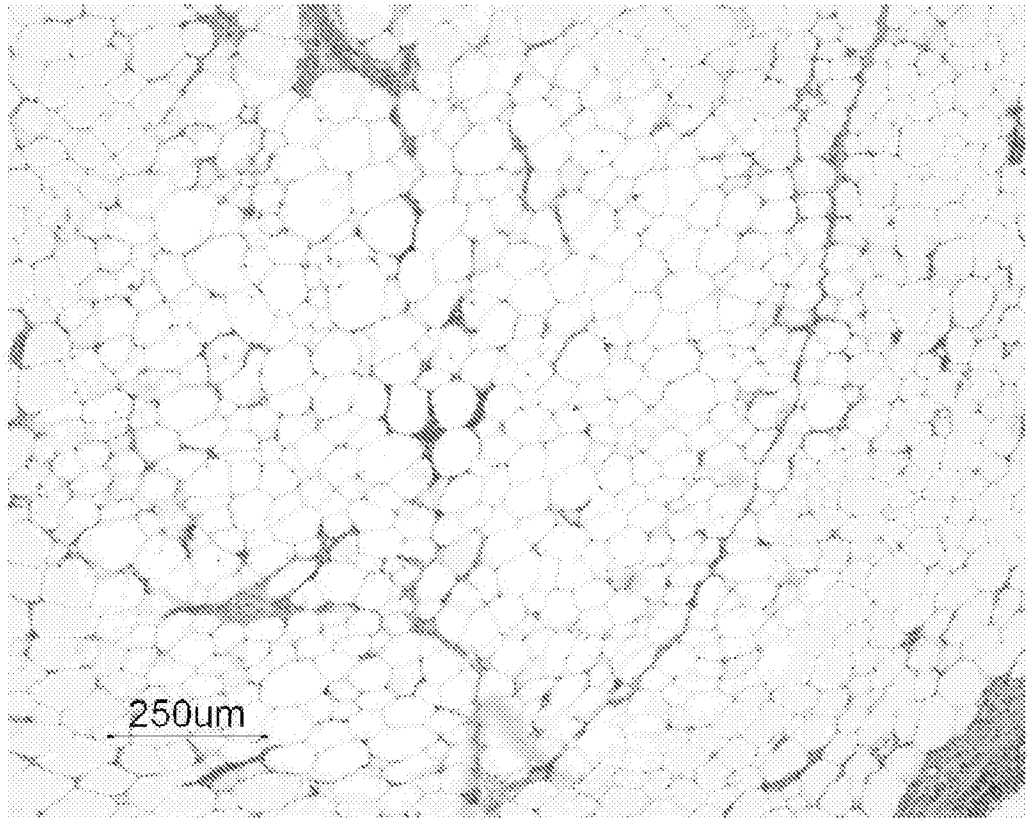

Fig. 6H

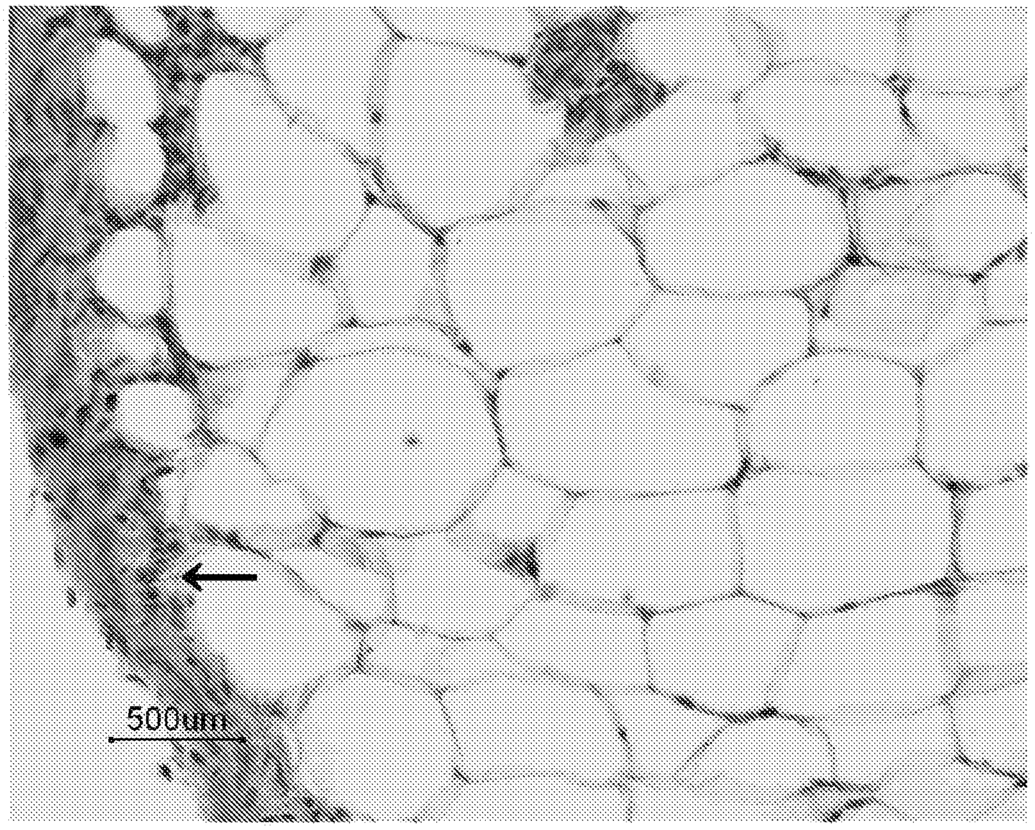

Fig. 7

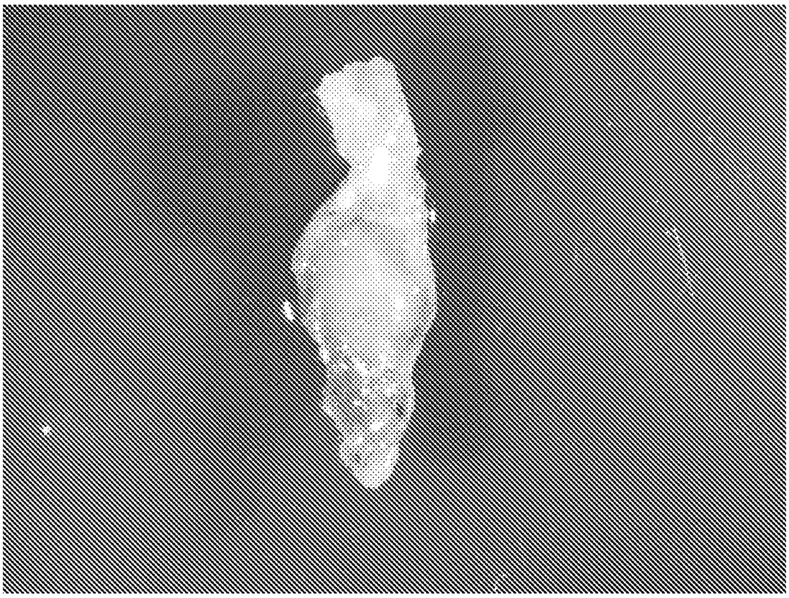

**Fig. 8**

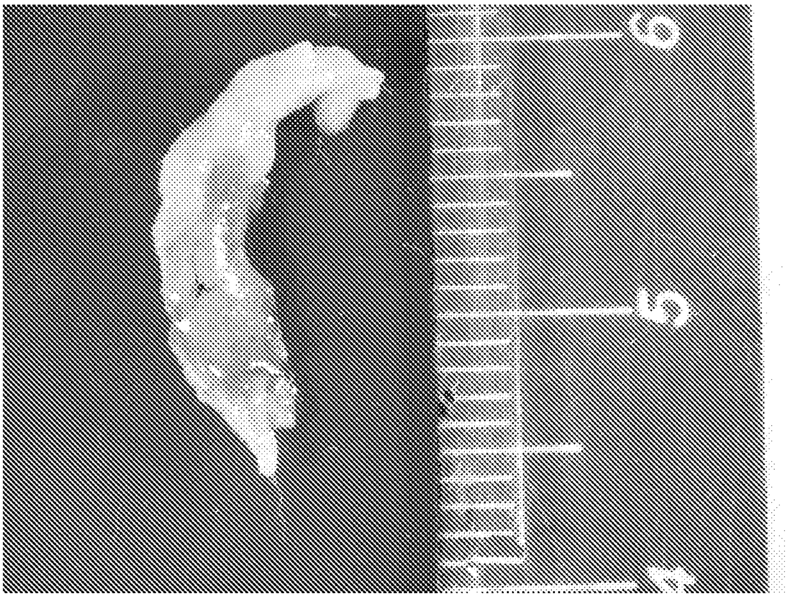

**Fig. 9**

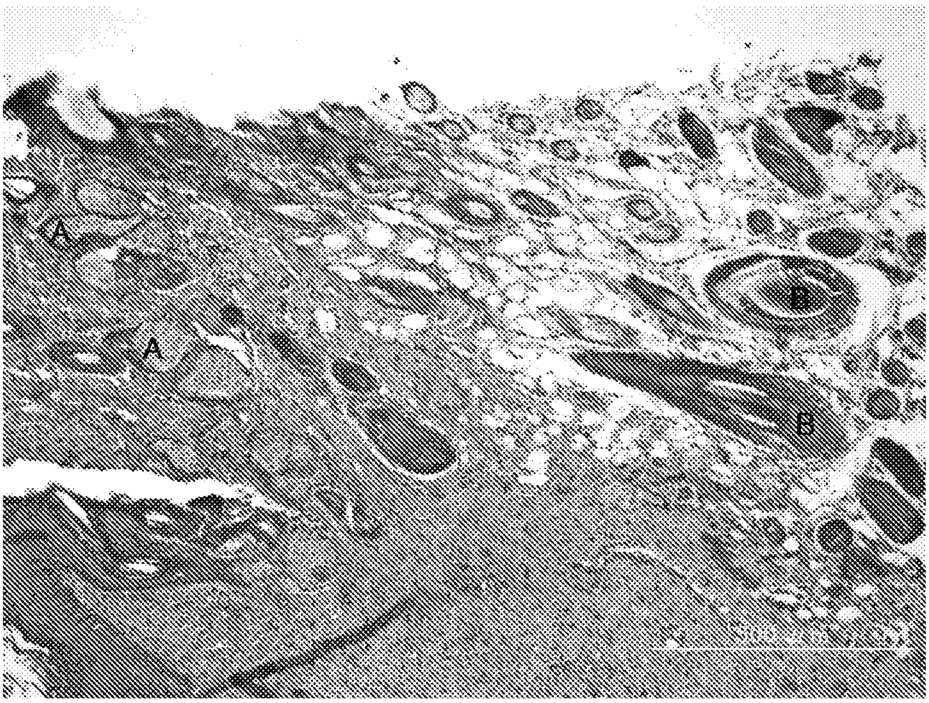

**INTERNATIONAL SEARCH REPORT**

International application No.
PCT/JP2009/063768

A. CLASSIFICATION OF SUBJECT MATTER
C12N5/10(2006.01)i, A61L27/00(2006.01)i, C12N1/04(2006.01)i, C12N5/06(2006.01)i, C12N15/09(2006.01)n

According to International Patent Classification (IPC) or to both national classification and IPC

B. FIELDS SEARCHED

Minimum documentation searched (classification system followed by classification symbols)
C12N5/10, A61L27/00, C12N1/04, C12N5/06, C12N15/09

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/BIOSIS/MEDLINE/WPIDS(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X/Y/A | JP 2007-524360 A (The Regents of the University of California), 30 August, 2007 (30.08.07), Claims; Par. No. [0012] & US 2006/0292670 A1 & US 2007/0134291 A1 & US 2007/0128697 A1 & US 2008/0274186 A1 & EP 1594889 A & WO 2004/024893 A2 & WO 2004/072100 A2 | 1-5,7,16-25, 27,36-41, 46-52,57-62, 64-66,68, 70-72,74-76/ 63/6,8-15, 26,28-35, 42-45,53-56, 67,69,73, 77-100 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>16 September, 2009 (16.09.09) | Date of mailing of the international search report<br>06 October, 2009 (06.10.09) |
| Name and mailing address of the ISA/<br>Japanese Patent Office<br>Facsimile No. | Authorized officer<br>Telephone No. |

Form PCT/ISA/210 (second sheet) (April 2007)

INTERNATIONAL SEARCH REPORT

International application No.
PCT/JP2009/063768

C (Continuation). DOCUMENTS CONSIDERED TO BE RELEVANT

| Category* | Citation of document, with indication, where appropriate, of the relevant passages | Relevant to claim No. |
|---|---|---|
| X/Y/A | TARA Aghaloo, et al., Nell-1-induced bone regeneration in calvarial defects., Am J Pathol. (2006), Vol.169, No.3, p.903-915 | 1-5,16-25, 36-40,46-51, 57-62,64-66, 70-72,74-76/ 7,27,41,52, 63,68/6, 8-15,26, 28-35,42-45, 53-56,67,69, 73,77-100 |
| Y/A | LEAH Y. LIU, et al., CHARACTERIZING THE ROLE OF THE NELL1 GENE IN CARDIOVASCULAR DEVELOPMENT., Journal of Undergraduate Research(2007), Volume VII, p.63-70 <URL:http://www.scied.science.doe.gov/SciEd/JUR_v7/pdfs/Characterizing%20Role%20of%20NELL1.pdf> | 1-5,7,16-25, 27,36-41, 46-52,57-66, 68,70-72, 74-76/6, 8-15,26, 28-35,42-45, 53-56,67,69, 73,77-100 |
| Y/A | JP 2003-265169 A (Yasuhiko TAHATA), 24 September, 2003 (24.09.03), Par. No. [0012] (Family: none) | 1-5,7,16-25, 27,36-41, 46-52,57-66, 68,70-72, 74-76/6, 8-15,26, 28-35,42-45, 53-56,67,69, 73,77-100 |
| P,X/P,A | WO 2009/042859 A (UT BATTELLE L.L.C.), 02 April, 2009 (02.04.09), Full text & US 2009/0142312 A1 | 1-5,16-25, 36-40,46-51, 57-62,64-66, 70-72,74-76/ 6-15,26-35, 41-45,52-56, 63,67-69,73, 77-100 |
| P,X/P,A | WO 2009/057790 A1 (Katayama Chemical Industries Co., Ltd.), 07 May, 2009 (07.05.09), Full text (Family: none) | 1-5,16-25, 36-40,46-51, 57-62,64-66, 70-72,74-76/ 6-15,26-35, 41-45,52-56, 63,67-69,73, 77-100 |

Form PCT/ISA/210 (continuation of second sheet) (April 2007)

**INTERNATIONAL SEARCH REPORT**

International application No.
PCT/JP2009/063768

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The common matter among claims 1-100 is "the induction of the differentiation of a cell capable of being differentiated in a given direction to thereby produce a cell, a tissue or an organ through the further induction of the differentiation in the given direction".
However, as a result of the search, it is found that the common technical matter among all of the claims is not novel, because a method for differentiating a stem cell in a living body, which is a cell capable of being differentiated in a given direction, into a bone by using NELL-1 is already disclosed in a document JP 2007-524360 A (The Regents of the University of California) 30 August 2007 (30.08.07). (continued to extra sheet)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

**INTERNATIONAL SEARCH REPORT**

International application No.
PCT/JP2009/063768

Continuation of Box No.III of continuation of first sheet(2)

Therefore, the common matter does not go beyond the scope of the prior art and cannot be regarded as a special technical matter in the meaning within PCT Rule 13.2.

Consequently, there is no common special technical matter among all of the claims, and the above-stated inventions cannot be regarded as a group of inventions so linked as to form a single general inventive concept.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006089023 A
- WO 2004072100 A
- WO 2004024893 A
- WO 01024821 A
- JP 2000217571 A
- US 4554101 A

### Non-patent literature cited in the description


- *J. Bone Miner Res.,* June 2007, vol. 22 (6), 918-30
- *J. Dent. Res.,* 2003, vol. 82 (8), 581-584
- *Plast. Reconstr. Surg.,* 2001, vol. 108, 952
- **Isogai N.** Comparison of different chondrocytes for use in tissue engineering of cartilage model structures. *Tissue Eng.,* April 2006, vol. 12 (4), 691-703
- **Kuroda, S. ; Oyasu, M. ; Kawakami, M. ; Kanayama, N. ; Tanizawa, K. ; Saito, N. ; Abe, T. ; Matsuhashi, S. ; Ting, K.** *Biochem. Biophys. Res. Commun.,* 1999, vol. 265, 79-86
- **Zhang, X. ; Kuroda, S. ; Carpenter, D. ; Nishimura, I. ; Soo, C. ; Moats, R. ; Iida, K. ; Wisner, E. ; Hu, F. Y. ; Miao, S.** *J. Clin. Invest.,* 2002, vol. 18, 2126-2134
- **Batzer et al.** *Nucleic Acid Res.,* 1991, vol. 19, 5081
- **Ohtsuka et al.** *J. Biol. Chem.,* 1985, vol. 260, 2605-2608
- **Rossolini et al.** *Mol. Cell. Probes,* 1994, vol. 8, 91-98
- **Altschul et al.** *J. Mol. Biol.,* 1990, vol. 215, 403-410
- **Pearson ; Lipman.** *Proc. Natl. Acad. Sci., USA,* 1988, vol. 85, 2444-2448
- **Smith ; Waterman.** *J. Mol. Biol.,* 1981, vol. 147, 195-197
- **Needleman ; Wunsch.** *J. Mol. Biol.,* 1970, vol. 48, 443-453
- Laboratory Techniques In Biochemistry And Molecular Biology-Hybridization With Nucleic Acid Probes Part I. **Tijssen.** Overview of principles of hybridization and the strategy of nucleic acid probe assay. Elsevier, 1993
- Current Protocols in Molecular Biology. DNA Cloning 1: Core Techniques, A Practical Approach. Oxford UniversityPress, 1995, vol. 1
- **Sambrook et al.** Molecular Cloning: A Laboratory Manual. Cold Spring Harbor Laboratory, 1989
- **Anderson et al.** Nucleic Acid Hybridization: a Practical approach. IRL Press Limited, vol. IV
- **Anderson et al.** Nucleic Acid Hybridization: a Practical Approach. IRL Press Limited
- **Suggs et al.** Developmental Biology Using Purified Genes. 1981, 683
- **Mark Zoller ; Michael Smith.** site-specific mutagenesis. *Methods in Enzymology,* 1983, vol. 100, 468-500
- **Kyte. J ; Doolittle, R. F.** *J. Mol. Biol.,* 1982, vol. 157 (1), 105-132
- **Alison MR.** Hepatocytes from non-hepatic adult stem cells. *Nature,* 20 July 2000, vol. 406 (6793), 257
- **Ogawa K.** Living donor liver transplantation with reduced monosegments for neonates and small infants. *Transplantation,* 27 May 2007, vol. 83 (10), 1337-40
- **Zuk PA.** Multilineage cells from human adipose tissue: implications for cell-based therapies. *Tissue Eng.,* April 2001, vol. 7 (2), 211-28
- **Alison MR.** Hepatocytes from non-hepatic adult stem cells. *Nature,* 20 July 2000, vol. 406 (6793), 257
- **Mayahara M.** *Anat Rec A Discov Mol Cell Evol Biol,* September 2003, vol. 274 (1), 817-26
- **Ogawa K.** Living donor liver transplantation with reduced monosegments for neonates and small infants. *Transplantation,* 27 May 2007, vol. 83 (10), 1337-40
- **Kamiya A.** Oncostatin M and hepatocyte growth factor induce hepatic maturation via distinct signaling pathways. *FEBS Lett.,* 09 March 2001, vol. 492 (1-2), 90-4
- **Kojima I.** Conophylline: a novel differentiation inducer for pancreatic beta cells. *Int J Biochem Cell Biol.,* 2006, vol. 38 (5-6), 923-30
- **Noritaka Isogai ; Hirohisa Kusuhara ; Yoshito Ikada ; Hitoshi Ohtani ; Robin Jacquet ; Jennifer Hillyer ; Elizabeth Lowder ; William J. Landis.** *Tissue Engineering.,* 01 April 2006, vol. 12 (4), 691-703